# EUROPEAN PATENT APPLICATION

(11) **EP 2 762 498 A1**
(43) Date of publication of application: **06.08.2014**
(21) Application number: 13179127.9
(22) Date of filing: 18.04.2008
(51) Int. Cl.: C07K 19/00, C07K 14/415, C07K 2/00, C12N 15/62, C12N 15/63, C12N 15/29

(54) **Modified plant defensin**

(30) Priority: 20.04.2007 US 912984 P
(62) Divisional of application: 08733377.9
(71) Applicant: Hexima Limited, Melbourne, VIC 3000 (AU)
(72) Inventor: Anderson, Marilyn Anne, Keilor, Victoria 3036 (AU); Heath, Robyn Louise, Clifton Hill, Victoria 3068 (AU); Lay, Fung Tso, Reservoir, Victoria 3073 (AU); Poon, Simon, Collingwood, VIC 3066 (AU)
(74) Representative: Rigby, Barbara Nicole

(57) **Abstract**

The invention herein includes a general method for reducing or eliminating a toxic effect of transgenic defensin expression in a host plant. The invention also includes a method of modifying a nucleic acid encoding a defensin, a nucleic acid modified thereby and a modified defensin encoded by the modified nucleic acid sequence. The invention also includes a transgenic plant containing and expressing the modified defensin-coding nucleic acid sequence, the plant exhibiting reduced or eliminated toxic effects of defensin, compared with otherwise comparable transgenic plants expressing an unmodified defensin. The modified defensin is termed a chimeric defensin having a mature defensin domain of a first plant defensin combined with a C-terminal propeptide domain of a second plant defensin or a vacuolar translocation peptide.

SEQUENCE LISTING

<110>  Hexima LTD
       Anderson, Marilyn
       Heath, Robyn

<120>  Modified Plant Defensin

<130>  46-06

<140>  unassigned
<141>  2008-01-01

<150>  US 60/912,984
<151>  2007-04-20

<160>  44

<170>  PatentIn version 3.5

<210>  1
<211>  105
<212>  PRT
<213>  Nicotiana alata

<400>  1

Met Ala Arg Ser Leu Cys Phe Met Ala Phe Ala Ile Leu Ala Met Met
1               5                   10                  15

Leu Phe Val Ala Tyr Glu Val Gln Ala Arg Glu Cys Lys Thr Glu Ser
            20                  25                  30

Asn Thr Phe Pro Gly Ile Cys Ile Thr Lys Pro Pro Cys Arg Lys Ala
        35                  40                  45

Cys Ile Ser Glu Lys Phe Thr Asp Gly His Cys Ser Lys Ile Leu Arg
    50                  55                  60

Arg Cys Leu Cys Thr Lys Pro Cys Val Phe Asp Glu Lys Met Thr Lys
65                  70                  75                  80

Thr Gly Ala Glu Ile Leu Ala Glu Glu Ala Lys Thr Leu Ala Ala Ala
                85                  90                  95

Leu Leu Glu Glu Glu Ile Met Asp Asn
            100                 105

<210>  2
<211>  103
<212>  PRT
<213>  Petunia hybrida

<400>  2

Met Ala Arg Ser Ile Cys Phe Phe Ala Val Ala Ile Leu Ala Leu Met

1               5                   10                  15

Leu Phe Ala Ala Tyr Asp Ala Glu Ala Ala Thr Cys Lys Ala Glu Cys
            20                  25                  30

Pro Thr Trp Asp Ser Val Cys Ile Asn Lys Lys Pro Cys Val Ala Cys
        35                  40                  45

Cys Lys Lys Ala Lys Phe Ser Asp Gly His Cys Ser Lys Ile Leu Arg
    50                  55                  60

Arg Cys Leu Cys Thr Lys Glu Cys Val Phe Glu Lys Thr Glu Ala Thr
65                  70                  75                  80

Gln Thr Glu Thr Phe Thr Lys Asp Val Asn Thr Leu Ala Glu Ala Leu
                85                  90                  95

Leu Glu Ala Asp Met Met Val
            100

<210>  3
<211>  101
<212>  PRT
<213>  Petunia hybrida

<400>  3

Met Ala Arg Ser Ile Cys Phe Phe Ala Val Ala Ile Leu Ala Leu Met
1               5                   10                  15

Leu Phe Ala Ala Tyr Glu Thr Glu Ala Gly Thr Cys Lys Ala Glu Cys
            20                  25                  30

Pro Thr Trp Glu Gly Ile Cys Ile Asn Lys Ala Pro Cys Val Lys Cys
        35                  40                  45

Cys Lys Ala Gln Pro Glu Lys Phe Thr Asp Gly His Cys Ser Lys Ile
    50                  55                  60

Leu Arg Arg Cys Leu Cys Thr Lys Pro Cys Ala Thr Glu Glu Ala Thr
65                  70                  75                  80

Ala Thr Leu Ala Asn Glu Val Lys Thr Met Ala Glu Ala Leu Val Glu
                85                  90                  95

Glu Asp Met Met Glu
            100

<210>  4

<211>  105
<212>  PRT
<213>  Nicotiana tabacum

<400>  4

Met Ala Arg Ser Leu Cys Phe Met Ala Phe Ala Ile Leu Ala Met Met
1               5                   10                  15

Leu Phe Val Ala Tyr Glu Val Gln Ala Arg Glu Cys Lys Thr Glu Ser
            20                  25                  30

Asn Thr Phe Pro Gly Ile Cys Ile Thr Lys Pro Pro Cys Arg Lys Ala
        35                  40                  45

Cys Ile Ser Glu Lys Phe Thr Asp Gly His Cys Ser Lys Leu Leu Arg
    50                  55                  60

Arg Cys Leu Cys Thr Lys Pro Cys Val Phe Asp Glu Lys Met Ile Lys
65                  70                  75                  80

Thr Gly Ala Glu Thr Leu Val Glu Glu Ala Lys Thr Leu Ala Ala Ala
                85                  90                  95

Leu Leu Glu Glu Glu Ile Met Asp Asn
            100                 105


<210>  5
<211>  106
<212>  PRT
<213>  Nicotiana attenuata

<400>  5

Met Ala Arg Ser Leu Cys Phe Met Ala Phe Ala Val Leu Ala Met Met
1               5                   10                  15

Leu Phe Val Ala Tyr Glu Val Gln Ala Lys Ser Thr Cys Lys Ala Glu
            20                  25                  30

Ser Asn Thr Phe Glu Gly Phe Cys Val Thr Lys Pro Pro Cys Arg Arg
        35                  40                  45

Ala Cys Leu Lys Glu Lys Phe Thr Asp Gly Lys Cys Ser Lys Ile Leu
    50                  55                  60

Arg Arg Cys Ile Cys Tyr Lys Pro Cys Val Phe Asp Gly Lys Met Ile
65                  70                  75                  80

Asn Thr Gly Ala Glu Thr Leu Ala Glu Glu Ala Asn Thr Leu Ala Glu
                85                  90                  95

Ala Leu Leu Glu Glu Glu Met Met Asp Asn
                100                 105

<210>  6
<211>  105
<212>  PRT
<213>  Nicotiana excelsior

<400>  6

Met Ala Arg Ser Val Cys Phe Met Ala Phe Ala Ile Leu Ala Val Met
1               5                   10                  15

Leu Phe Val Ala Tyr Asp Val Glu Ala Lys Asp Cys Lys Thr Glu Ser
            20                  25                  30

Asn Thr Phe Pro Gly Ile Cys Ile Thr Lys Pro Pro Cys Arg Lys Ala
        35                  40                  45

Cys Ile Lys Glu Lys Phe Thr Asp Gly His Cys Ser Lys Ile Leu Arg
    50                  55                  60

Arg Cys Leu Cys Thr Lys Pro Cys Val Phe Asp Glu Lys Met Ile Lys
65                  70                  75                  80

Thr Gly Ala Glu Thr Leu Ala Glu Glu Ala Thr Thr Leu Ala Ala Ala
                85                  90                  95

Leu Leu Glu Glu Glu Ile Met Asp Asn
            100                 105

<210>  7
<211>  106
<212>  PRT
<213>  Nicotiana paniculata

<400>  7

Met Ala Arg Ser Leu Cys Phe Met Ala Phe Ala Val Leu Ala Met Met
1               5                   10                  15

Leu Phe Val Ala Tyr Glu Val Gln Ala Lys Ser Thr Cys Lys Ala Glu
            20                  25                  30

Ser Asn Thr Phe Pro Gly Leu Cys Ile Thr Lys Pro Pro Cys Arg Lys
        35                  40                  45

Ala Cys Leu Ser Glu Lys Phe Thr Asp Gly Lys Cys Ser Lys Ile Leu
    50                  55                  60

Arg Arg Cys Ile Cys Tyr Lys Pro Cys Val Phe Asp Gly Lys Met Ile
65                  70                  75                  80

Gln Thr Gly Ala Glu Asn Leu Ala Glu Glu Ala Glu Thr Leu Ala Ala
                85                  90                  95

Ala Leu Leu Glu Glu Glu Met Met Asp Asn
            100                 105

<210>  8
<211>  105
<212>  PRT
<213>  Lycopersicon esculentum

<400>  8

Met Ala Arg Ser Ile Phe Phe Met Ala Phe Leu Val Leu Ala Met Met
1               5                   10                  15

Leu Phe Val Thr Tyr Glu Val Glu Ala Gln Gln Ile Cys Lys Ala Pro
            20                  25                  30

Ser Gln Thr Phe Pro Gly Leu Cys Phe Met Asp Ser Ser Cys Arg Lys
        35                  40                  45

Tyr Cys Ile Lys Glu Lys Phe Thr Gly Gly His Cys Ser Lys Leu Gln
    50                  55                  60

Arg Lys Cys Leu Cys Thr Lys Pro Cys Val Phe Asp Lys Ile Ser Ser
65                  70                  75                  80

Glu Val Lys Ala Thr Leu Gly Glu Glu Ala Lys Thr Leu Ser Glu Val
                85                  90                  95

Val Leu Glu Glu Glu Ile Met Met Glu
            100                 105

<210>  9
<211>  107
<212>  PRT
<213>  Capsicum chinense

<400>  9

Met Ala Arg Ser Ile Tyr Phe Met Ala Phe Leu Val Leu Ala Val Thr
1               5                   10                  15

Leu Phe Val Ala Asn Gly Val Gln Gly Gln Asn Asn Ile Cys Lys Thr
            20                  25                  30

Thr Ser Lys His Phe Lys Gly Leu Cys Phe Ala Asp Ser Lys Cys Arg
        35                  40                  45

Lys Val Cys Ile Gln Glu Asp Lys Phe Glu Asp Gly His Cys Ser Lys
    50                  55                  60

Leu Gln Arg Lys Cys Leu Cys Thr Lys Asn Cys Val Phe Asp Asn Ile
65                  70                  75                  80

Pro Asn Asp Val Gly Thr Ile Leu Val Gln Asp Ala Lys Thr Leu Glu
                85                  90                  95

Ala Gln Leu Leu Glu Glu Glu Ile Leu Gly Leu
            100                 105

<210>  10
<211>  78
<212>  PRT
<213>  Nicotiana tabacum

<400>  10

Met Ala Asn Ser Met Arg Phe Phe Ala Thr Val Leu Leu Ile Ala Leu
1               5                   10                  15

Leu Val Thr Ala Thr Glu Met Gly Pro Met Thr Ile Ala Glu Ala Arg
            20                  25                  30

Thr Cys Glu Ser Gln Ser His Arg Phe Lys Gly Pro Cys Ser Arg Asp
        35                  40                  45

Ser Asn Cys Ala Thr Val Cys Leu Thr Glu Gly Phe Ser Gly Gly Asp
    50                  55                  60

Cys Arg Gly Phe Arg Arg Arg Cys Phe Cys Thr Arg Pro Cys
65                  70                  75

<210>  11
<211>  78
<212>  PRT
<213>  Nicotiana tabacum

<400>  11

Met Ala Asn Ser Met Arg Phe Phe Ala Thr Val Leu Leu Ile Ala Leu
1               5                   10                  15

Leu Val Thr Ala Thr Glu Met Gly Pro Met Thr Ile Ala Glu Ala Arg
            20                  25                  30

Thr Cys Glu Ser Gln Ser His Arg Phe Lys Gly Pro Cys Ser Arg Asp

35                  40                  45

Ser Asn Cys Ala Thr Val Cys Leu Thr Glu Gly Phe Ser Gly Gly Arg
    50                  55                  60

Cys Pro Trp Ile Pro Pro Arg Cys Phe Cys Thr Ser Pro Cys
65                  70                  75

<210>  12
<211>  72
<212>  PRT
<213>  Lycopersicon esculentum

<400>  12

Met Arg Leu Phe Ala Thr Met Leu Leu Leu Ala Met Leu Val Met Ala
1               5                   10                  15

Thr Gly Pro Met Arg Ile Val Glu Ala Arg Thr Cys Glu Ser Gln Ser
            20                  25                  30

His Arg Phe Lys Gly Pro Cys Val Ser Glu Lys Asn Cys Ala Ser Val
        35                  40                  45

Cys Glu Thr Glu Gly Phe Ser Gly Gly Asp Cys Arg Gly Phe Arg Arg
    50                  55                  60

Arg Cys Phe Cys Thr Arg Pro Cys
65                  70

<210>  13
<211>  78
<212>  PRT
<213>  Petunia integrifolia

<400>  13

Met Gly Arg Ser Ile Arg Leu Phe Ala Thr Phe Phe Leu Ile Ala Met
1               5                   10                  15

Leu Phe Leu Ser Thr Glu Met Gly Pro Met Thr Ser Ala Glu Ala Arg
            20                  25                  30

Thr Cys Glu Ser Gln Ser His Arg Phe His Gly Thr Cys Val Arg Glu
        35                  40                  45

Ser Asn Cys Ala Ser Val Cys Gln Thr Glu Gly Phe Ile Gly Gly Asn
    50                  55                  60

Cys Arg Ala Phe Arg Arg Arg Cys Phe Cys Thr Arg Asn Cys
65                  70                  75

<210>  14
<211>  75
<212>  PRT
<213>  Capsicum annuum

<400>  14

Met Ala Gly Phe Ser Lys Val Val Ala Thr Ile Phe Leu Met Met Leu
1               5                   10                  15

Leu Val Phe Ala Thr Asp Met Met Ala Glu Ala Lys Ile Cys Glu Ala
            20                  25                  30

Leu Ser Gly Asn Phe Lys Gly Leu Cys Leu Ser Ser Arg Asp Cys Gly
        35                  40                  45

Asn Val Cys Arg Arg Glu Gly Phe Thr Asp Gly Ser Cys Ile Gly Phe
    50                  55                  60

Arg Leu Gln Cys Phe Cys Thr Lys Pro Cys Ala
65                  70                  75

<210>  15
<211>  74
<212>  PRT
<213>  Capsicum annuum

<400>  15

Met Ala Gly Phe Ser Lys Val Ile Ala Thr Ile Phe Leu Met Met Met
1               5                   10                  15

Leu Val Phe Ala Thr Gly Met Val Ala Glu Ala Arg Thr Cys Glu Ser
            20                  25                  30

Gln Ser His Arg Phe Lys Gly Leu Cys Phe Ser Lys Ser Asn Cys Gly
        35                  40                  45

Ser Val Cys His Thr Glu Gly Phe Asn Gly Gly His Cys Arg Gly Phe
    50                  55                  60

Arg Arg Arg Cys Phe Cys Thr Arg His Cys
65                  70

<210>  16
<211>  80
<212>  PRT
<213>  Raphanus sativus

<400>  16

Met Ala Lys Phe Ala Ser Ile Ile Ala Leu Leu Phe Ala Ala Leu Val
1               5                   10                  15

Leu Phe Ala Ala Phe Glu Ala Pro Thr Met Val Glu Ala Gln Lys Leu
            20                  25                  30

Cys Glu Arg Pro Ser Gly Thr Trp Ser Gly Val Cys Gly Asn Asn Asn
        35                  40                  45

Ala Cys Lys Asn Gln Cys Ile Asn Leu Glu Lys Ala Arg His Gly Ser
    50                  55                  60

Cys Asn Tyr Val Phe Pro Ala His Lys Cys Ile Cys Tyr Phe Pro Cys
65                  70                  75                  80

<210>  17
<211>  80
<212>  PRT
<213>  Raphanus sativus

<400>  17

Met Ala Lys Phe Ala Ser Ile Ile Val Leu Leu Phe Val Ala Leu Val
1               5                   10                  15

Val Phe Ala Ala Phe Glu Glu Pro Thr Met Val Glu Ala Gln Lys Leu
            20                  25                  30

Cys Gln Arg Pro Ser Gly Thr Trp Ser Gly Val Cys Gly Asn Asn Asn
        35                  40                  45

Ala Cys Lys Asn Gln Cys Ile Arg Leu Glu Lys Ala Arg His Gly Ser
    50                  55                  60

Cys Asn Tyr Val Phe Pro Ala His Lys Cys Ile Cys Tyr Phe Pro Cys
65                  70                  75                  80

<210>  18
<211>  107
<212>  PRT
<213>  Zea mays

<400>  18

Met Pro Ser Tyr Lys Lys Leu Val Ile Val Gly Phe Ala Leu Thr Leu
1               5                   10                  15

Leu Leu Val Ser Phe Gly Met Asp Ala Ser Ala Lys Leu Cys Ser Thr
            20                  25                  30

Thr Met Asp Leu Leu Ile Cys Gly Gly Ala Ile Pro Gly Ala Val Asn

35                  40                  45

Gln Ala Cys Asp Asp Thr Cys Arg Asn Lys Gly Tyr Thr Gly Gly Gly
    50                  55                  60

Phe Cys Asn Met Lys Ile Gln Arg Cys Val Cys Arg Lys Pro Cys Ala
65                  70                  75                  80

Leu Glu Glu Gln Thr Glu Ala Arg Ala Gly Asp Glu Ala Ala Gly Gly
                85                  90                  95

Ala Gly Asp Met Met Ser Arg Thr Met Ala Asp
            100                 105

<210>  19
<211>  108
<212>  PRT
<213>  Artemisia vulgaris

<400>  19

Ala Gly Ser Lys Leu Cys Glu Lys Thr Ser Lys Thr Tyr Ser Gly Lys
1               5                   10                  15

Cys Asp Asn Lys Lys Cys Asp Lys Lys Cys Ile Glu Trp Glu Lys Ala
            20                  25                  30

Gln His Gly Ala Cys His Lys Arg Glu Ala Gly Lys Glu Ser Cys Phe
        35                  40                  45

Cys Tyr Phe Asp Cys Ser Lys Ser Pro Pro Gly Ala Thr Pro Ala Pro
    50                  55                  60

Pro Gly Ala Ala Pro Pro Pro Ala Ala Gly Gly Ser Pro Ser Pro Pro
65                  70                  75                  80

Ala Asp Gly Gly Ser Pro Pro Pro Pro Ala Asp Gly Gly Ser Pro Pro
                85                  90                  95

Val Asp Gly Gly Ser Pro Pro Pro Pro Ser Thr His
            100                 105

<210>  20
<211>  154
<212>  PRT
<213>  Helianthus annuus

<400>  20

Asp Ile Ala Thr Val Asn Gly Lys Ile Cys Glu Lys Pro Ser Lys Thr
1               5                   10                  15

Trp Phe Gly Asn Cys Lys Asp Thr Asp Lys Cys Asp Lys Arg Cys Ile
            20                  25                  30

Asp Trp Glu Gly Ala Lys His Gly Ala Cys His Gln Arg Glu Ala Lys
        35                  40                  45

His Met Cys Phe Cys Tyr Phe Asp Cys Asp Pro Gln Lys Asn Pro Gly
    50                  55                  60

Pro Pro Pro Gly Ala Pro Gly Thr Pro Gly Thr Pro Pro Ala Pro Pro
65                  70                  75                  80

Gly Lys Gly Glu Gly Asp Ala Pro His Pro Pro Pro Thr Pro Ser Pro
                85                  90                  95

Pro Gly Gly Asp Gly Gly Ser Gly Pro Ala Pro Pro Ala Gly Gly Gly
            100                 105                 110

Ser Pro Pro Pro Ala Gly Gly Asp Gly Gly Gly Gly Ala Pro Pro Pro
        115                 120                 125

Ala Gly Gly Asp Gly Gly Gly Gly Ala Pro Pro Pro Ala Gly Gly Asp
    130                 135                 140

Gly Gly Gly Gly Ala Pro Pro Pro Gly Ala
145                 150

<210>  21
<211>  22
<212>  PRT
<213>  Nicotiana tabacum

<400>  21

Val Ser Gly Gly Val Trp Asp Ser Ser Val Glu Thr Asn Ala Thr Ala
1               5                   10                  15

Ser Leu Val Ser Glu Met
            20

<210>  22
<211>  20
<212>  PRT
<213>  Nicotiana tabacum

<400>  22

Asn Gly Gln Ala His Pro Asn Phe Pro Leu Glu Met Pro Gly Ser Asp
1               5                   10                  15

Glu Val Ala Lys
            20

<210>  23
<211>  21
<212>  PRT
<213>  Bertholletia excelsa

<400>  23

Ile Pro Ser Arg Cys Asn Leu Ser Pro Met Arg Cys Pro Met Gly Gly
1               5                   10                  15

Ser Ile Ala Gly Phe
            20

<210>  24
<211>  15
<212>  PRT
<213>  Hordeum vulgare

<400>  24

Val Phe Ala Glu Ala Ile Ala Ala Asn Ser Thr Leu Val Ala Glu
1               5                   10                  15

<210>  25
<211>  14
<212>  PRT
<213>  Triticum aestivum

<400>  25

Val Phe Ala Glu Ile Thr Ala Asn Ser Thr Leu Leu Gln Glu
1               5                   10

<210>  26
<211>  11
<212>  PRT
<213>  Nicotiana tabacum

<400>  26

Met Asn Val Leu Val Ser Pro Val Asp Lys Glu
1               5                   10

<210>  27
<211>  10
<212>  PRT
<213>  Glycine max

<400>  27

Pro Leu Ser Ser Ile Leu Arg Ala Phe Tyr
1               5                   10

<210>  28
<211>  9
<212>  PRT
<213>  Canavalia ensiformis

<400>  28

Glu Ile Pro Asp Ile Thr Ala Val Val
1               5


<210>  29
<211>  8
<212>  PRT
<213>  Hordeum vulgare

<400>  29

Lys Tyr Asp Asp Glu Leu Lys Ala
1               5


<210>  30
<211>  7
<212>  PRT
<213>  Nicotiana tabacum

<400>  30

Gly Leu Leu Val Asp Thr Met
1               5


<210>  31
<211>  4
<212>  PRT
<213>  Phaseolus vulgaris

<400>  31

Ala Phe Val Tyr
1


<210>  32
<211>  237
<212>  DNA
<213>  Nicotiana alata


<220>
<221>  CDS
<222>  (1)..(234)

<400>  32
atg gca aac tcc atg cgc ttc ttt gct act gtg tta ctt cta aca ttg        48
Met Ala Asn Ser Met Arg Phe Phe Ala Thr Val Leu Leu Leu Thr Leu
1               5                   10                  15

ctt ttc atg gct aca gag atg gga cca atg aca att gca gag gca aga        96
Leu Phe Met Ala Thr Glu Met Gly Pro Met Thr Ile Ala Glu Ala Arg
            20                  25                  30

act tgc gag tct cag agc cac cgt ttc aag gga cca tgc gca aga gat 144
Thr Cys Glu Ser Gln Ser His Arg Phe Lys Gly Pro Cys Ala Arg Asp
        35                  40                  45

agc aac tgt gcc acc gtc tgt ttg aca gaa gga ttt tcc ggt ggc gac 192
Ser Asn Cys Ala Thr Val Cys Leu Thr Glu Gly Phe Ser Gly Gly Asp
    50                  55                  60

tgc cgt gga ttc cgc cgc cgt tgt ttc tgt acc agc cct tgc taa 237
Cys Arg Gly Phe Arg Arg Arg Cys Phe Cys Thr Ser Pro Cys
65                  70                  75

<210> 33
<211> 78
<212> PRT
<213> Nicotiana alata

<400> 33

Met Ala Asn Ser Met Arg Phe Phe Ala Thr Val Leu Leu Leu Thr Leu
1               5                   10                  15

Leu Phe Met Ala Thr Glu Met Gly Pro Met Thr Ile Ala Glu Ala Arg
            20                  25                  30

Thr Cys Glu Ser Gln Ser His Arg Phe Lys Gly Pro Cys Ala Arg Asp
        35                  40                  45

Ser Asn Cys Ala Thr Val Cys Leu Thr Glu Gly Phe Ser Gly Gly Asp
    50                  55                  60

Cys Arg Gly Phe Arg Arg Arg Cys Phe Cys Thr Ser Pro Cys
65                  70                  75

<210> 34
<211> 32
<212> PRT
<213> Lycopersicon esculentum

<400> 34

Val Phe Asp Lys Ile Ser Ser Glu Val Lys Ala Thr Leu Gly Glu Glu
1               5                   10                  15

Ala Lys Thr Leu Ser Glu Val Val Leu Glu Glu Glu Ile Met Met Glu
            20                  25                  30

<210> 35
<211> 25
<212> PRT
<213> Nicotiana alata

<400> 35

Glu Tyr Ala Ser Lys Val Asp Glu Tyr Val Gly Glu Val Glu Asn Asp
1               5                   10                  15

Leu Gln Lys Ser Lys Val Ala Val Ser
            20                  25

<210>  36
<211>  16
<212>  PRT
<213>  Ipomoea batatas

<400>  36

His Ser Arg Phe Asn Pro Ile Arg Leu Pro Thr Thr His Glu Pro Ala
1               5                   10                  15

<210>  37
<211>  17
<212>  PRT
<213>  Solanum tuberosum

<400>  37

Phe Thr Ser Glu Asn Pro Ile Val Leu Pro Thr Thr Cys His Asp Asp
1               5                   10                  15

Asn

<210>  38
<211>  12
<212>  PRT
<213>  Hordeum vulgare

<400>  38

Ser Ser Ser Ser Phe Ala Asp Ser Asn Pro Ile Arg
1               5                   10

<210>  39
<211>  11
<212>  PRT
<213>  Solanum tuberosum

<400>  39

Phe Thr Ser Gln Asn Leu Ile Asp Leu Pro Ser
1               5                   10

<210>  40
<211>  34
<212>  PRT
<213>  Artificial

<220>
<223>  Synthetic construct:  CTTP peptide modified to enable

cross-linkage to maleimide-activated Megathura crenulata keyhole
      limpet hemocyanin

<400>  40

Val Phe Asp Glu Lys Met Thr Lys Thr Gly Ala Glu Ile Leu Ala Glu
1               5                   10                  15

Glu Ala Lys Thr Leu Ala Ala Ala Leu Leu Glu Glu Glu Ile Met Asp
            20                  25                  30

Asn Cys

<210>  41
<211>  4
<212>  PRT
<213>  Artificial Sequence

<220>
<223>  Synthetic construct:  vaculoar targeting sequence.

<400>  41

Val Phe Ala Glu
1

<210>  42
<211>  33
<212>  PRT
<213>  Artificial Sequence

<220>
<223>  Synthetic construct:  CTPP derived from Nicotiana attenuata
      protein NatD1.

<400>  42

Val Phe Asp Gly Lys Met Ile Asn Thr Gly Ala Glu Thr Leu Ala Glu
1               5                   10                  15

Glu Ala Asn Thr Leu Ala Glu Ala Leu Leu Glu Glu Glu Met Met Asp
            20                  25                  30

Asn

<210>  43
<211>  33
<212>  PRT
<213>  Artificial Sequence

<220>
<223>  Synthetic construct:  CTTP derived from Nicotiana tabaccum
      protein (SGN-U372549).

<400>  43

Val Phe Asp Glu Lys Met Ile Lys Thr Gly Ala Glu Thr Phe Ala Glu
1               5                   10                  15

Glu Ala Lys Thr Leu Ala Ala Ala Leu Leu Glu Glu Glu Ile Met Asp
            20                  25                  30

Asn

<210>  44
<211>  29
<212>  PRT
<213>  Nicotiana alata

<400>  44

Met Ala Ala His Arg Val Ser Phe Leu Ala Leu Leu Leu Leu Phe Gly
1               5                   10                  15

Met Ser Leu Leu Val Ser Asn Val Glu His Ala Asp Ala
            20                  25

## Description

### BACKGROUND OF THE INVENTION

Plants produce a variety of chemical compounds, either constitutively or inducibly, to protect themselves against environmental stresses, wounding, or microbial invasion.

Among the chemical defenses that are elaborated by plants, the *de novo* synthesis of defense-related proteins is of pivotal importance (see Lay, F.T. et al. (2005), Curr. Protein Pept. Sci. 6:85-101 and references cited therein). The suite of defense-related proteins can either be expressed constitutively and/or be induced as a result of wounding by herbivores or by microbial invasion. As such, these proteins form pre- and post-infection defensive barriers, respectively. Examples of these proteins include enzyme inhibitors such as α-amylase and proteinase inhibitors, hydrolytic enzymes such as 1,3-β-glucanases and chitinases and other low molecular weight, cysteine-rich antimicrobial proteins. The accumulation of antimicrobial compounds such as oxidized phenolics, tannins and other low molecular weight secondary metabolites (such as phytoalexins) also play an important role in the chemical defense strategy of plants.

Of the plant antimicrobial proteins that have been characterized to date, a large proportion share common characteristics. They are generally small (<10 kDa), highly basic proteins and often contain an even number of cysteine residues (typically 4, 6 or 8). These cysteines all participate in intramolecular disulfide bonds and provide the protein with structural and thermodynamic stability (Broekaert, W.F. et al. (1997) Crit. Rev. Plant Sci. 16:297-323). Based on amino acid sequence identities, primarily with reference to the number and spacing of the cysteine residues, a number of distinct families have been defined. They include the plant defensins (Broekaert *et al.* (1997) *supra;* Broekaert, W.F. et al. (1995) Plant Physiol. 108:1353-1358; Lay, F.T. et al. (2003) Plant Physiol. 131:1283-1293), thionins (Bohlmann, H. (1994) Crit. Rev. Plant Sci. 13:1-16), lipid transfer proteins (Kader, J.C. (1997) Annu. Rev. Plant Physiol. Plant Mol. Biol. 47:627-654; Kader, J.C. (1997) Trends Plant Sci. 2:66-70), herein (Broekaert, W.F. et a/. (1992) Biochemistry 32:4308-4314) and knottin-type proteins (Cammue, B.P. et al. (1992) J. Biol. Chem. 267:2228-2233), as well as antimicrobial proteins from *Macadamia integrifolia* (Marcus, J.P. et al. (1997) Eur. J. Biochem. 244:743-749; McManus, A.M. et al. (1999) J. Mol. Biol. 293:629-638) and *Impatiens balsamina* (Tailor, R.H. et al. (1997) J. Biol. Chem. 272:24480-24487; Patel, S.U. et al. (1998) Biochemistry 37:983-990) (Table 1). All these antimicrobial proteins appear to exert their activities at the level of the plasma membrane of the target microorganisms, although it is likely that the different protein families act via different mechanisms (Broekaert *et al.* (1997) *supra).* The cyclotides are a new family of small, cysteine-rich plant peptides that are common in members of the Rubiaceae and Violaceae families (reviewed in Craik, D.J. et al. (1999) J. Mol. Biol. 294:1327-1336; Craik, D.J. (2001) Toxicon 39:1809:1813; Craik, D.J. et al. (2004) Curr. Prot. Pept. Sci. 5:297-315). These unusual cyclic peptides (Table 1) have been ascribed various biological activities including antibacterial (Tam, J.P. et al. (1999) Proc. Natl. Acad. Sci. U.S.A. 96:8913-8918), anti-HIV (Gustafson, K.R. et al. (1994) J. Am. Chem. Soc. 116:9337-9338) and insecticidal (Jennings, C. et al. (2001) Proc. Natl. Acad. Sci. U.S.A. 98:10614-10619) properties.

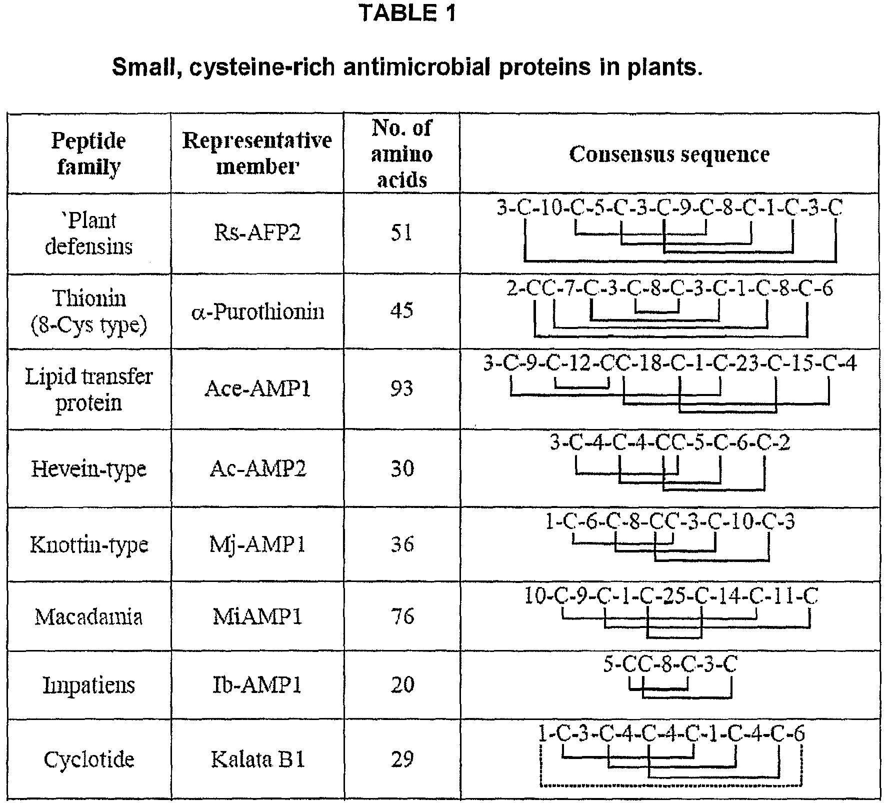

The size of the mature protein and spacing of cysteine residues for representative members of plant antimicrobial proteins is shown. The numbers in the consensus sequence represent the number of amino acids between the highly conserved cysteine residues. The disulfide connectivities are given by connecting lines. The cyclic backbone of the cyclotides is depicted by the broken line. (From Lay and Anderson 2005).

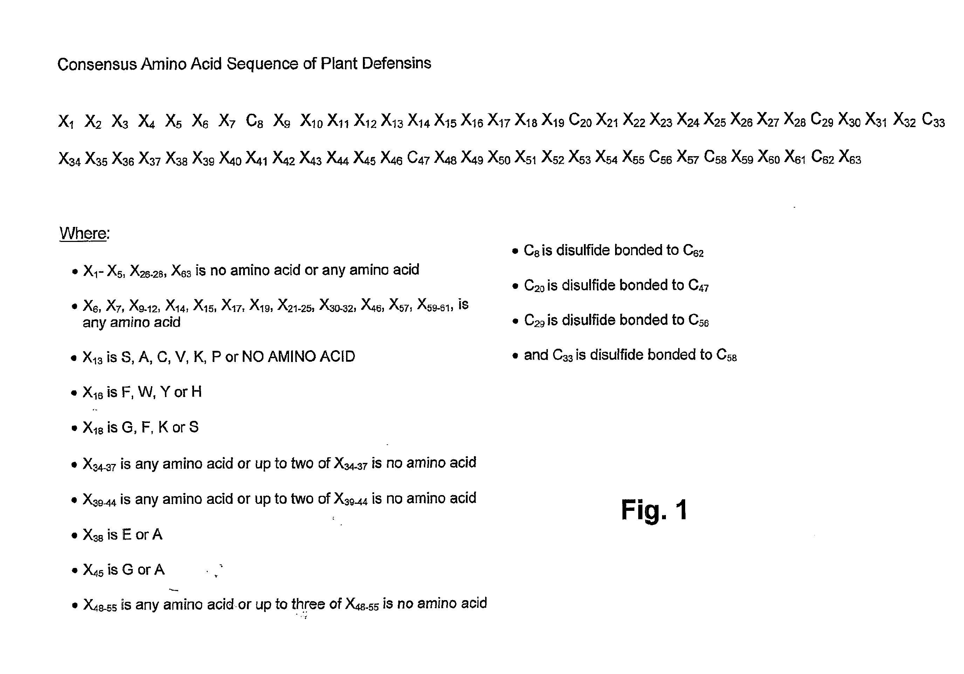

Plant defensins are small (∼5 kDa, 45 to 54 amino acids), basic, cysteine-rich (typically eight cysteine residues) proteins. The first members of this family were

isolated from the endosperm of barley (Mendez, E. et al. (1990) Eur. J. Biochem., 194:533-539) and wheat (Colilla, F.J. et al. (1990) FEBS Lett. 270:191-194) and were proposed to form a novel subclass of the thionin family (γ-thionins) that was distinct from the α- and β-subclasses (Bohlmann *et al.* (1994) *supra*). Thus, these barley and wheat proteins were named γ1-hordothionin (γ1-H) and γ1-and γ2-purothionin (γ1-P and γ2-P), respectively (Mendez *et al.* (1990) *supra*; Colilla *et al.* (1990) *supra).* Their original assignment as the γ-thionin subclass of the thionin family was based on similarities in size, charge and cysteine content to the α- and β-thionins, however the spacing of the cysteines was significantly different (Bohlmann (1994) *supra*; Mendez *et al.* (1990) *supra;* Colilla *et al.* (1990) *supra*) (Table 1, Fig. 1).

In subsequent years, numerous other γ-thionin-like proteins were identified, either as purified protein or deduced from cDNAs from both monocotyledonous and dicotyledonous plants (reviewed in Broekaert *et al.* (1997) and (1995) *supra*)*.* The name "plant defensin" was coined in 1995 by Terras and colleagues who isolated two antifungal proteins from radish seeds (Rs-AFP1 and Rs-AFP2) and noticed that these proteins were more related to insect defensins than to the plant thionins at the level of primary and three-dimensional structure (Terras, F.R.G. et al. (1995) Plant Cell 7:573-588).

Plant defensins have a widespread distribution throughout the plant kingdom and are likely to be present in most, if not all, plants (Broekaert (1997) and (1995) *supra;* Osborn, R.W. et al. (1995) FEBS Lett. 368:257-262; Osborn, R.W. et al. (1999) in Seed proteins (Shewry, P.R. and Casey, R. Eds.) pp. 727-751. Kluwer Academic Publishers, Dordrecht; Shewry, P.R. et al. (1997) Adv. Bot. Res. 26:135-192]. Most plant defensins have been isolated from seeds where they are abundant and have been characterized at the molecular, biochemical and structural levels (Broekaert et al. (1995) *supra;* Thomma, B.P.H.J. et al. (2003) Curr. Drug. Targets - Infect. Dis. 3:1-8). Defensins have also been identified in other tissues including leaves (Terras *et al.* (1995) *supra;* Kragh, K.M. et al. (1995) Mol. Plant-Microbe Interact. 8:424-434; Yamada S. et al. (1997) Plant Physiol. 115:314; Komori, T. et al. (1997) Plant Physiol. 115;314; Segura, A. et al. (1998) FEBS Lett. 435:159-162), pods (Chiang, C.C. et al. (1991) Mol. Plant-Microbe Interact. 4:324-331), tubers (Moreno, M. et al. (1994) Eur. J. Biochem 223:135-139), fruit (Meyer, B. et al. (1996) Plant Physiol. 112:615-622; Aluru, M. et al. (1999) Plant Physiol. 120:633; Wisniewski, M.E. et al. (2003) Physiol. Plant. 119:563-572), roots (Sharma, P. et al. (1996) Plant Mol. Biol 31:707-712), bark (Wisniewski *et al.* (2003) *supra*) and floral tissues (Lay et al 2003 *supra*; Moreno *et al.* (1994) *supra*; Gu, Q. et al. (1992) Mol. Gen. Genet. 234:89-96; Milligan, S.B. et al. (1995) Plant Mol. Biol. 28:691-711; Karunanandaa, B. et al. (1994) Plant Mol. Biol. 26:459-464; Li, H.-Y. et al. (1999) Plant Physiol. 120:633; Urdangarin, M.C. et al. (2000) Plant Physiol. Biochem. 38:253-258; van den Heuvel, K.J.P.T. et al. (2001) J. Exp. Bot. 52:1427-1436; Park, C.H. et al. (2001) Plant Mol. Biol. 50:59-69).

An amino acid sequence alignment of several defensins that have been identified, either as purified protein or deduced from cDNAs, has been published by Lay, *et al.* (2005) *supra.* Other plant defensins have been disclosed in U.S. Patent 6,911,577 issued June 28, 2003; International Publication WO 00/11196 issued March 2, 2000; and U.S. Patent 6,855,865 issued February 15, 2005. Plant defensins exhibit clear, although relatively limited, sequence conservation. Strictly conserved are the eight cysteine residues (numbering relative to Rs-AFP2). In most cases, two glycines (position 13 and 34), a serine (position 8), an aromatic residue (position 11) and a glutamic acid (position 29) are also conserved.

### Two classes of plant defensins

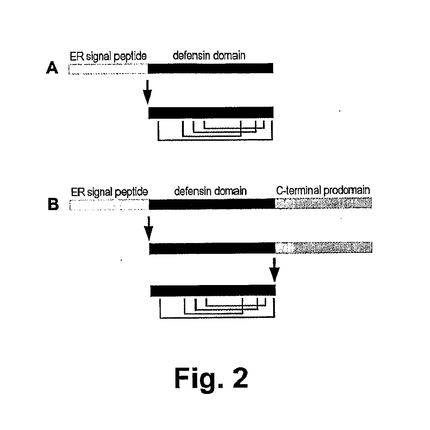

Plant defensins can be divided into two major classes according to the structure of the precursor proteins predicted from cDNA clones (Lay *et al* 2003 *supra*) (Fig_2A-2B). In the first and largest class, the precursor protein is composed of an endoplasmic reticulum (ER) signal sequence and a mature defensin domain. These proteins enter the secretory pathway and have no obvious signals for post-translational modification or subcellular targeting (Fig. 2A).

The second class of defensins are produced as larger precursors with C-terminal prodomains or propeptides (CTPPs) of about 33 amino acids (Fig. 2B). Most of these defensins have been identified in solanaceous species where they are expressed constitutively in floral tissues (Lay *et a*l 2003 *supra;* Gu *et al.* (1992) *supra*; Milligan, S.B. et al. (1995) Plant Mol. Biol. 28:691-711; Brandstadter, J. et al. (1996) Mol. Gen. Genet. 252:146-154) and fruit (Aluru *et al.* (1999) *supra*)*.* Moreover, defensin expression can also be induced by salt-stress in the leaves of some *Nicotiana* species (Yamada *et al.* (1997) *supra;* Komori *et al.* (1997) *supra*)*.* The prodomain of the solanaceous defensins from *Nicotiana alata* (NaD1) and *Petunia hybrida* (PhD1 and PhD2) is removed proteolytically during maturation (Lay *et al* 2003 *supra*)*.*

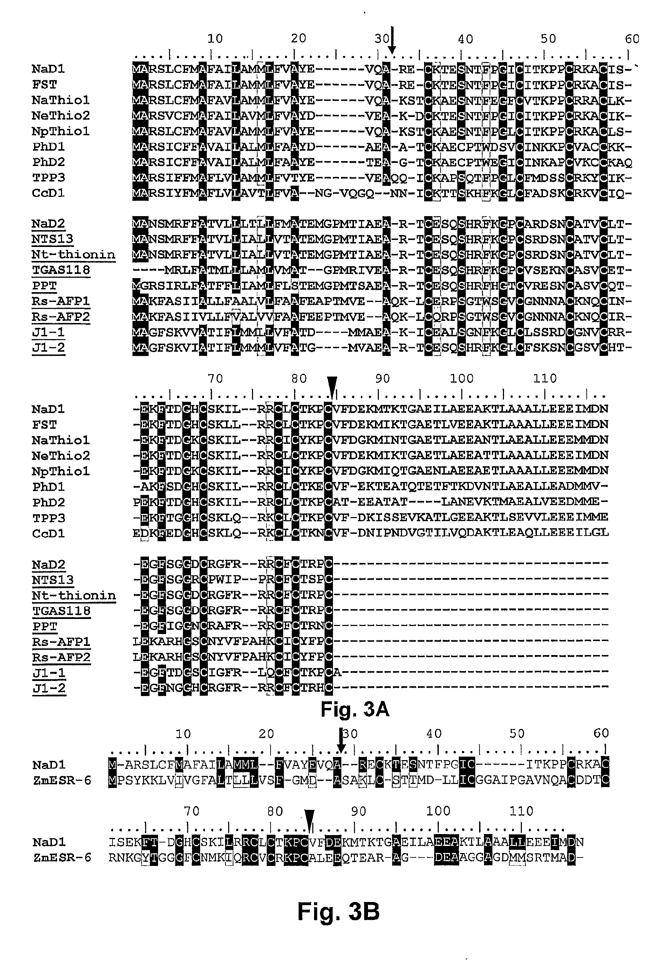

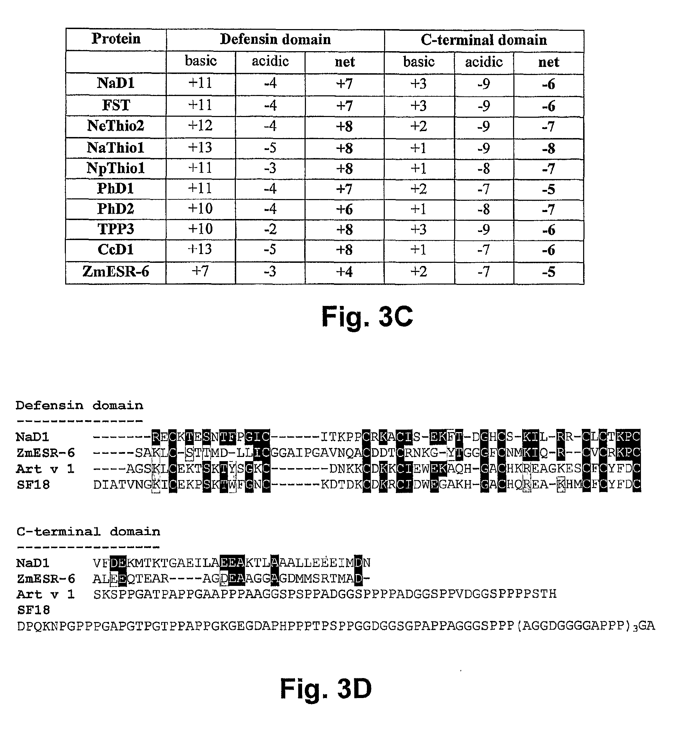

The C-terminal prodomains on the solanaceous defensins have an unusually high content of acidic and hydrophobic amino acids (refer to Fig. 3A-3D). Interestingly, at neutral pH, the negative charge of the prodomain counterbalances the positive charge of the defensin domain (Fig. 3C). This feature is reminiscent of the prodomains (also referred to as propieces or prosegments) present on the mammalian (Michaelson, D. et al. (1992) J. Leucoc. Biol. 51:634-639; Yount, N.Y. et al. (1995) J. Immunol. 155:4476-4484) and insect defensins (Lowenberger, C.A. et al. (1999) Insect Mol. Biol. 8:107-118), as well as the plant thionins (Bohlmann (1994) *supra*; Romero, A. et al. (1997) Eur. J. Biochem. 243:202-208). One difference, however, is that the prodomains in the mammalian and insect defensins are located on the N-terminal side of the defensin domain. Although defensins with C-terminal prodomains are most common in the Solanaceae, they are not restricted to this plant family. ZmESR-6 from *Zea mays* (Poaceae) encodes a defensin with a 28 amino acid C-terminal domain that is enriched in hydrophobic and acidic amino acids and is a potential vacuolar targeting signal (Fig. 3B, Fig. 3D). A sunflower (Asteraceae) defensin, Ha-DEF1, has also been identified that encodes a precursor protein with a C-terminal prodomain (de Zélicourt A. et al. (2007) Planta 226:591-600). Interestingly, its 30 amino acid C-terminal domain carries an unusual overall positive charge. The protein and DNA databases also describe sequences corresponding to another class of chimeric defensin proteins (Fig. 3D). These chimeric defensins have C-terminal domains of 50 amino acids or more, that are proline-rich and do not resemble vacuolar targeting signals (Fig. 3D).

Several possible role(s) for the C-terminal prodomain have been suggested. One hypothesis is that it may function as a targeting sequence for subcellular sorting. Such a function has been proposed for the prodomain of human neutrophil α-defensin 1 (HNP-1) where it may be important for normal subcellular trafficking and post-translational proteolytic processing Liu, L. et al. (1995) Blood 85:1095-1103]. Similarly, the prodomain on the plant thionins appears to have a role in vacuolar targeting and processing (Romero *et al.* (1997) *supra).*

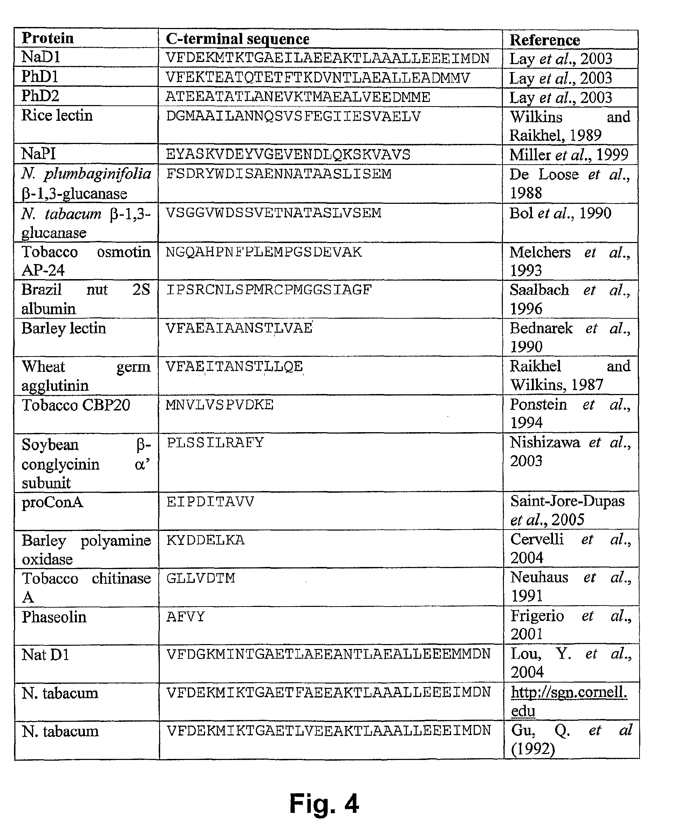

In immunogold electron microscopy experiments performed on ultra-thin sections of *N. alata* anthers and ovaries, Lay and colleagues (Lay *et al* 2003 *supra*) demonstrated that NaD1 was located specifically in the vacuole. This contrasts with the extracellular location of the well-studied seed defensins such as Rs-AFP2 (radish) and alfAFP (alfalfa) that lack the prodomain (Terras *et al.* (1995) *supra;* Gao, A.G. et al. (2000) Nat. Biotechnol. 18:1307-1310). Furthermore, while there are no consensus sequences that define C-terminal vacuolar sorting determinants (Nielsen, K.J. et al. (1996) Biochemistry 35:369-378; Neuhaus, J.-M. (1996) Plant Physiol. Biochem. 34:217-221), the high content of acidic and hydrophobic amino acids in the prodomains of the solanaceous defensins is consistent with other plant vacuolar sorting determinants (Lay *et al* 2003 *supra*) (Fig. 4). Furthermore, the prodomains contain motifs of four amino acids (shaded in Fig. 4) that are also present in the vacuolar targeting sequences from barley lectin and wheat germ agglutinin. The similarity between the vacuolar targeting motifs on tobacco defensins and barley lectin and wheat germ agglutinin suggests that the C-terminal propeptides from the solanacous defensins would function as vacuolar targeting signals in monocotyledous as well as dicotyledonous plants. The VFAE (SEQ ID NO:43) sequence in barley lectin (Fig. 4) is known to be sufficient for vacuolar targeting (Bednarek, S.Y. and Raikhel, N.V. (1992) Plant Mol Biol. 20:133-150). Other C-terminal sequences responsible for vacuolar targeting have been described (Shinshi et al Plant Molec. Biol. 14:357-368(1990)).

On the other hand, the disparity in the electrostatic charges associated with the defensin and the prodomain suggests that the prodomain could assist in the maturation of the defensin by acting as an intramolecular steric chaperone and/or by preventing deleterious interactions between the defensin and other cellular proteins or lipid membranes during translocation through the secretory pathway. These hypotheses have been proposed for the mammalian α-defensins, insect defensins and the thionins (Bohlmann (1994) *supra;* Michaelson *et al.* (1992) *supra;* Liu *et al.* (1995) *supra*; Florack, D.E.A. et al. (1994) Plant Mol. Biol. 26:25-37; Florack, D.E. et al. (1994) Plant Mol. Biol. 24:83-96).

### Defensins are expressed as multigene families

Plant defensins, like many other plant defense-related proteins, are encoded by multigene families. This is particularly well illustrated in *Arabidopsis thaliana* and *Medicago truncatula* where comparative sequence analysis of publically available sequence databases revealed that there are several hundred defensin-like (DEFL) genes present in these plants alone (Silverstein, K.A. et al. (2005) Plant Physiol. 138:600-610; Fedorova, M.J. et al. (2002) Plant Physiol. 130:519-537; Graham, M.A. et al. (2004) Plant Physiol. 135:1179-1197; Mergaert, P.K. et al. (2003) Plant Physiol. 132:161-173).

Expression studies with several *Arabidopsis* defensins have revealed that these genes display distinct organ-specific expression patterns (Epple, P. et al. (1997) FEBS Lett. 400:168-172; Thomma, B.P.H.J. et al. (1998a) Proc. Natl. Acad. Sci. U.S.A. 95:15107-15111; Thomma, B.P.H.J. et al. (1998b) Plant Physiol. Biochem. 36:553-537). Some are expressed constitutively, while others are up-regulated in leaves following pathogen infection (Epple *et al.* (1997) *supra*; Thomma *et al.* (1998-A) *supra*; Thomma *et al.* (1998-B) *supra*). As a result, most plant tissues constitutively express two or more .defensin genes, suggesting that individual defensins are expressed under specific circumstances or at specific sites.

### Purification of defensins

Over the last two decades, numerous plant defensins have been purified, particularly from seeds where the proteins are relatively abundant (Osborn *et al.* (1995) *supra*; Terras, F.R.G. et al. (1992) J. Biol. Chem. 267:15301-15309). While several different methods have been reported for defensin purification, many of these rely on the intrinsic physico-biochemical properties of the protein such as their small size, overall net positive charge, tolerance to acids and organic solvents, and their thermostability. Similarly, purification of other small, basic, cysteine-rich proteins such as the thionins (Bohlmann (1994) *supra*) and the plant cyclotides (Craik et al. (1999) *supra*) has exploited these properties. This is reflected in the use of mild acids (e.g. 50 mM sulfuric acid) (Lay *et al* 2003 *supra*; Ozaki, Y. et al. (1980) J. Biochem. 187:549-555; Zhang, N.Y. et al. (1997-A) Cereal Chem. 74:119-122; Zhang, Y. and Lewis, K. (1997-B) FEMS Microbiol. Lett. 149:59-64) or organic solvents (Craik *et al.* (1999) *supra*) in the initial extraction, heating of the samples to remove heat labile proteins (Lay et al 2003 *supra*; Terras *et al.* (1992) *supra*; Saitoh, H. et al. (2001) Mol. Plant-Microbe Interact. 14:111-115) and a combination of various chromatographic steps including gel (size exclusion) filtration, ion-exchange and reverse-phase high performance liquid chromatography (Lay *et al* 2003 *supra* ; Terras *et al* (1992) *supra*; Zhang *et al.* (1997a) *supra*; Zhang and Lewis (1997-B) *supra*)*.*

Heterologous expression systems have been used for producing defensins in quantity. Vilas Alves, A.L. et al. (1994) FEBS Lett. 348:228-232 produced functional Rs-AFP2 *in S. cerevisiae,* while Kristensen, A.K. et al. (1999) Protein Expr. Purif. 16:377-387, Almeida, M.S. et al. (2001) Arch. Biochem. Biophys. 395:199-207 and Wisniewski, M.E. et al. (2003) Physiol. Plant. 119:563-572 expressed a sugar beet (AX2), a pea (Psd1) and a peach (PpDfn1) defensin in *Pichia pastoris,* respectively. In a more novel approach, Saitoh *et al supra.* produced a wasabi defensin (WT1) in the leaves of *Nicotiana benthamiana* by infecting the plants with a potato virus X vector carrying the *WTI* cDNA. In 2002, Chen and colleagues used an intein-based system to express a mung bean defensin (VrCRP) in *Escherichia coli* (Chen, K.C. et al. (2002) J. Agric. Food Chem. 50:7258-7263).

### Biological activity of plant defensins

A wide range of biological activities have been attributed to plant defensins including growth inhibitory effects on a broad range of fungi (Broekaert *et al.* (1997) *supra*; Lay *et al* 2003 *supra*; Osborn et al. (1995) *supra*; Terras et al. (1993) FEBS Lett. 316:233-240), and Gram-positive and Gram-negative bacteria (Segura *et al.* (1998) *supra;* Moreno *et al.* (1994) *supra*; Zhang *et al.* (1997-B) *supra*)*.* Some defensins are also effective inhibitors of digestive enzymes such as α-amylases (Zhang *et al.* (1997a) *supra*; Bloch C. Jr. et al. (1991) FEBS Lett. 279:101-104) and serine proteinases (Wijaya, R. et al. (2000) Plant Sci. 159:243-2555; Melo, F.R. et al. (2002) Proteins 48:311-319), two functions consistent with a role in protection against insect herbivory. This is supported by the observation that bacterially expressed mung bean defensin, VrCRP, is lethal to the bruchid *Callosobruchus chinensis* when incorporated into an artificial diet at 0.2% (w/w) (Chen *et al*. (2002) *supra*)*.* Some defensins also inhibit protein translation (Mendez *et al.* (1990) *supra*; Colilla *et al.* (1990) *supra;* Mendez, E. et al. (1996) Eur. J. Biochem. 239:67-73) or bind to ion channels (Kushmerick, C. et al. (1998) FEBS Lett. 440-302-306) (Table 2). A defensin from *Arabidopsis halleri* also confers zinc tolerance, suggesting a role in stress adaptation (Mirouze, M.J. et al. (2007) Plant J. 47:329-342). More recently, a sunflower defensin was shown to induce cell death in *Orobanche* parasite plants (de Zélicourt *et al.* (2007) *supra*)*.* Intriguingly, individual defensins exhibit one or two, but not all of these properties.

### Antifungal activity

The best characterized activity of plant defensins is their ability to inhibit, with varying potencies, a large number of fungal species (for examples, see (Broekaert *et al.* (1997) *supra*; Lay *et al* 2003 *supra;* Osborn *et al.* (1995) *supra*)*.* Rs-AFP2, for example, inhibits the growth of *Phoma betae* at 1 µg/mL, but is ineffective against *Sclerotinia sclerotiorum* at 100 µg/mL (Terras *et al.* (1992) *supra*)*.* Based on their effects on the growth and morphology of the fungus, *Fusarium culmorum,* two groups of defensins can be distinguished. The "morphogenic" plant defensins cause reduced hyphal elongation with a concomitant increase in hyphal branching, whereas the "non-morphogenic" plant defensins reduce the rate of hyphal elongation, but do not induce marked morphological distortions (Osborn *et al.* (1995) *supra*)*.*

Many defensins display antifungal activities, but the molecular basis for such activity has been elucidated for only four defensins. The DmAMP1 and RsAFP2 defensins bind to distinct sphingolipid targets in fungal membranes and consequently show different specificity against these fungi. The gene (*IPT1*) encoding inositol phosphotransferase was identified as determining sensitivity to DmAMP1 in *Saccharomyces cerevisiae.* This enzyme catalyses the last step of the biosynthesis of the sphingolipid mannosyldiinositolphosphoceramide (M(IP)₂C). Mutant strains that lacked a functional *IPT1* gene were devoid of M(IP)₂C in their plasma membrane, bound less DmAMP1 compared to wild-type strains and became highly resistant to DmAMP1-mediated inhibition. For RsAFP2, the gene in *Pichia pastoris* that determines sensitivity is GCS which encodes for glucosylceramide synthase, an enzyme involved in the synthesis of glucosylceramide. See Lay, F.T. *et al.* (2005) *supra;* Thevissen, K. et al. (2003) FEBS Microbiol. Lett. 226:169-173; Thevissen K. et al. (2004) J. Biol. Chem. 279:3900-3905.

More recently, the pea defensin Psd1 has been shown to be taken up intracellularly and enter the nuclei of *Neurospora crassa* where it interacts with a nuclear cyclin-like protein involved in cell cycle control (Lobo, D.S. et al. (2007) Biochemistry 46:987-96). For MsDef1, a defensin from alfalfa, two mitogen-activated protein (MAP) kinase signalling cascades have a major role in regulating MsDef1 activity on *Fusarium graminearum* (Ramamoorthy, V. et al. (2007) Cell Microbiol. 9:1491-506).

### Exploitation of plant defensins in transgenic plants

To date, several plants have been transformed with plant defensin genes. A list of these genes, their recipient plants and target pathogens is presented in Table 2. Constitutive expression of the radish defensin (Rs-AFP2) enhanced resistance of tobacco plants to the fungal leaf pathogen *Alternaria longipes* and similarly in tomato to *A*. *solani* (Ohtani, S. et al. (1977) J. Biochem. (Tokyo) 82:753-7657). Canola (*Brassica napus*) constitutively expressing a pea defensin had slightly enhanced resistance against blackleg (*Leptosphaeria maculans*) disease (Wang, Y. et al. (1999) Mol. Plant-Microbe Interact. 12:410-418). However, the most extensively studied and best example of the potential of defensins in transgenic crops comes from the work of Gao and colleagues on the alfalfa defensin (alfAFP) in potatoes (Gao *et al.* (2000) *supra*).

**TABLE 2.**

| **Plant defensins in transgenic plants.** | | | | |
|---|---|---|---|---|
| **Transgene** | **Source plant** | **Recipient plant(s)** | **Promoter** | **Increased resistance against test organism(s)** |
| *Rs-AFP2* | Radish | Tobacco | Cauliflower mosaic virus (CaMV) 35S | *Alternaria longipes* |
| *Rs-AFP2* | Radish | Tomato, oil rape | CaMV 35S | *A. solani, Fusarium oxysporum, Phytophthora infestans, Rhizoctonia solani, Verticillium dahliae* |
| *AIfAFP* | Alfalfa | Potato | Figwort mosaic virus 35S | *V*. *dahliae* |
| *Spi1* | Norway spruce | Tobacco, Norway spruce embryonic cultures | CaMV 35S | *Erwinia carotovora, Heterobasidion annosum* |
| *DRR230-a* | Pea | Canola | CaMV 35S | *Leptoshaeria maculans* |
| *DRR230-a* | Pea | Tobacco | Duplicated CaMV 35S | *F. oxysporum, Asochyta pinodes, Trichoderma reesei, Ascochyta lentis, F. solani, L. maculans, Ascochyta pisi, Alternaria alternata* |
| *DRR230-c* | | | | |
| *BSD1* | Chinese cabbage | Tobacco | CaMV 35S | *P. parasitica* |
| *WT1* | Wasabi | Rice | Maize ubiquitin-1 | *Magnaporthe grisea* |
| *WTI* | Wasabi | *Phalaenopsis* orchid | CaMV 35S | *Erwinia carotovora* |
| *WTI* | Wasabi | Potato | CaMV 35S | *Botrytis cinerea* |
| *DmAMP1* | Dahlia | Eggplant | CaMV 35S | *Botrytis cinerea, Verticillium alboatrum* |
| *DmAMP1* | Dahlia | Papaya | CaMV 35S | *Phytophthora palmivora* |

Lay and Anderson (2005) Current Protein and Peptide Science 6:85-101 .
Sjahril1 R, Chin DP, Khan RS, Yamamura S, Nakamura I, Amemiya Y, Mii1 M (2006) Plant Biotechnology 23:191-194.
Khan RS, Nishihara M, Yamamura S, Nakamura I, Mii M (2006) Plant Biotechnology 23:179-183.
Turrini A, Sbrana C, Pitto L, Ruffini Castiglione M, Giorgetti L, Briganti R, Bracci T, Evangelista M, Nuti MP, Giovannetti M (2004) New Phytologist 163:393-403.
Zhu YJ, Agbayani R, Moore PH (2007) Planta 226:87-97.

Gao and colleagues (Gao *et al.* (2000) *supra*) demonstrated that constitutive expression of alfAFP (also known as MsDef1) in potatoes provided a robust resistance against the agronomically important fungus *Verticillium dahliae.* Levels of fungus in the transformed plants were reduced by approximately six-fold compared to the non-transformed plants. The protection conferred by the alfAFP transgene was not only maintained under glasshouse conditions, but also in the field and over several years at different geographical sites (Gao *et al.* (2000) *supra*)*.* Furthermore, the level of Verticillium wilt resistance in the transgenic plants was equal to, or greater than, the level of resistance obtained with non-transgenic plants grown in fumigated, non-infested soil (Gao *et al.* (2000) *supra*)*.*

U.S. Patent 7,041,877, incorporated herein by reference to the extent consistent herewith, reported transgenic expression of full-length NaD1 in cotton and tobacco. Leaves of resulting transformed plants were fed to *Helicoverpa armigera* and *H. punctigera* larvae, resulting in growth inhibition compared to larvae fed on control diets. U.S. Patent 7,041,877 also reported that purified NaD1 (minus the C-terminal prodomain) inhibited growth of *Fusarium oxysporum f. sp. dianthi* Race 2 and *Botrytis cinerea in vitro.*

### DESCRIPTION OF THE DRAWINGS

Fig. 1 is a generic sequence of plant defensins based on Lay and Anderson 2005.
Fig. 2A and Fig 2B diagrammatically illustrate the two classes of plant defensins. Fig. 2A: All plant defensins are produced with an ER signal sequence in addition to the mature defensin domain. Fig. 2B: In some plants, particularly those from the solanaceae, cDNA clones have been isolated that encode plant defensins with an additional C-terminal prodomain. The four strongly conserved disulfide bonds in the defensin domain are illustrated by connecting lines.
Figs. 3A-3D provide a comparison of predicted amino acid sequences of defensin precursor proteins with and without C-terminal prodomains. Fig. 3A: The predicted cleavage site of the ER signal sequence is indicated with an arrow and the junction between the defensin and C-terminal prodomains (where applicable) is marked with a triangle. Spaces have been introduced to maximize the alignment. Residues that are similar or identical in some or most of the sequences are shaded in grey and black, respectively. The alignment was generated using the BioEdit sequence alignment editor (version 5.0.9) software (Hall, T.A. (1999) Nucl. Acids Symp. 41:95-98). The references for the protein sequences are NaD1 (SEQ ID NO:1), PhD1 (SEQ ID NO:2) and PhD2 (SEQ ID NO:3) (Lay, F.T. et al. (2003) Plant Physiol. 131:1283-1293), FST (SEQ ID NO:4) (Gu Q., et al. (1992) Mol Gen Genet 234:89-96), NaThio1 (SEQ ID NO:5) (Lou, Y. and Baldwin, I.T. (2004) Plant Physiol. 135:496-506), NeThio2 (SEQ ID NO:6) (Yamada, S. et al. (1997) Plant Physiol.115:314), NpThio1 (SEQ ID NO:7) (Komori, T. et al. (1997) Plant Physiol. 115:314), TPP3 (SEQ ID NO:8) (Milligan, S.B. and Gasser, C.S. (1995) Plant Mol Biol. 28:691-711, CcD1 (SEQ ID NO:9) (AF128239, Aluru, M. et al. (1999) Plant Physiol. 120:633), Nt-thionin (SEQ ID NO:10) (accession number BAA95697), NTS13 (SEQ ID NO:11) (X99403) Li, H.Y. and Gray, J.E. (1999) Plant Physiol. 120:663), TGAS118 (SEQ ID NO:12) (Van den Heuvel, K.J.P.T. et al. (2001) J Expt. Biol. 52:1427-1436), PPT (SEQ ID NO:13) (Karunanandaa, B. et al. (1994) Plant Mol Biol. 26:459-464), J1-1 (SEQ ID NO:14) and J1-2 (SEQ ID NO:15) (Meyer, B. et al. (1996) Plant Physiol. 112:615-622), Rs-AFP1 (SEQ ID NO:16) and Rs-AFP2 (SEQ ID NO:17) (Terras, F.R.G. et al. (1992) J.Biol.Chem. 267:15301-15309). NaD2 corresponds to SEQ ID NO:33.
Fig. 3B: Comparison of the amino acid sequence of a solanaceous defensin NaD1 (SEQ ID NO: 1) with ZmESR-6 (SEQ ID NO:18), a prodefensin with a C-terminal propeptide from *Zea mays* (Balandin et al, (2005) Plant Mol Biol. 58:269-282). An arrow marks the N-terminus of the mature defensin domain and the triangle indicates the N-terminus of the C-terminal propeptide for the aligned sequences.
Fig. 3C: Distribution of charged amino acids in the defensin and C-terminal prodomains of the solanaceous and *Zea mays* defensins at neutral pH. (Modified from Lay and Anderson 2005). Portions of NaD1, ZmESR-6, Art v1 and SF18 are from SEQ ID NOs:1, 18, 19 and 20.
Fig. 3D: Alignment of the mature defensin (SEQ ID NO:1, residues 26-72; SEQ ID NO:18, residues 24-78) and C-terminal domains (SEQ ID NO:1, residues 73-105; SEQ ID NO:18, residues 79-106) of NaD1 and ZmESR-6, respectively, with the major allergen of mugwort pollen Art v1 (SEQ ID NO:19, residues 1-53 mature defensin) (AF493943, Himly, M. et al, (2003) FASEB J 17:106-108) and the defensin-like protein from sunflower SF18 (SEQ ID NO:20, residues 1-57 mature defensin) (X53375, Domon, C. et al. (1990) Plant Mol Biol 15:643-646). Both Art v1 (SEQ ID NO:19) and SF18 (SEQ ID NO:20) have long proline-rich C-terminal domains (SEQ ID NO:19, residues 53-108; SEQ ID NO:20, residues 58-152).
Fig. 4 provides a comparison of the C-terminal prodomains from the solanaceous defensins NaD1 (SEQ ID NO:1, residues 73-105), PhD1 (SEQ ID NO:2, residues 73-103) and PhD2 (SEQ ID NO:3, residues 75-101) with C-terminal prodomains from the other plant proteins that are essential for vacuolar targeting (SEQ ID NOs:21-31, 35, 40 and 41). The shaded sequences highlight motifs that are present in the C-terminal propeptide from NaD1 as well as those from barley lectin and wheat germ agglutinin. The four amino acid peptide VFAE (amino acids 1-4 of SEQ ID NO: 24) from barley lectin is sufficient for vacuolar targeting (Bednarek, S.Y. and Raikhel, N.V. (1992) Plant Mol. Biol. 20:133-150).
   The references for the sequences shown in Figs. 1-4 are Bednarek, S. et al. (1990) Plant Cell 2:1145-1155; Bol, J. F. et al. (1990) Annu Rev Phytopathol 28:113-138; Cervelli, M. et al. (2004) Plant J 40:410-418; DeLoose, M., et al. (1988) Gene 70:13-23; Frigerio, L. et al. (2001) J Plant Physiol. 158:499-503; Lay, F. T. et al. (2003) Plant Physiol 131:1283-1293; Lou, Y. and Baldwin, I.P. (2004) Plant Physiol. 136 :496-506 ; Melchers, L. S. et al. (1993) Plant Mol Biol 21:583-593; Miller, E. A. et al. (1999) Plant Cell 11:1499-1508; Neuhaus, J.-M. et al. (1991) Proc Natl Acad Sci USA 88:10362-10366; Nishizawa, K. et al. (2003) Plant J 34:647-659; Ponstein, A.S. et al. (1994) Plant Physiol 104:109-118; Raikhel, N. and Wilkins, T. (1987) Proc Natl Acad Sci USA 84:6745-6749; Saalbach, G. et al. (1996) Plant Physiol 112:975-985; Saint-Jore-Dupas, C. et al. (2005) Plant Cell Physiol 46:1603-1612; Wilkins, T. and Raikhel, N. (1989) Plant Cell 1:541-549.

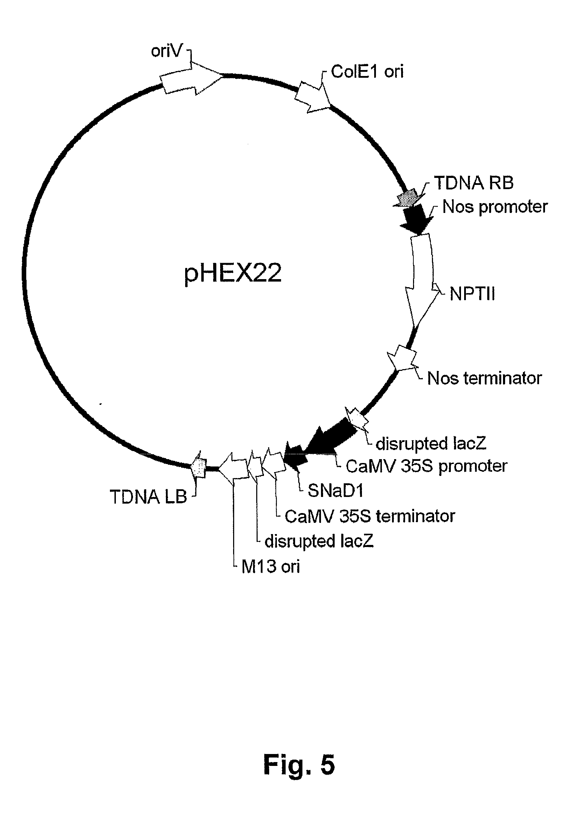

Fig. 5 is a diagram of the pHEX22 construct. (Example 1) The DNA was inserted between the left and right borders of the binary vector pBIN19 (Bevan M. (1984, Nucleic Acids Research 12: 8711-8721)). 99 bp of the NaD1 gene was removed from the C-terminus to produce the NaD1m (SMΔT) gene encoding SEQ ID NO: 1, residues 1-72. Abbreviations in clockwise order are:
   oriV; origin of vegetative replication;
   ColE1 ori: replication origin derived from colicin E1;
   TDNA RB: right border of Agrobaccterium tumefacious TDNA;
   Nos promoter: promoter of nopaline synthase Nos gene;
   NPTII: genetic sequence encoding neomycin phosphotransferase II;
   Nos terminator: terminator sequences of Nos gene;
   Disrupted lacZ: DNA segment encoding partial sequence of ß-galactosidase;
   CaMV 35S promoter: promoter of Cauliflower mosaic virus (CaMV) 35S protein;
   SNaD1: DNA encoding NAD1 lacking a CTPP (SMΔT);
   CaMV 35S terminator: terminator sequence of genes encoding Ca MV 35S protein;
   M13 ori: origin of M13 virus replication;
   TDNA LB: TDNA left border;
   All arrows indicate direction of transcription.

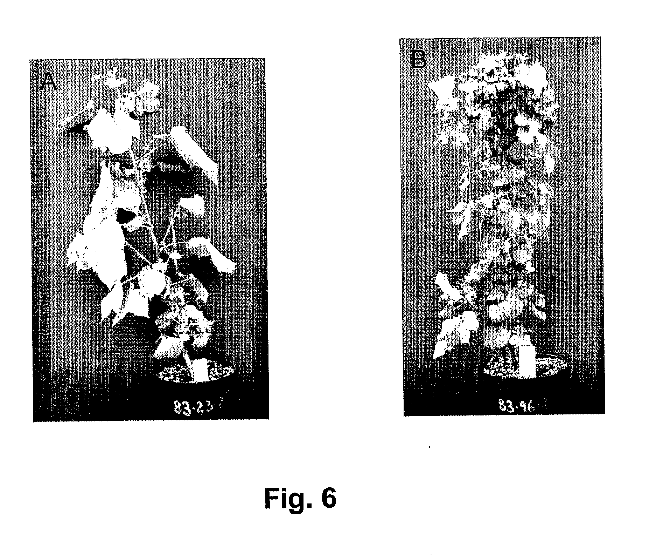

Fig. 6 shows photos of primary transgenic plants 83.23.2 (A) and 83.96.2 (B) transformed with pHEX22. (Example 1) Plant 83.96.2 has a higher level of NaD1 expression and displays a more abnormal phenotype with distorted leaves and short internodes.

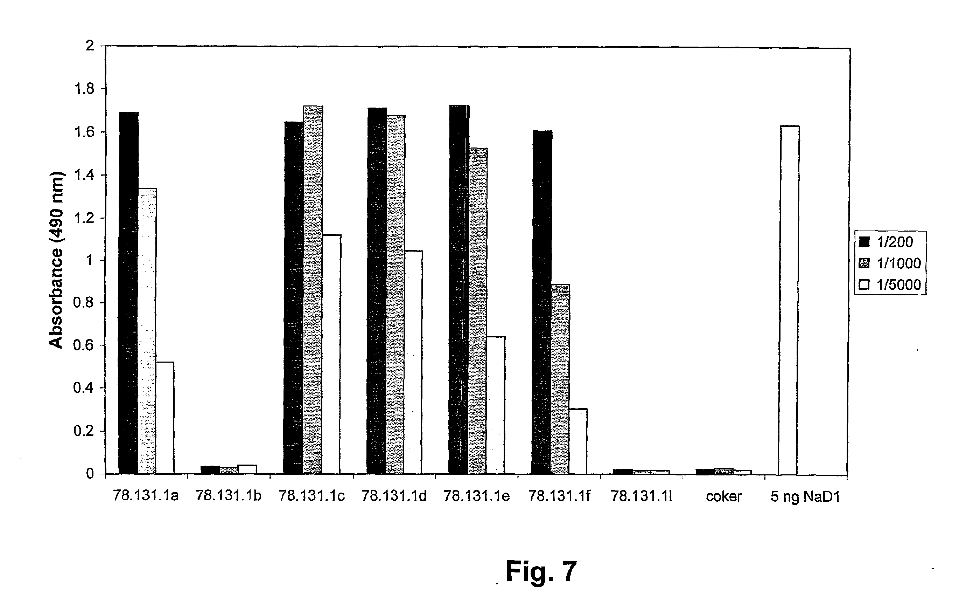

Fig. 7 is a bar graph showing relative NaD1 levels as determined by ELISA in the leaves of the T2 generation of line 78.131.1 transformed with pHEX22. Plants b and I are null segregants. (Example 1) Numbers on horizontal axis refer to progeny of primary transgenic plant 78.131.1. Absorbance at 490nm refers to ELISA assay data as described in Example 1.

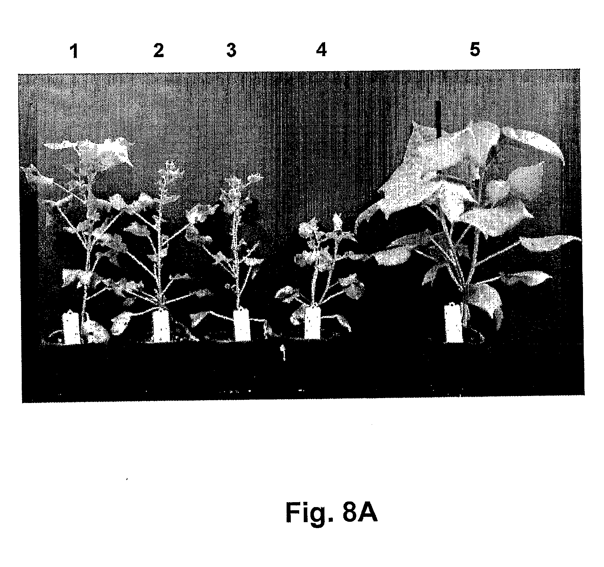

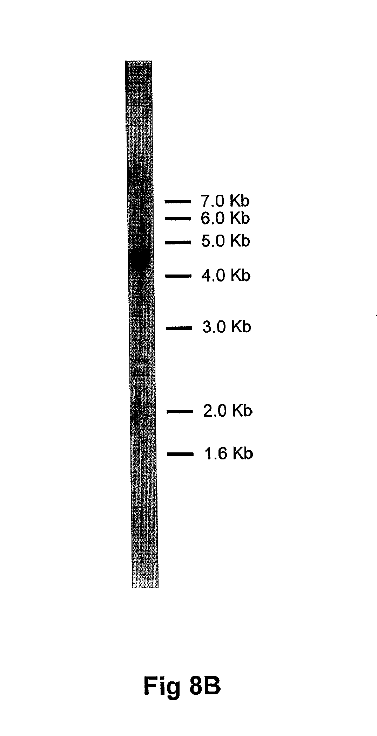

Fig. 8 is a photo depicting the phenotype of some of the line 78.131.1 T2 plants analysed in Fig. 7. Lane 1: plant a, lane 2: plant c, lane 3: plant d, lane 4: plant e (all plants expressed NaD1 minus CTPP, SMΔT) (SEQ ID NO:1, residues 1-72), lane 5: plant b (null segregant). (Example 1). Fig 8B. Southern blot of Vcl1 digested genomic DNA from 78.131.1 T1 plant showing presence of single DNA fragment that hybridized with the NaD1 DNA probe.

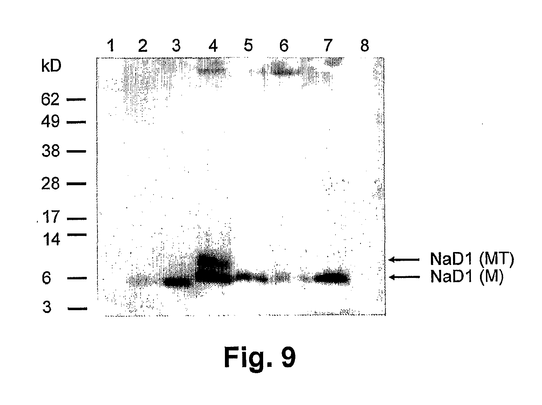

Fig. 9 depicts an immunoblot of total soluble protein extracted from leaves from cotton plants transformed with pHEX22. (Example 1) Proteins were separated on a 10-20% Novex™ (Invitrogen, Carlsbad, CA 92008) Tricine SDS gel and transferred onto a 0.22 micron nitrocellulose membrane. Lane 1: SeeBlue Plus2™ (Invitrogen, Carlsbad, CA 92008) protein standards, numbers on vertical axis indicate protein molecular mass in kilodaltons (kD), lane 2: 50 ng mature NaD1 (M) (SEQ ID NO:1, residues 26-72), lane 3: 150 ng mature NaD1 (M), lane 4: 35.125.1 (homozygous T4 generation), lane 5: 78.131.1 (T2 generation), lane 6, 83.68.2 (T2 generation), lane 7: 83.68.2 (T2 generation), lane 8: untransformed Coker 315 (Department of Primary Industries and Fisheries, Queensland). Mature NaD1 (M, SEQ ID NO:1, residues 26-72) (arrow) was detected in lanes 4, 5, 6 and 7. NaD1 with the C-terminal propeptide (MT, SEQ ID NO:1, residues 26-105) (arrow) was detected in lane 4 (35.125.1).

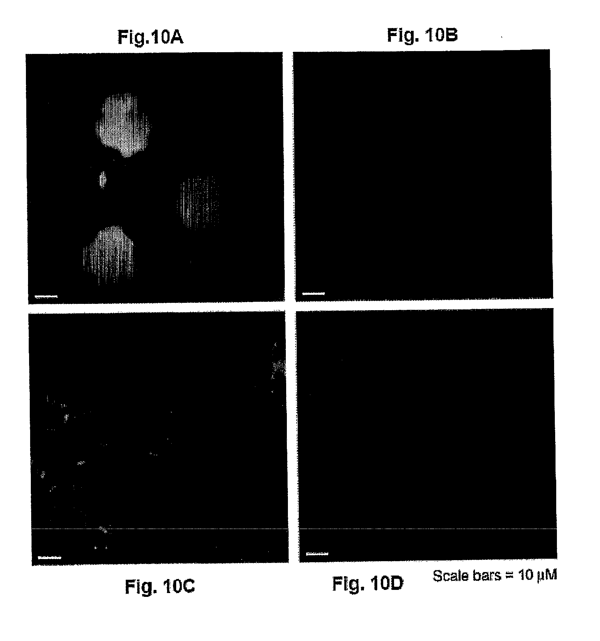

Figs. 10A-10D are photomicrographs showing sub-cellular location of NaD1 in stably transformed cotton plants expressing NaD1 from constructs encoding NaD1 with (pHEX3) and without (pHEX22) the NaD1 CTPP (T). (Example 1) NaD1 location was determined using immunofluorescence with the anti-NaD1 antibody on paraffin embedded leaf sections. A and B: Leaf sections from line 35.125.1 (pHEX3, SMT). A. Anti-NaD1 antibody showing location of NaD1 in the vacuole. B. Pre-immune antibody control. C and D: Leaf sections from line 78.131.1 (pHEX22, SMΔT). C. Anti-NaD1 antibody showing NaD1 is not in the vacuole but can be detected in the cytoplasm and extracellular space (arrowed). D. Pre-immune antibody control.

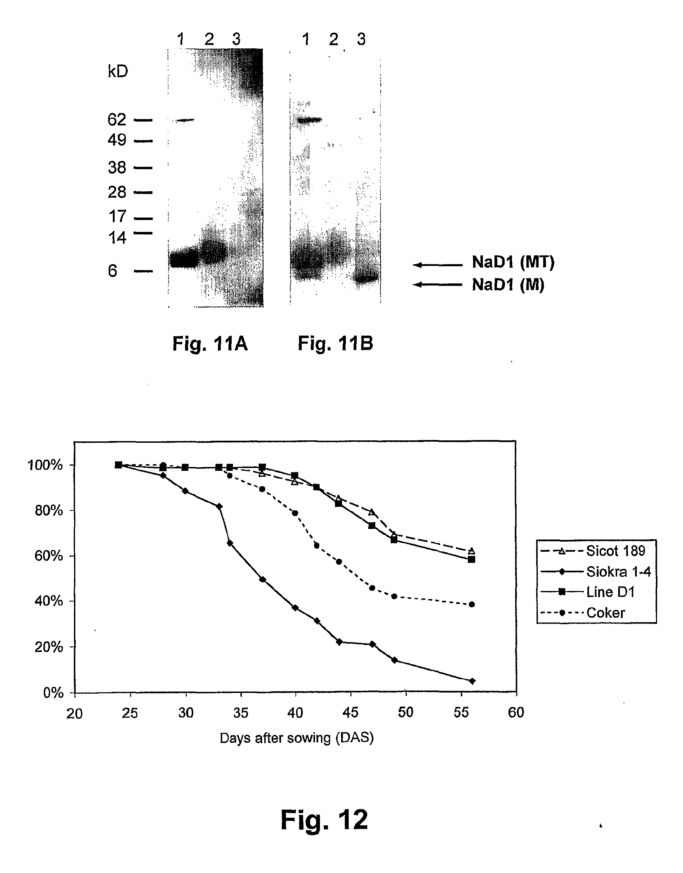

Fig. 11 depicts an immunoblot of protein extracted from immature buds of *N. alata* (lane 1) and leaves of transgenic cotton line 35.125 (lane 2). Lane 3 contains 50 ng mature NaD1 (M) (SEQ ID NO:1, residues 26-72). (Example 2) Proteins were separated on a 10 - 20% Novex™ (Invitrogen, Carlsbad, CA 92008) Tricine SDS - polyacrylamide gel and transferred onto a 0.22 micron nitrocellulose membrane. A: Blot probed with NaD1 CTPP (T) antibody, B: Blot probed with NaD1 (M) antibody. NaD1 with the NaD1 CTPP (T) (MT, SEQ ID NO:1, residues 26-105) (arrow) was detected in lanes 1 and 2. Mature NaD1 (M, SEQ ID NO:1, residues 26-72) (arrow) was detected in lanes 1 and 3 (panel B). Vertical axis depicts band positions of molecular weight standards.
Fig. 12 is a graph of percent survival of cotton plants infected with *Fusarium oxysporum* f. sp. *vasinfectum* (Fov) (vertical axis) plotted against days after sowing, in a glasshouse bioassay. (Example 3) Coker: untransformed Coker 315; Line D1: transgenic line 35.125.1 (Coker 315 transformed with pHEX3, SMT); Siokra 1-4 (Cotton Seed Distributors Limited, Wee Waa, NSW Australia 2388): Fov susceptible cotton variety: Sicot 189™ (Cotton Seed Distributors Limited, Wee Waa, NSW Australia 2388). Fov less-susceptible variety, Australian Industry Standard.

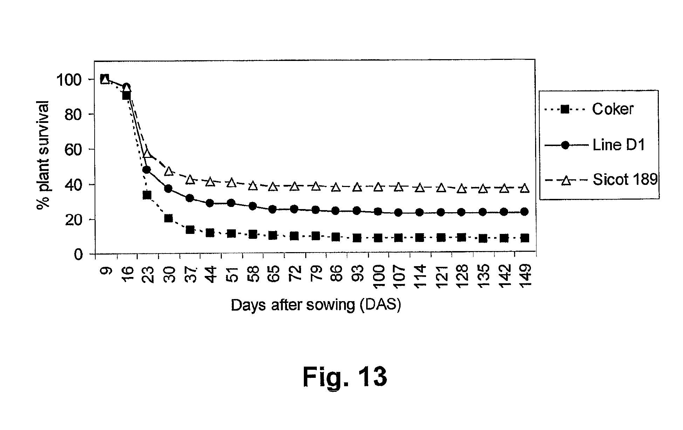

Fig. 13 is a graph of the field trial results (Example 3) for transgenic cotton expressing NaD1. Percent survival of cotton plants infected with *Fusarium oxysporum* f. sp. *vasinfectum* (Fov) (vertical axis) is plotted against days after sowing in the field. Coker: untransformed Coker 315; Line D1: transgenic line 35.125.1 Coker 315 transformed with pHEX3, SMT; Sicot 189: Fov less-susceptible variety, Australian Industry Standard.

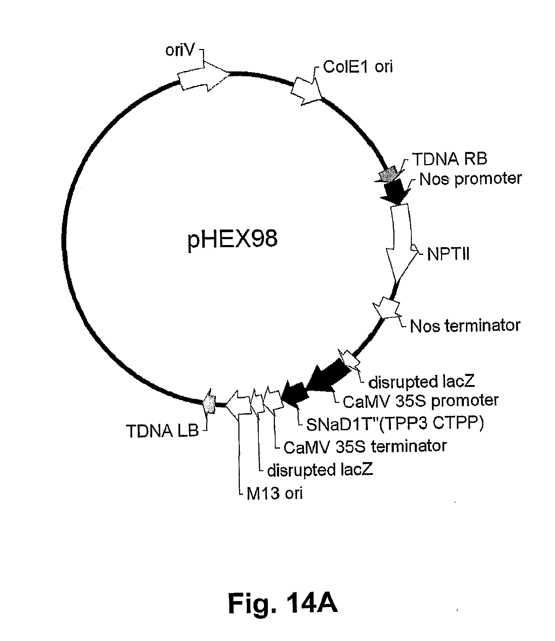

Figs. 14A-14E illustrate expression of a chimeric defensin composed of the NaD1 mature domain and the CTPP from a tomato defensin (Example 4). A: Is a diagram of the pHEX98 construct. DNA encoding mature NaD1 (M) with the NaD1 ER signal sequence (S) and the CTPP (T") from the tomato defensin TPP3 (Fig. 3) was inserted between the CaMV 35S promoter and terminator and ligated between the left and right borders of the binary vector pBIN19 (Bevan M. (1984, Nucleic Acids Research 12: 8711-8721)); other abbreviations as described for Fig. 5. B: Diagram of the pHEX98 protein product. C: Relative levels of NaD1 produced during transient expression of pHEX43 (SMT) and pHEX98 (SMT") in the leaves of tomato seedlings assayed by ELISA. Extracts (1:20, fresh weight:buffer) from three leaf samples were used in the ELISA. D: Relative levels of NaD1 by ELISA produced during transient expression in cotton cotyledons of pHEX98 (SMT"); pHEX43 (SMT); pHEX80 (S'MT') and pHEX89 (SMT'). See Table 10 for pHEX construct list. E: Protein immunoblot showing NaD1 (M) and NaD1 (MT) produced during stable or transient expression of the pHEX constructs in cotton.

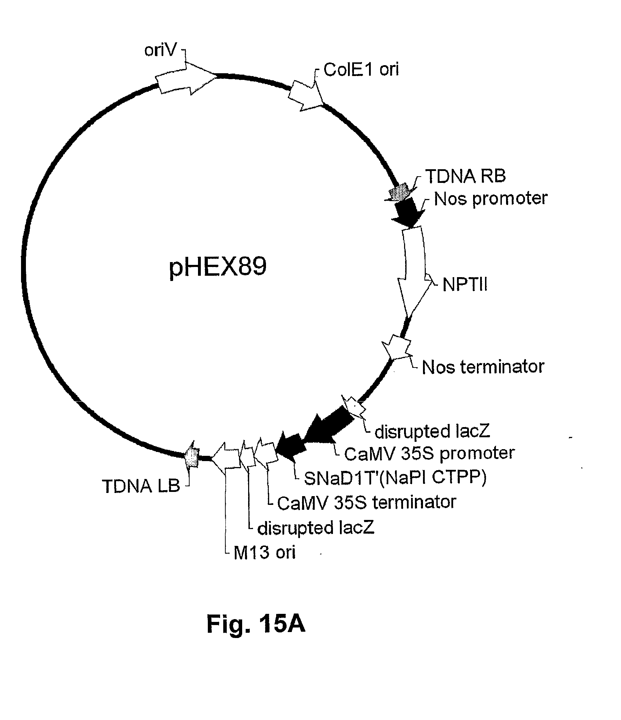

Figs. 15A-15D illustrate expression of a chimeric defensin composed of the NaD1 mature domain and the CTPP from the multidomain proteinase inhibitor NaPI (Example 5). A: Is a diagram of the pHEX89 construct containing DNA encoding mature NaD1 (M) with the NaD1 ER signal sequence (S) and the CTPP (T') from NaPI in a binary vector; other abbreviations as detailed in Fig. 5. B: The pHEX89 protein product. C: A diagram of pHEX80 containing NaD1 (M) domain with the ER signal sequence (S') and CTPP (T') from NaPI in a binary vector; other abbreviations as detailed in Fig. 5. D: The pHEX80 protein product.

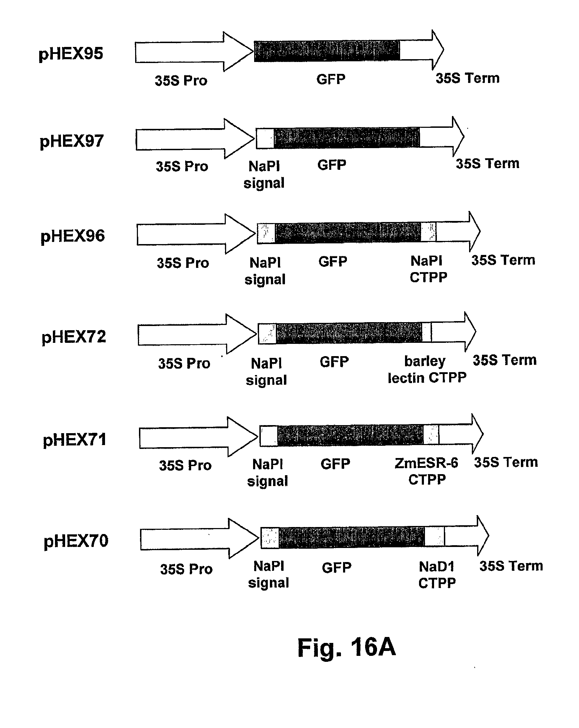

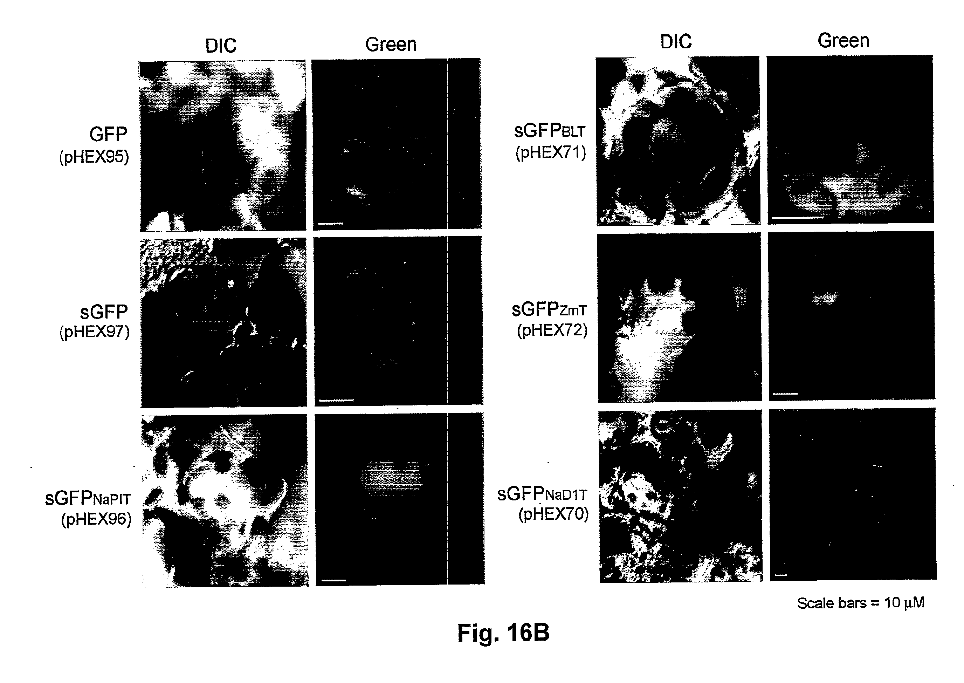

Fig. 16A and 16B diagram gene constructs (A) and photomicrographs (B) of studies showing the location of Green Flourescent Protein (GFP) in *N*. *benthamiana* cells after transient expression of various GFP-CTPP chimeras. (Example 5) A: Diagram of gene constructs encoding a series of GFP-CTPP chimeras to assess whether the CTPP sequences are sufficient to direct a protein to the plant vacuole. The constructs were inserted into the binary vector pBIN19 for expression in plant cells B: Micrographs showing location of GFP produced during transient expression of the constructs shown in A in the leaves of *N*. *benthamiana.* Left hand panels: Differential interference contrast (DIC) images of leaf mesophyll cells. Right hand panels: Green wavelength analysis (505-530nm) of the same sections to identify GFP fluorescence.

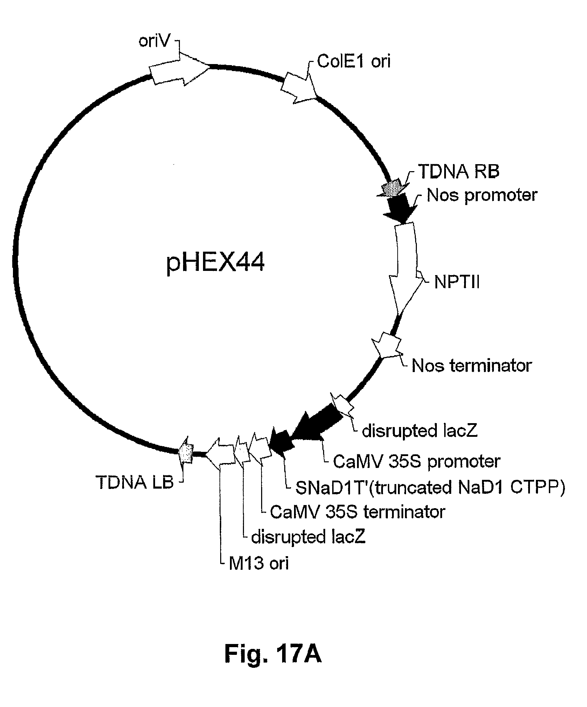

Figs. 17A-17E illustrate expression of chimeric defensin composed of the NaD1 mature domain (M) and a truncated NaD1 CTPP (T') (Example 6). A: Is a diagram of the pHEX44 construct containing DNA encoding mature NaD1 (M) with the NaD1 ER signal sequence (S) and the first four amino acids from the NaD1 CTPP in a binary vector; other abbreviations as given in Fig. 5. B: Diagram of the pHEX44 protein product. C: Relative levels of NaD1 produced during transient expression of pHEX44 (SMT') and pHEX43 (SMT) in cotton cotyledons assayed by ELISA. Extracts (1:100) from three seedlings were used in the ELISA. D: Protein immunoblot showing NaD1 (M) and NaD1 (MT) produced from pHEX43 and 44 during transient expression in *N. benthamiana* leaves. See Table 10 for pHEX construct list. E: Relative levels of NaD1 produced in cotton stably transformed with pHEX44 assayed by ELISA. Leaf extracts (1:500) from 5 plants in tissue culture were used in the ELISA with the NaD1 antibody. NaD1 purified from flowers and a leaf extract from the pHEX3 homozgote 35.125.1 were used as positive controls.

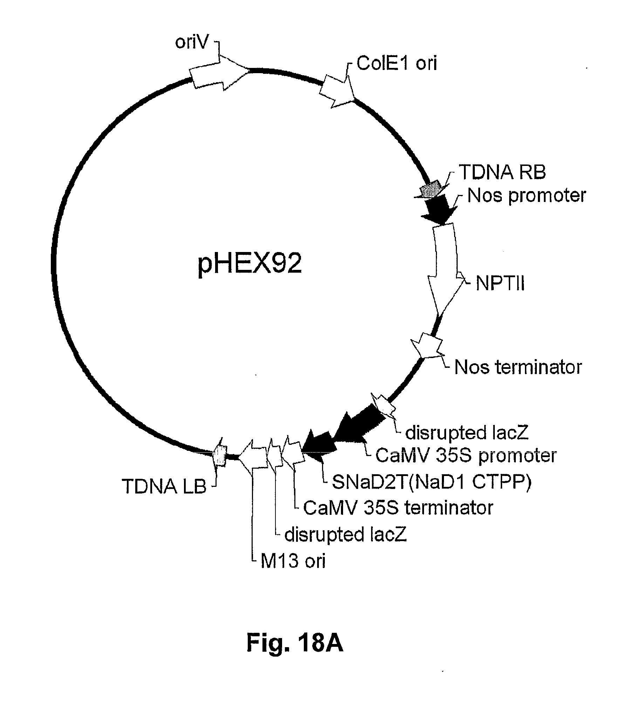

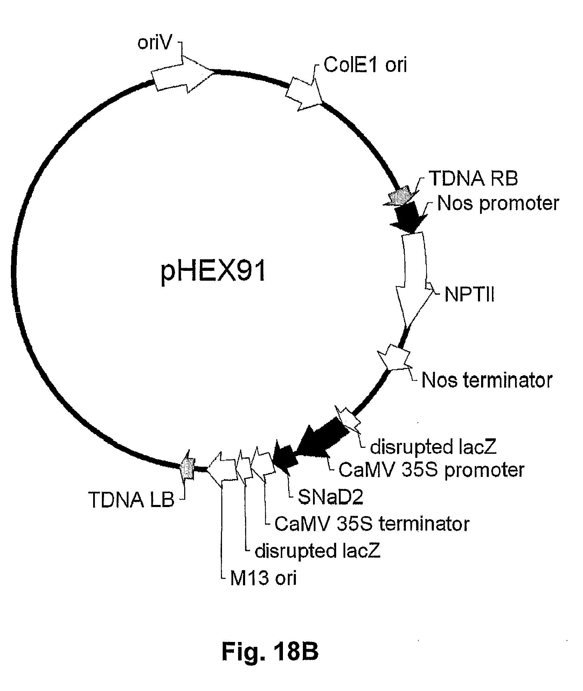

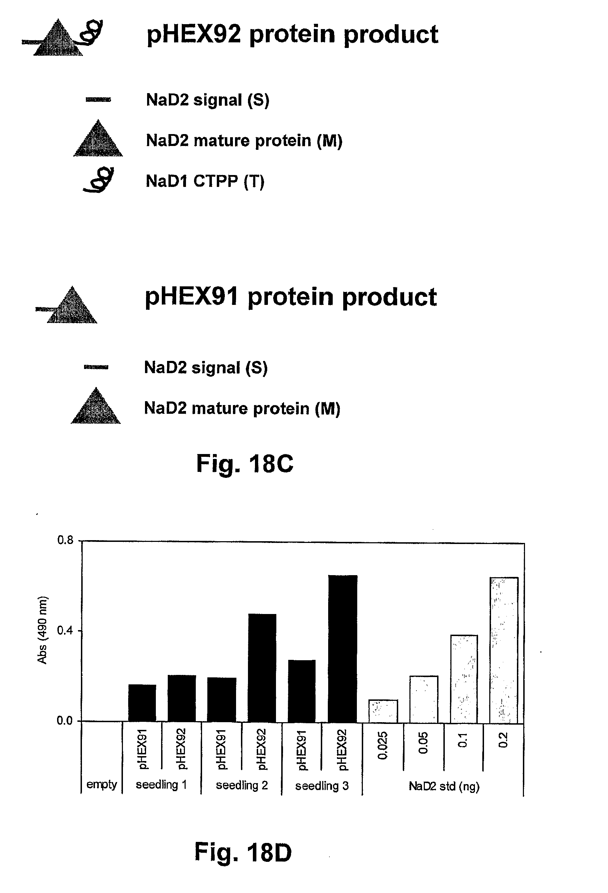

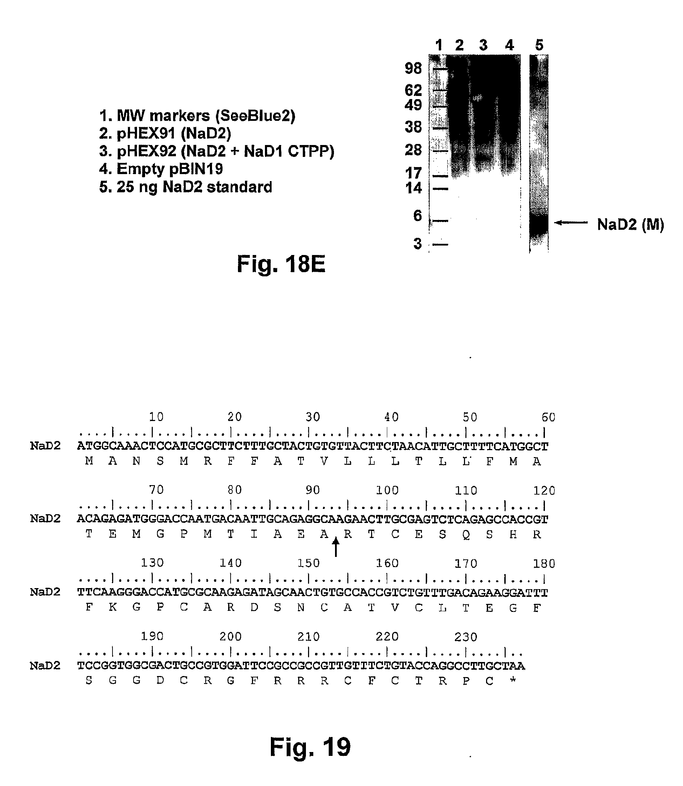

Fig. 18 illustrates the effect of adding the NaD1 CTPP to NaD2, a defensin without an endogenous CTPP (Example 7). A: is a diagram of the pHEX92 construct containing DNA encoding mature NaD2 (M) with the NaD2 ER signal sequence (S) and the CTPP from NaD1 (T) in a binary vector; other abbreviations as given for Fig. 5. B: The pHEX91 construct which is identical to pHEX92 except DNA encoding the CTPP from NaD1 (T) is not present. C: Diagram of the proteins encoded by pHEX91 and 91. D: Relative levels of NaD2 produced during transient expression of pHEX92 and 91 in cotton cotyledons assayed by ELISA. Extracts (1:100) from three seedlings were used in the ELISA with the NaD2 antibody and NaD2 purified from *Nicotiana alata* flowers as a positive control. E: Protein immunoblot with the NaD2 antibody showing relative levels of NaD2 (arrowed) produced during transient expression from the pHEX92 and 91 constructs.
Fig. 19 provides the nucleic acid (coding) and amino acid sequence of NaD2 (SEQ ID NO:32 and SEQ ID NO:33, respectively). cDNA encoding NaD2 was produced from mRNA isolated from the flowers of the ornamental tobacco, *Nicotiana alata.* NaD2 is produced as a proprotein with an endoplasmic reticulum signal sequence and a mature defensin domain. It does not have a natural C-terminal propeptide. The junction between the ER signal and the defensin domain is indicated by an arrow.

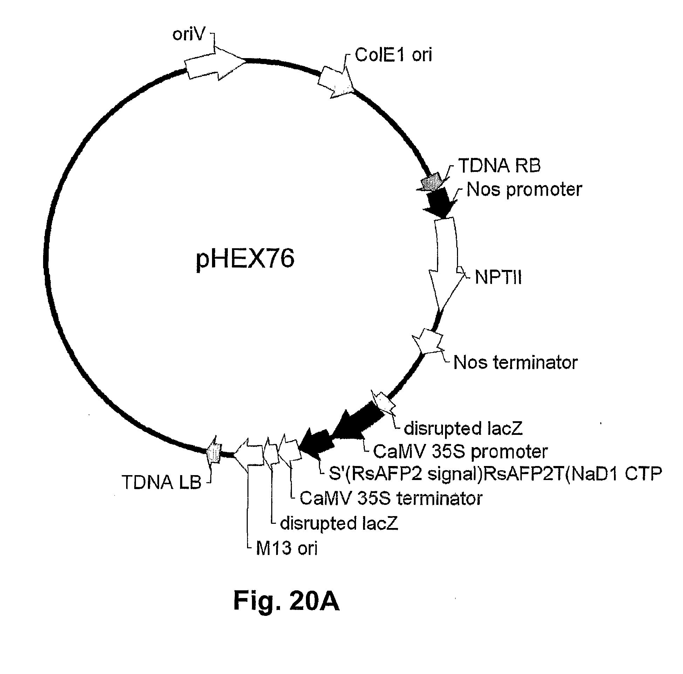

Figs. 20A-20C illustrate expression of a chimeric defensin composed of the radish defensin RsAFP2 (M) and the CTPP from NaD1 (Example 8). A: Is a diagram of the pHEX76 construct containing DNA encoding RsAFP2 (M) with the RsAFP2 ER signal sequence (S') and the CTPP (T) from NaD1 in a binary vector; other abbreviations as set forth for Fig. 5. B: The pHEX76 protein product. C: Protein immunoblot with the antibody directed to the CTPP from NaD1 assayed by ELISA. Extracts prepared from cotton cotyledons transiently expressing pHEX76 and pHEX43 and from the leaves of cotton stably transformed with pHEX3. See Table 10 for pHEX construct list.

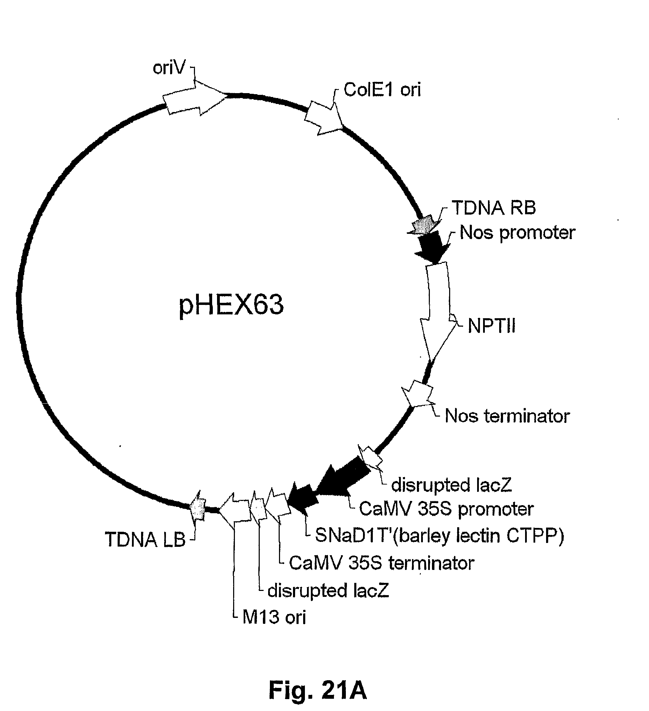

Figs. 21A-21E illustrate expression of a chimeric defensin composed of the NaD1 mature domain (M) and the CTPP from barley lectin (Example 9). A: is a diagram of the pHEX63 construct containing DNA encoding mature NaD1 (M) with the NaD1 ER signal sequence (S) and the CTPP (T') from the barley lectin precursor in a binary vector; other abbreviations as set forth for Fig. 5. B: Diagram of the pHEX63 protein product. C: Relative levels of NaD1 produced during transient expression of pHEX63, pHEX62 (encodes NaD1 [M] plus Zm ESR-6 CTPP [T"]) and pHEX43 in cotton cotyledons assayed by ELISA. Extracts (1:100) from two seedlings were used in the ELISA with the NaD1 antibody. D: Transient expression of NaD1 from the same constructs in the leaves of *N*. *benthamiana.* Extracts (1:500) from two leaves were used in the ELISA. E: Protein immunoblot showing NaD1 (M) and NaD1 (MT) produced from pHEX43, 62 and 63 during transient expression in *N. benthamiana.* See Table 10 for pHEX construct list.

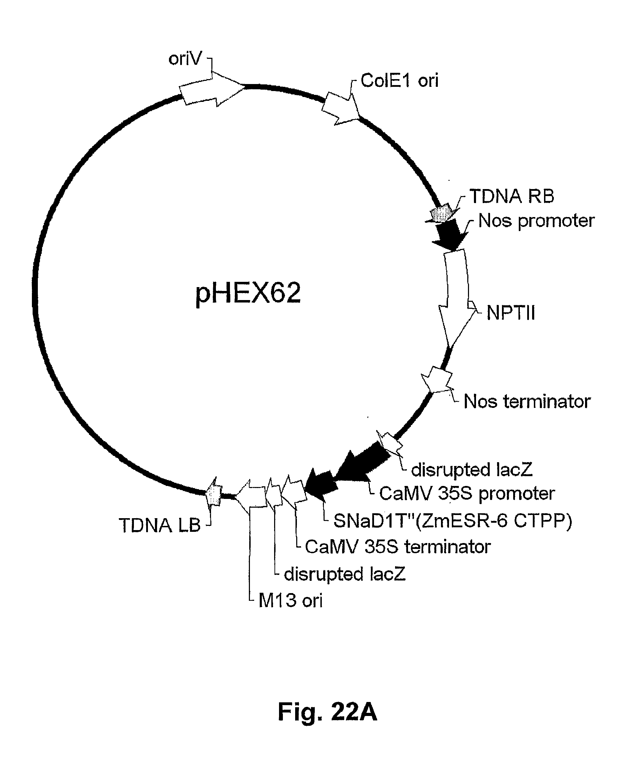

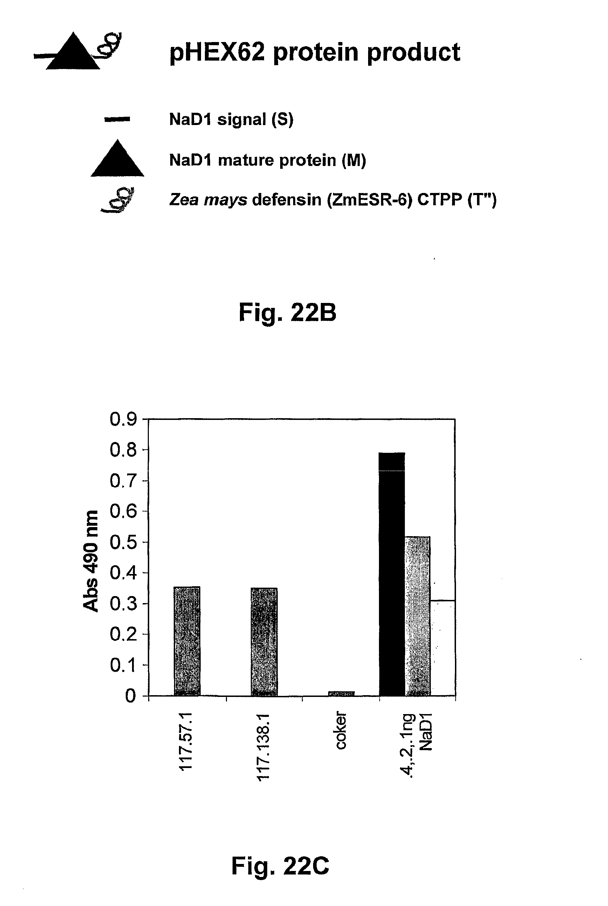

Figs. 22A-C illustrate the chimeric defensin composed of the NaD1 defensin (M) and the CTPP from the maize defensin Zm ESR-6 (T"). A: Is a diagram of the pHEX62 construct containing DNA encoding NaD1 (M) with the NaD1 ER signal sequence (S) and the CTPP (T") from the Zm ESR-6 defensin in a binary vector; other abbreviations as set forth for Fig. 5. B: The pHEX62 protein product. C: Relative levels of NaD1 produced in cotton stably transformed with pHEX62 determined by ELISA with the NaD1 antibody. Leaf extracts were diluted 1:500.

### SUMMARY OF THE INVENTION

Despite published reports of successful expression of certain functional plant defensins in certain transgenic plants, it has now been discovered by the present inventors that some defensins have toxic effects when expressed transgenically. Furthermore, the inventors herein have learned that the toxic effects are related to the level of defensin expression. The invention herein includes a general method for reducing or eliminating a toxic effect of transgenic defensin expression in a host plant. The invention also includes a method of modifying a nucleic acid encoding a defensin, a nucleic acid modified thereby and a modified defensin encoded by the modified nucleic acid sequence. The invention also includes a transgenic plant containing and expressing the modified defensin-coding nucleic acid sequence, the plant exhibiting reduced or eliminated toxic effects of defensin, compared with otherwise comparable transgenic plants expressing an unmodified defensin. The modified defensin is termed a chimeric defensin having a mature defensin domain of a first plant defensin combined with a C-terminal propeptide domain of a second plant defensin or a non-defensin plant vacuolar translocation peptide (VTP). A complete listing of SEQ ID NOS is set forth in Table 11.

### DETAILED DESCRIPTION OF THE INVENTION

The term "domain" is used herein and in the art to indicate a part of a peptide that has a distinct and recognizable function and is often separale from other parts of the peptide by post-translational-processing. In order to avoid ambiguity, certain terms are employed herein. As noted *supra,* the majority of known plant defensins are encoded by DNA coding for an endoplasmic reticulum signal sequence (hereinafter abbreviated "S") and a mature defensin domain (hereinafter "M"). Such defensins are also termed "seed defensins" in the literature, but they can be obtained from plant sources other than seeds. These are herein designated as SM type defensins. A minority of known defensins are encoded by DNA segments that code for an additional C-terminal prodomain/propeptide (CTPP), sometimes also termed an "acidic tail" (hereinafter "T"). Such defensins, sometimes called "floral defensins", are designated SMT type defensins. The designations "SM" and "SMT" are used throughout to refer to naturally occurring defensins. The term "chimeric defensin" is used herein to indicate a defensin of the present invention. Chimeric defensins can be of five general classes: (1) SM-type defensins combined with a T of an exogenous source (SMT'); (2) SMT-type defensins having an exogenous T of a different SMT-type defensin or other non-defensin plant VTP substituted for the naturally-occurring T (SMT"); (3) SM or SMT-type defensins having an exogenous substituted S segment (S'MT). Similarly, (4) S'MT' and (5) S'MT" chimeric defensins can be constructed, as will be understood in the art. Also described herein are instances where an SMT defensin having a T deletion is expressed. These are designated by the defensin name followed by a delta (Δ) symbol. Example, NaD1, an SMT defensin, is designated NaD1ΔT when expressed in tail-less or T-deleted form.

According to the invention, a defensin coding sequence to be expressed in a transgenic plant is modified by addition of a C-terminal prodomain (CTPP) as a C-terminal extension of the natural coding sequence, (SMT' or SMT"). CTPP (T' or T') enables the chimeric defensin to be translocated within the transgenic cell to a vacuole. Expression as a chimeric defensin protects the cell from any toxic effects the SM or ΔT defensin may exert in the host cell. The method can be carried out using any of a variety of known CTPP encoding nucleic acid segments such as, but not limited to, a coding segment for an acidic domain found in some SMT defensins (Fig. 4).

Vacuolar translocation peptides (VTPs) which occur as N-terminal peptide segments are also known. The abbreviation used herein for an N-terminal VTP is "X." A chimeric defensin with a N-terminal VTP has a general structure SXM, where S and M have their previously defined meaning. Examples of N-terminal VTPs useful herein are shown in Table 3.

**TABLE 3**

| **Examples of N-terminal propeptides (VTPs) of vacuolar plant proteins.** | | |
|---|---|---|
| **Protein** | **N-terminal sequence** | **Reference** |
| Sweet potato sporamin | HSRFNPIRLPTTHEPA (SEQ ID NO:36) | Matsuoka, K. and Nakamura, K. (1991) PNAS 88:834-838 |
| Potato 22 kDa protein | FTSENPIVLPTTCHDDN (SEQ ID NO:37) | Chrispeels, M. J., and Raikhel, N. V. (1992) Cell 68:613-616 |
| Barley aleurain | SSSSFADSNPIR (SEQ ID NO:38) | Holwerda et al., (1992) Plant Cell 4:307-318. |
| Potato cathepsin D inhibitor | FTSQNLIDLPS (SEQ ID NO:39) | Chrispeels and Raikhel (1992) |

According to one aspect of the invention, novel chimeric defensins are created of the type designated SMT', SMT" or SXM herein. The presence of an N-terminal signal peptide (S) causes the protein to enter a secretory pathway, where the signal peptide is removed. Removal of the signal sequence S exposes X at the N-terminus and permits binding of XM to a receptor in the Golgi, resulting ultimately in transport to the vacuole. The novel chimeric defensins of the invention are advantageous for expression in transgenic plants, where they allow higher levels of defensin expression with reduced toxic effects on the host plant, compared to transgenic plants expressing unmodified defensins, or tail-deleted defensins.

The invention in its broadest aspect includes the targeted use of S', T' and X domains to optimize the efficacy of mature defensins in transgenic plants. Internal cellular transport and export can be optimized in cells of a transgenic host species by use of a chimeric defensin, S'M, where the M domain, chosen for its activity, is combined with a signal peptide, S, that is compatible with the host cell species. Where a chosen SM-type defensin is poorly expressed or toxic in a transgenic host, a chimeric SMT' or SXM defensin can be provided for optimum defensin efficacy in the transgenic host. Other chimeric combinations including S'MT', S'MT", S"MT, S'XM and the like, will be recognized as useful in certain circumstances, as will be understood by those skilled in the art.

More than 60 plant defensins have been identified and characterized by amino acid sequence (Lay and Anderson 2005 Current Protein and Peptide Science 6:85-101 incorporated herein by reference to the extent not inconsistent herewith). All are expressed as a pre-defensin having an N-terminal signal peptide. The great majority of known plant defensins are expressed without a CTPP (SM type). Rarely, primarily in floral tissues of solanaceous species, certain defensins are expressed as pre-prodefensins having a CTPP in addition to a signal peptide (SMT type).

After post-translational processing, the mature defensins (M) range from 45 to 54 amino acids in length. All possess at least eight cysteine residues which form a characteristic disulfide bond pattern. If the eight C residues are numbered in sequence from N-terminus to C-terminus, the disulfide linkage pattern of plant defensins can be characterized as 1-8, 2-5, 3-6 and 4-7 (see Table 1 *supra*). A fifth disulfide has been identified in at least two plant defensins (Lay and Anderson 2005). Within the foregoing structural framework, very few amino acids are conserved other than the C residues, notably G34, an aromatic amino acid at position 11 followed by G13, S8, and E29, (see generic sequence in Fig. 1). All M-domain peptides having the placement and cross-linking pattern of C-residues characteristic of plant defensins as described are defined as plant defensins herein, whether isolated from nature, or derived by synthetic or combinatorial methods.

The CTPP is represented in some of the examples described herein by a C-terminal extension of 33 amino acids (SEQ ID NO:1, residues 73-105) of the floral defensin, NaD1, of *Nicotiana alata.* Other examples herein illustrate the breach of the invention by demonstrating VTP function for CTTP's from a tomato (*Solanum lycopersicon*) defensin, TPP3, a defensin of corn (*Zea mays*), ZmESR-6, a barley lectin, and a proteinase inhibitor isolated from *Nicotiana alata,* NaPI. Other such C-terminal domains are known including two from *Petunia hybrida,* PhD1 (SEQ ID NO:2, residues 73-103) and PhD2 (SEQ ID NO:3, residues 75-101). The inventors have now demonstrated that these extensions, also termed acidic domains or tails, function as a prodomain that targets transport to a storage vacuole within the cell. Many other vacuole-translocation peptides (VTP's) have been identified as C- or N-terminal prodomains (CTPP and NTPPs) that function in vacuolar translocation of other proteins besides defensins (Fig. 4; SEQ ID NOs:21-31, 35 and 40-45).

In general, the known C-terminal propeptides (CTPPs) tend to have a high proportion of acidic amino acids and hydrophobic amino acids. Any of the known CTPPs or part thereof can be employed in the present invention. Selection of a suitable CTPP will depend on suitability for use within the intended host cell.

Combining the coding sequence of a SM or SMΔT defensin with a sequence encoding a CTPP or a C or N-terminal VTP results in a chimeric pro-defensin. It is understood that defensins expressed in a transgenic host exert varying levels of toxicity on the host, from none to very toxic, depending on the functional activity of the defensin and the nature of the host. Toxic effects can be manifested in many ways that those skilled in the art recognize. The effects can include, for example, reduced cell growth, reduced efficiency of regeneration, reduced fertility of regenerated transgenic plants and abnormal morphology of regenerated plants. The degree of toxicity can vary as well, in response to the level of defensin expression, tissue specificity of expression, developmental stage of the host plant when expression occurs, and the like. In any instance where a toxic effect of expressing a defensin is observed, the toxic effect can be reduced or eliminated by modifying the defensin by addition of a CTPP. The modified defensin is herein termed a chimeric defensin. A chimeric defensin, (SMT', SMT", or SXM) as the term is used herein, is distinguished from defensins that exist in nature with a CTPP (SMT type). Any defensin which is provided with a CTPP to which it is not connected in nature, either SMT', SMT", or SXM, is considered a chimeric defensin and part of the present invention.

Applicants make no representation as to the mode of operation by which expression of a chimeric defensin reduces or eliminates a toxic effect of unmodified defensin expression. While applicants have observed a correlation between expression as a pro-defensin, reduced or eliminated toxicity and translocation of expressed pro-defensin to a storage vacuole and a reverse correlation between lack of CTPP, increased toxicity and lack of vacuole storage, it will be appreciated by those skilled in the art that other activities conferred by the presence of a CTPP can also reduce toxicity.

Transport of a chimeric defensin into a storage vacuole can confer other advantages. Once it is transported into the vacuole, the expressed chimeric defensin is likely to be prevented from exerting toxic effects within the cell. The host cell can tolerate higher levels of chimeric defensin expression than of unmodified defensin. Nevertheless, expression of a chimeric defensin provides a protective effect for the host plant against the action of a pathogenic agent, when an insect or invading fungus causes destruction of the cell, releases the defensin and exposes the pathogen to the defensin. Alternatively, a chimeric defensin can be harvested from host plant cells, to be used *ex-planta,* for example as an antifungal agent, or other purpose that utilizes the biological activity of the defensin.

.The choice of defensin to be expressed depends on the biological activity that is desired and the known properties of the defensin to be expressed. Defensin activity can be optimized by combinatorial methods for introducing single or multiple amino acid replacements at any amino acid position that is not essential for defensin structure and function, as long as a quantitative assay for defensin activity is available.

Selection of a CTPP to be combined with the defensin of choice is influenced by the host plant species. A CTPP derived from a first plant species can function comparably in a second plant species. For example, a CTPP from a *Nicotiana* species has been shown to provide effective intracellular transport and reduced toxicity in transgenic *Gossypium.* Efficacy can be optimized by using a CTPP of the host species where expression of the recombinant pro-defensin takes place, or by using a CTPP of a species related to the intended host. For example, the ZmESR-6 cDNA encoding CTPP predicted from a defensin from *Zea mays* (Fig. 3B and 3D; SEQ ID NO:18, residues 80-107) can provide optimal protection in transgenic corn expressing NaD1 to increase fungus resistance in that crop.

Both dicotyledonous and monocotyledonous transgenic plants can be routinely generated by methods known in the art. A chimeric defensin can be expressed in plants or plant cells after being incorporated into a plant transformation vector. Many plant transformation vectors are well known and available to those skilled in the art, e.g., BIN19 (Bevan, (1984) Nucl. Acid Res. 12:8711-8721), pBI 121 (Chen, P-Y, et al., (2003) Molecular Breeding 11:287-293), pHEX 22 (U.S. Patent 7,041,877), and vectors exemplified herein. Such vectors are well-known in the art, often termed "binary" vectors from their ability to replicate in a bacteria such as *Agrobacterim tumefaciens* and in a plant cell. A typical plant transformation vector, such as exemplified herein, includes genetic elements for expressing a selectable marker such as NPTII under control of a suitable promoter and terminator sequences, active in the plant cells to be transformed (hereinafter "plant-active" promoter or terminator) a site for inserting a gene of interest, including a chimeric defensin gene under expression control of suitable plant-active promoter and plant-active terminator sequences and T-DNA borders flanking the defensin and selectable marker to provide integration of the genes into the plant genome.

Plants are transformed using a strain of *A. tumefaciens,* typically strain LBA4404 which is widely available. After constructing a plant transformation vector that carries a DNA segment encoding the desired proteins, the vector is used to transform an *A. tumefaciens* strain such as LBA4404. The transformed LBA4404 is then used to transform the desired plant cells using an art-known protocol appropriate for the plant species to be transformed. Standard and art-recognized protocols for selecting transformed plant cells, multiplication and regeneration of selected cells are employed to obtain transgenic plants. The examples herein further disclose methods and materials used for transformation and regeneration of cotton plants, as well as transgenic cotton plants transformed by and expressing a variety of natural and chimeric defensins. A desired DNA segment can be transferred into plant cells by any of several known methods besides those exemplified herein. Examples of well-known methods include microprojectile bombardment, electroporation, and other biological vectors including other bacteria or viruses.

A chimeric defensin can be expressed in any monocotylodenous or dicotyledonous plant. Particularly, useful plants are food crops such as corn (maize) wheat, rice, barley, soybean, tomato, potato and sugarcane and oilseed crops such as sunflower and rape. Particularly useful non-food common crops include cotton, flax and other fiber crops. Flower and ornamental crops include rose, carnation, petunia, lisianthus, lily, iris, tulip, freesia, delphinium, limonium and pelargonium.

Techniques for introducing vectors, chimeric genetic constructs and the like into cells include, but are not limited to, transformation using CaCl₂ and variations thereof, direct DNA uptake into protoplasts, PEG-mediated uptake to protoplasts, microparticle bombardment, electroporation, microinjection of DNA, microparticle bombardment of tissue explants or cells, vacuum-infiltration of tissue with nucleic acid, and T-DNA-mediated transfer from *Agrobacterium* to the plant tissue.

For microparticle bombardment of cells, a microparticle is propelled into a cell to produce a transformed cell. Any suitable ballistic cell transformation methodology and apparatus can be used in performing the present invention. Exemplary procedures are disclosed in Sanford and Wolf (U.S. Patent Nos. 4,945,050, 5,036,006, 5,100,792, 5,371,015). When using ballistic transformation procedures, the genetic construct can incorporate a plasmid capable of replicating in the cell to be transformed.

Examples of microparticles suitable for use in such systems include 0.1 to 10 µm and more particularly 10.5 to 5 µm tungsten or gold spheres. The DNA construct can be deposited on the microparticle by any suitable technique, such as by precipitation.

Plant tissue capable of subsequent clonal propagation, whether by organogenesis or embryogenesis, can be transformed with a natural or chimeric defensin gene of the present invention and a whole plant generated therefrom, as exemplified herein. The particular tissue chosen will vary depending on the clonal propagation systems available for, and best suited to, the particular species being transformed. Examples of tissue targets include leaf disks, pollen, embryos, cotyledons, hypocotyls, megagametophytes, callus tissue, existing meristematic tissue (e.g. apical meristem, axillary buds, and root meristems), and induced meristem tissue (e.g. cotyledon meristem and hypocotyl meristem).

The regenerated transformed plants can be propagated by a variety of means, such as by clonal propagation or classical breeding techniques. For example, a first generation (or T1) transformed plant may be selfed to give a homozygous second generation (or T2) transformant and the T2 plants further propagated through classical breeding techniques.

Accordingly, this aspect of the present invention, insofar as it relates to plants, further extends to progeny of the plants engineered to express the nucleic acid of the chimeric defensins of the invention, as well as vegetative, propagative and reproductive parts of the plants, such as flowers (including cut or severed flowers), parts of plants, fibrous material from plants (for example, cotton) and reproductive portions including cuttings, pollen, seeds and callus.

Another aspect of the present invention provides a genetically modified plant cell or multicellular plant or progeny thereof or parts of a genetically modified plant capable of producing a protein or peptide encoded by the chimeric defensin gene as herein described wherein said transgenic plant has acquired a new phenotypic trait associated with expression of the protein or peptide.

### EXAMPLES

### EXAMPLE 1:

### Production and characterisation of transgenic cotton plants expressing mature NaD1 (minus tail) (SEQ ID NO:31, residues 26-72)

### 1. Production of transgenic plants

Transgenic cotton plants were generated from transformation experiments using the gene construct pHEX22 (Fig. 5). pHEX22 contains the antifungal *NaD1* gene lacking the sequence encoding the C-terminal prodomain (SMΔT).

Two cotton transformation experiments (CT 78 and CT 83, Table 4) were conducted. The transgenic cotton lines were produced by *Agrobacterium-*mediated transformation using standard protocols (Umbeck P. (1991) Genetic engineering of cotton plants and lines. US Patent 5,004,863). The binary vector pHEX22 was transferred into *Agrobacterium tumefaciens* strain LBA4404 by electroporation and the presence of the plasmid confirmed by gel electrophoresis. Cultures of *Agrobacterium* were used to infect hypocotyl sections of cotton cv Coker 315. Embryogenic callus was selected on the antibiotic kanamycin at 35 mg/L, and embryos were germinated using standard protocols for cotton. Plantlets were transferred to soil, and the expression of NaD1 (SEQ ID NO:1 residues 26-72) from DNA encoding SMΔT was determined by immunoblot analysis and ELISA using specific antisera.

**TABLE 4**

| **Summary of two cotton transformation experiments using the pHEX22 construct** | | |
|---|---|---|
| | **CT 78** | **CT 83** |
| **No. hypocotyl explants** | **558** | **550** |
| **No. plantlets** | **17** | **43** |
| **No. plants expressing NaD1** | **1** | **9** |

### 2. Detection of NaD1 in transgenic leaf tissue and plant phenotype

### Method used for ELISA assays of NaD1 M domain:

ELISA plates (Nunc Maxisorp™ (In Vitro, Noble Park VIC 3174) #442404) were coated with 100 µL/well of primary antibody in PBS: 50ng/well NaD1 antibody (protein A purified polyclonal rabbit antibody raised in response to the mature NaD1 domain (M, SEQ ID NO: 1, residues 26-72) by a standard method) and incubated overnight at 4°C in a humid box.

The next day, the plates were washed with PBS/0.05% (v/v) Tween 20, 2 min x 4. Plates were then blocked with 200 µL/well 3% (w/v) BSA (Sigma A (Castle Hill, NSW Australia 1765)-7030: 98% ELISA grade) in PBS and incubated for 2 h at 25°C and then washed with PBS/0.05% (v/v) Tween 20, 2 min x 4.

For preparation of leaf samples, 100 mg of frozen cotton leaf tissue was ground in liquid nitrogen using a mixer mill for 2 x 10 sec at frequency 30. One mL of 2% (w/v) insoluble PVP (Polyclar)/PBS/0.05% (v/v) Tween 20 was added to each sample and the mixture vortexed, centrifuged for 10 min and the supernatant collected. Dilutions of the cotton protein extracts were prepared in PBS/0.05% (v/v) Tween 20, applied to each well (100 µL/well) and incubated for 2h at 25°C.

Plates were washed with PBS/0.05% (v/v) Tween 20, 2 min x 4. Secondary antibody in PBS (50 ng/well biotin-labelled anti-NaD1, raised to mature defensin domain) was applied to each well at 100 µL/well and incubated for 1 h at 25°C.

Plates were washed with PBS/0.05% (v/v) Tween 20, 2 min x 4. NeutriAvidin HRP-conjugate (Pierce, Rockford, II 61105) #31001; 1:1000 dilution; 0.1µL/well) in PBS was applied to each well at 100 µL/well and incubated for 1 h at 25°C.

Plates were washed with PBS/0.05% Tween 20, 2 min x 4 then with H₂O, 2 min x 2. Fresh substrate was prepared by dissolving one ImmunoPure OPD (peroxidase substrate) tablet (Pierce, Rockford, II 61105 #34006) in 9 mL water, then adding 1 mL stable peroxide buffer (10x, Pierce, Rockford, II 61105 #34062). Substrate (100 µL/well) was added to each well and incubated at 25°C. The reaction was stopped with 50 µL of 2.5 M sulfuric acid and the absorbance measured at 490 nm in a plate reader.

### Method for Southern blot analysis

Cotton genomic DNA was extracted using the Qiagen DNeasy™ plant mini kit (cat#69104), following manufacturer's instructions, Genomic DNA (10µg) was digested with 30 units of the restriction enzyme B*cl* 1 (Promega, cat# R6651) at 37°C overnight. Digested genomic DNA was electrophoresed for 16 hours at 35V on a 0.8% agarose gel in 1X TBE. The gel was treated for 10 minutes in 0.2 M HCl, 30 minutes in 1.5 M NaCl, 0.5 M NaOH and 30 minutes 1 M Tris-HCL pH 7.5, 1.5 M NaCl prior to transfer to membrane. DNA was transferred to a nylon membrane (Hybond N+, Amersham Biosciences, cat# RPN203B) by capillary transfer in 10 X SSC overnight at room temperature. DNA was crosslinked to the memrane using 1200 µJ of UV light (Hoefer UV crosslinker). The membrane was pre-hyridized at 42°C in a solution of 50% v/v formamide, 5 X SSPE, 5 X Denhardt's solution, 0.5% SDS w/v and 0.1 mg/mL of acid degraded herring sperm DNA for 6 hours. The radioactive probe was prepared using the Prim-a-gene laeling system with P³² labeled dCTP (Promegia, cat# U1100). The memrane was incubated with probe overnight at 42°C. The membrane was washed twice at 42°C for 30 minutes in a solution of 2 X SSC and 0.1 % SDS to remove unbound probe. The blot was exposed to X-RAY film (Fuji, cat#10335) for 5 days at -80°C before development.

### Results

Screening of the primary transgenic plants by ELISA resulted in the identification of 10 plants expressing detectable levels of mature NaD1 (one plant from CT 78 and 9 plants from CT 83). The levels of mature NaD1 (SEQ ID NO:1, residues 26-72) varied among plants (Table 4). All the plants expressing mature NaD1, except for line 83.68.2, had distorted or small leaves often with short internodes (Fig. 6). Seven of the 10 primary transformants were infertile, while the other 3 lines produced bolls with reduced numbers of seeds (78.131.1, 83.102, 83.68.2). These results suggest that the presence of mature NaD1 (M domain) (SEQ ID NO:1, residues 26-72) directly affected the growth and development of the transgenic plants.

Eleven plants from the transformation experiments with pHEX22 that were ELISA negative (i.e no NaD1 expression) were allowed to continue growing in the glasshouse. Nine of these plants (82%) had a normal phenotype, and the other two had slightly distorted leaves (18%). This result is similar to other transformation experiments with cotton. For example, there is always a low number of plants (less than 20%) that have an unusual phenotype, apparently resulting from the cotton embryogenesis regeneration method used. The altered phenotype is attributed to the passage of cells through tissue culture.

Note that we have previously produced transgenic cotton expressing DNA encoding NaD1 with the tail (SEQ ID NO:1, residues 1-105) (experiment CT 35, U.S. Patent 7,041,877; see also Example 3). Of the 11 plantlets produced in this experiment, two plants (18%) had an unusual phenotype. Neither of these plants was found to express NaD1. Importantly, the three highest expressing NaD1 (plus CTPP) (SEQ ID NO:1, residues 1-105) plants from experiment CT 35 (35.9.1, 35.105.1 and 35.125.1) (see Example 3) had a normal phenotype and were fertile.

Estimates based on separate ELISAs suggest that the level of NaD1 M domain in plants with defensin plus tail (SMT) is significantly higher than in plants expressing NaD1 minus tail (SMΔT). The one exception may be line 78.131.1 which expressed very high levels of mature NaD1 (SMΔT). This plant had distorted leaves and small bolls. Figure 6 shows two primary transgenic plants: 82.23.2(A) and 83.96.2(B). Plant 83.96.2 has a higher level of NaD1 expression (see Table 5) and displayed a more severe phenotype variation. Both of these plants were infertile.

**TABLE 5**

| Characterization of primary transgenic plants expressing SMΔT produced in experiments CT 78 and CT 83. The ELISA results were rated from + (low expression) to ++++ (highest expression). | | |
|---|---|---|
| **Transgenic plant** | **ELISA score** | **Phenotype** |
| 78.131.1 | ++++ | Distorted leaves, normal internodes. Small bolls with reduced seeds. |
| 83.23.2 | ++ | Leaves slightly distorted, normal internodes, infertile |
| 83.54.1 | +++ | Distorted leaves, short internodes, infertile |
| 83.67.1 | + | Distorted leaves, short internodes, infertile |
| 83.68.2 | ++ | Normal leaves and internodes, fertile. Bolls with reduced number of seeds. |
| 83.96.2 | +++ | Distorted leaves, bushy, short internodes, infertile |
| 83.102.1 | +++ | Small leaves, normal internodes, fertile. Small bolls with reduced number of seeds. |
| 83.111.3 | ++ | Normal leaves, short internodes, infertile. |
| 83.166.1 | + | Distorted leaves, short internodes, infertile |
| 83.182.1 | +++ | Distorted leaves, spindly plant. Infertile. |

### Assessment of segregating T2 plants

Primary transgenic lin 78.131.1 was self-polinated and progeny plants assessed to determine whether the abnormal phenotype segregated with the defensin gene. The expression of NaD1 (M domain) was determined by ELISA (Table 6). A representative ELISA is shown in Figure 7.

**TABLE 6**

| Expression of NaD1 in progeny plants from the primary transgenic line 78.131.1 | |
|---|---|
| Transgenic cotton line | 78.131.1 |
| No. of seedlings (progeny of primary transgenic | 75 |
| No. of plants expressing NaD1 by ELISA | 58 (77%) |
| No. of plants NOT expressing NaD1 by ELISA | 17 (23%) |

All progeny plants expressing NaPI (M domain) displayed the same abnormal phenotype (i.e. severely distorted leaves) as the parent transgenic plant while all the plants that did not express NaD1 had a normal phenotype. Figure 8A shows a photograph of seen of the 78.131.1 progeny plants.

The segregation of NaD1 expression in the progeny of 78.131.1 is consistent with the primary transgenic plant having one copy of the NaD1 gene (Table 6). This was confirmed by Southern blot analysis (Figure 8B).

These results confirm that the presence of mature NaD1 lacking a CTPP causes an unusual abnormal phenotype. It can be concluded that mature NaD1 is toxic to the plant. Plants that had been transformed with the NaD1 gene containing the C-terminal tail (SMT) do not exhibit the abnormal phenotype, suggesting the CTPP (SEQ ID NO:1, residues 73-105) either protects the plant from a toxic part of the molecule or it targets the protein to the vacuole where it is sequestered.

### 3. Immunoblot analysis

Plants that were positive for NaD1 by ELISA were assessed by immunoblot analysis. Total protein from 100 mg leaf tissue (first fully expanded leaf) was extracted in acetone and precipitated proteins resuspended in PBS-T with 3% PVPP. After centrifugation, the supernatant was adjusted to 1 X LDS sample buffer (NuPAGE™ (Invitrogen, Carlsbad, CA 92008)) and 5% (v/v) β-mercaptoethanol. The NaD1 antibody (made against NaD1 with tail) was used at 1/1000 dilution of a 1 mg/ml stock. Controls were: 150 or 50 ng purified NaD1, 35.125.1 plant (NaD1 with tail, homozygous), untransformed Coker.

Mature NaD1 (sequence ID NO:1, residues 26-72) was detected in lines 35.125.1 (SMT construct), 78.131.1(SMΔT construct) and 83.68.2(SMΔT construct) (Fig. 9). The mature NaD1 plus CTPP was detected in line 35.125.1 (Fig. 9).

### 4. Subcellular location of NaD1

The subcellular location of NaD1 in the transgenic plants was determined using immuno-fluorescence with the anti NaD1-antibody.

At the time of fixation, samples were taken from the same leaves and NaD1 levels were determined by ELISA assays as outlined above. NaD1 levels in lines 35.125.1 and 78.131.1 were 0.01% and 0.02% total soluble protein respectively.

Leaf segments from non-transgenic Coker, line 35.125.1 (transformed with pHEX3 the NaD1, SMT construct) and line 78.131.1 (transformed with pHEX22 NaD1, SMΔT construct) were fixed in 4% paraformaldehyde and embedded in paraffin and sectioned by Austin Health. Sections were incubated with the anti-NaD1 antibody (50µG/mL in blocking solution) [0.2% Triton X 100, 1 mg/mL BSA in PBS] for 60 min, and were washed with 1 X PBS before application of the second antibody [Alex Fluor® Molecular Probes, diluted 1:200 in blocking solution. Sections were visualized on an Olympus BX50 microscope and images were captured using a monochrome spot camera with spot RT software.

The defensin, NaD1 was present in the vacuoles of leaf cells from line 35.125.1 which expressed NaD1 with the CTPP tail (SMT construct) Fig. 10A. In the control incubated with pro-immune serum, no immunofluorescence was observed (Fig. 10B). In contrast NaD1 was clearly absent from the vacuoles in line 78.131.1 expressing NaD1 without the CTPP tail (SMΔT construct) (Fig. 10C). The NaD1 in these cells formed punctate deposits in the cytoplasm and extracellular space. No fluorescence was detected in the vacuoles in the control incubated with pro-immune serum, no immunofluorescence was observed (Fig. 10D). The leaf cells in the 78.131.1 transformant appeared smaller and had a more irregular shape than the leaf cells in the 35.125.1 transformant (NaD1 with the CTPP tail). This observation may explain the altered leaf morphology presented in Figure 8.

### EXAMPLE 2:

### Identification of defensin precursors with the C-terminal propeptide in transgenic plants

Protein samples from immature buds of *N*. *alata* and from the leaves of transgenic cotton line 35.125.1 (NaD1 with tail, homozygous) were tested by immunoblot analysis as described in Example 1, using a CTPP specific antibody (Figure 11). Transgenic cotton lin 35.125.1 was previously described in U.S. Patent 7,041,877, incorporated herein by reference. Example 11 thereof disclosed line 35.125.1 was transformed with full length (SMT) nucleic acid encoding NaD1 (termed NaPdf1 therein).

Total soluble protein from immature *N*. *alata* buds was extracted in extraction buffer (100 mM Tris HCl, 10 mM EDTA, 2 mM CaCl₂ and 15 mM beta-mercaptoethanol 1:4). Protein from leaf tissue of 35.125.1 was extracted in acetone and the pellet resuspended in extraction buffer. After centrifugation, the supernatant was adjusted to 1 X LDS sample buffer (NuPAGE™ (Invitrogen, Carlsbad, CA 92008)) and 5% (v/v) beta-mercaptoethanol. Protein A purified CTPP antibody was used at 1/500 dilution of a 1mg/ml stock. Control was 1 ug of purified NaD1.

For the production of the CTPP antibody, the 33 amino acid CTPP of NaD1 (SEQ ID NO:1, residues 73-105) was chemically synthesised with an additional cysteine residue at the C-terminus (VFDEKMTKTGAEILAEEAKTLAAALLEEEIMDNC) (SEQ ID NO:42) to facilitate conjugation of the peptide to a carrier protein. The CTPP peptide was chemically cross-linked to maleimide-activated *Megathura crenulata* keyhole limpet hemocyanin (KLH) (Imject^{®} fromPierce, Rockford, II 61105) according to the manufacturer's instructions. The conjugated peptide was desalted on a PD-10 column (Amersham Pharmacia Biotech, Piscataway, NJ) before injection into a rabbit for polyclonal antibody production as previously described in Lay et al 2003 *supra.*

The mature NaD1 plus CTPP (SEQ ID NO:1, residues 26-105) was detected in *N. alata* immature buds and in line 35.125.1 leaves (Fig. 11A).

Directly following probing with the CTPP antibody, the blot was reprobed overnight with NaD1 antibody (1/1000 dilution of a 1mg/ml stock). Mature NaD1 (SEQ ID NO:1, residues 26-72) was detected in immature buds (Fig. 11B).

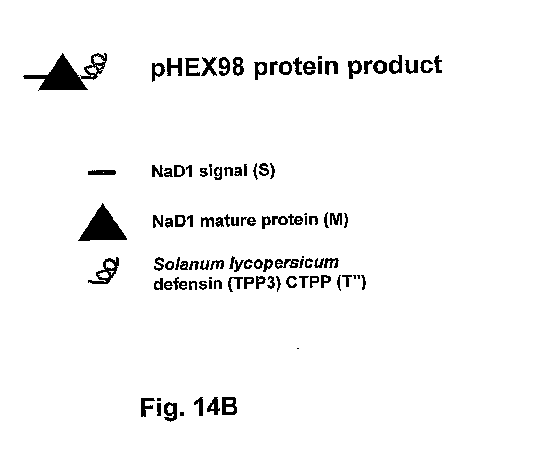

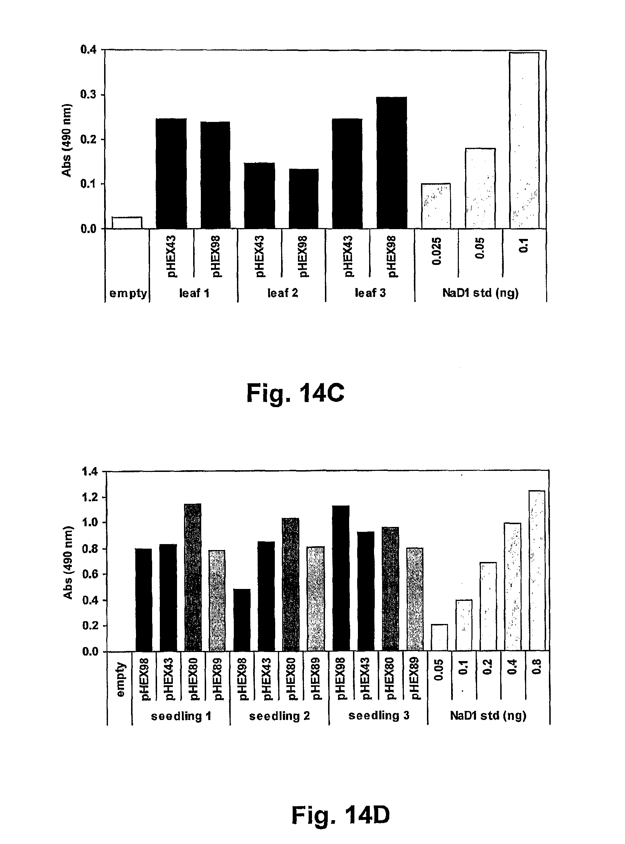

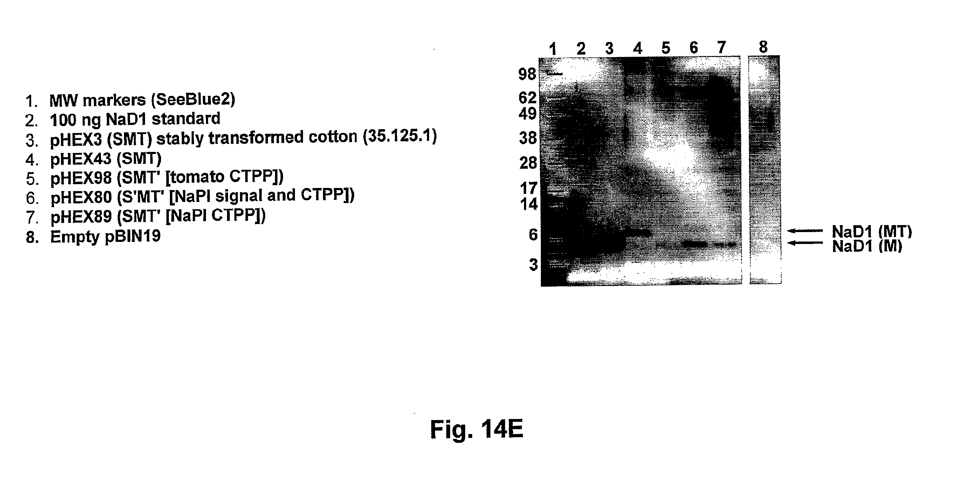

The results demonstrate that transgenic cotton plants transformed by DNA encoding full-length NaD1 (SMT) express both mature domain (M) and mature domain plus tail (MT), as described in Example 1, Fig. 9, lane 4, and confirmed using a polypeptide-specific antibody to the NaD1 CTPP as described above. The data herein differ from data reported in U.S. Patent 7,041,877 (see, e.g., Fig. 14 therein), which showed a single band of NaD1 reactive to antibody extracted from transgenic tobacco. Resolution of the apparent discrepancy is considered to be due to improved extraction, especially in the use of acetone precipitation. It is now evident that a proportion of the NaD1 CTPP is retained after post-translational processing of full-length (SMT) NaD1.

### EXAMPLE 3:

### Inhibition of Fusarium oxysporum f. sp. vasinfectum (Fov) infection in transgenic cotton expressing NaD1

Transgenic cotton line 35.125.1 was previously described in U.S. Patent 7,041,877, incorporated herein by reference. Example 11 thereof disclosed line 35.125.1 was transformed with full length (SMT) nucleic acid encoding NaD1 (termed NaPdf1 therein). Example 8 thereof disclosed that purified NaD1 M domain at 20 µg/mL inhibited *in vitro* growth of *Botrytis cinerea* and *Fusarium oxysporum f. sp. dianthi.*

### Glasshouse bioassay using infected soil

A glasshouse infected soil bioassay was used to assess the level of resistance to Fov in line 35.125.1. Cultures of Fov (Australian isolate VCG 01111 #24500 isolated from cotton. Gift from Wayne O'Neill, Farming Systems Institute, DPI, Queensland, Australia) were prepared in millet and incorporated into a soil mix. Cultures of Fov were prepared in 1/4 strength potato dextrose broth (6 g/L potato dextrose) and grown for approximately one week at 26°C. The culture (5 to 10 mL) was used to infect autoclaved hulled millet which was then grown for 2 to 3 weeks at room temperature. The infected millet was incorporated into a pasteurised peat based soil mix at 1% (v/v), by vigorous mixing in a 200 L compost tumbler. The infected soil was transferred to plastic containers (10 L of mix per 13.5 L container). Control soil contained uninfected millet. The infected soil was used to grow line 35.125.1, Siokra 1-4 (Fov susceptible), Sicot 189 (less susceptible; industry standard) and untransformed Coker. Eighty-seven (87) seeds of each variety/line were planted. Seed was sown directly into the containers, 12 seed per box in a 3 X 4 array. Two to three seed of each line was sown randomly in each box, and the boxes were rotated and moved weekly to reduce variation that may occur due to position in the glasshouse. Plants were grown for 8 weeks. Height and symptom development were measured throughout the trial and the disease score was determined by destructive sampling at the end of the trial.

### Results

The disease incidence was high in this bioassay and the progress of the disease was followed for 8 weeks. The susceptible variety Siokra 1-4 and the untransformed Coker were first to show wilting in the leaves while the less susceptible variety Sicot 189 (Australian cotton industry standard) and the transgenic line 35.125.1 (D1) started to show wilt symptoms several days later. By day 33, approximately 83% of Siokra 1-4 and 76% of untransformed Coker plants were showing symptoms, while for Sicot 189 and transgenic line 35.125.1 (D1) only 26% and 36%, respectively showed symptoms.

A similar trend was seen with plant survival (Figure 12). Plants of the susceptible variety Siokra 1-4 were first to die, and by the end of the bioassay only 5% of Siokra 1-4 plants had survived. Survival of the untransformed Coker plants was intermediate (final survival was 38%), while plants of Sicot 189 and line 35.125.1 had significantly lower mortality (final survival was 61% and 57% respectively, Table 7).

At the end of the bioassay, plants were assessed for disease by scoring the amount of vascular browning in a lateral section of each plant (Table 7). The susceptible variety Siokra 1-4 had the highest disease score (4.9) and the less susceptible variety Sicot 189 and the transgenic line 35.125.1 had the lowest disease scores (3.3 and 3.6). The untransformed Coker control had a disease score of 4.0 which was intermediate between the less susceptible (Sicot 189) and susceptible (Siokra 1-4) varieties.

The disease score was analysed using ordinal logistic regression and mortality was analysed using logistic regression. Table 8 represents the pair-wise comparison of the disease scores of the four lines tested. All lines (untransformed Coker, Sicot 189 and Line 35.125.1) are significantly better than Siokra 1-4 at a p value of <0.001. There was a significant difference between the disease score of transgenic line 35.125.1 and the parent Coker (P = 0.04). Similar statistical differences were also seen for plant mortality (Table 9).

**TABLE 7**

| Results of the *Fusarium* infected soil bioassay in the glasshouse. | | | | |
|---|---|---|---|---|
| | **Siokra 1-4** | **Coker 315** | **Sicot 189** | **Line 35.125.1** |
| No. of plants that germinated | 87 | 84 | 80 | 80 |
| No. dead plants | 83 | 52 | 31 | 34 |
| No. living plants | 4 | 32 | 49 | 46 |
| % living plants | 5% | 38% | 61% | 57% |
| Disease score* | 4.9 | 4.0 | 3.3 | 3.6 |

| | | | | |
|---|---|---|---|---|
| *Average of all seeds that germinated. 0 = no symptoms, 1 = vascular browning to base of stem, 2 = vascular browning to cotyledons, 3 = vascular browning past cotyledons, 4 = vascular browning to true leaves, 5 = dead. | | | | |

**TABLE 8**

| Analysis of disease score data. | | | |
|---|---|---|---|
| | **Siokra 1-4** | **Untransformed Coker 315** | **Sicot 189** |
| Siokra 1-4 | - | - | - |
| Coker 315 | p<0.001 | - | - |
| Sicot 189 | p<0.001 | P=0.005 | - |
| Line 35.125.1 | P<0.001 | P=0.04 | P=0.5 |

**TABLE 9**

| Analysis of plant mortality data. | | | |
|---|---|---|---|
| | **Siokra 1-4** | **Untransformed Coker 315** | **Sicot 189** |
| Siokra 1-4 | - | - | - |
| Coker 315 | p<0.001 | - | - |
| Sicot 189 | p<0.001 | P=0.003 | - |
| Line 35.125.1 | p<0.001 | P=0.01 | P=0.6 |

### Field trial

The transgenic line 35.125.1 (Line D1), untransformed Coker 315 and the less susceptible variety Sicot 189 (Australian Industry standard) were assessed in a field trial in the 2006-2007 cotton season. Plants were grown at a farm in the Darling Downs region of Queensland, Australia. Seed was hand planted into soil known to be infected with Fov. A total of 800 seed per variety were planted in four replicate plots, each containing 200 seed per variety. Emergence and plant survival was recorded. At the end of the trial the plants were assessed for disease by measuring the vascular discoloration visible in a cross section of the main stem cut as close as practicable to ground level (csd.net.au/ on the worldwide web, 2007 Variety Guide).

### Results

The seed was planted early in the season and favorable weather conditions (early rain and several days of low temperatures) resulted in a very high disease incidence. The results for plant survival at the end of the trial are presented in Figure 13*.* Highest mortality was seen with the untransformed Coker, where only 7.5% of plants had survived. The transgenic Line D1 had 22% plant survival and Sicot 189 36% plant survival (Fig. 13). Furthermore transgenic line D1 had a higher disease score (66), that is, higher resistance to *Fusarium oxysporum* than the non-transgenic Coker control (disease score 24). Overall transgenic line D1 had a 10-12% higher yield (boll weight) than the surviving non-transgenic Coker control plants. The average yield per plant was 52g for line D1 and 45g for the Coker control. The results are similar to the glasshouse bioassay data and show that the transgenic line has improved resistance to Fov compared to the untransformed Coker 315 parent.

### EXAMPLE 4.

A chimeric defensin, SMT"-type, was transiently expressed in tomato leaves and cotton cotyledons using construct pHEX98 (Fig. 14A). The S and M domains were sequences of NaD1 (SEQ ID NO:1, residues 1-72); T" was obtained from TPP3 (SEQ ID NO:8), a defensin of tomato *(Solanum lycopersicum).* The amino acid sequence of the C-terminal TPP3 acidic domain (T") (SEQ ID NO:8, residues 74-105 and Fig. 3A) is given in Lay et al 2003 *supra* and Genbank accession No. U20591. The exemplified chimeric defensin is diagrammed in Fig. 14B.

pHEX98 (Fig. 14A) was introduced into *A*. *tumefaciens* and the expression of NaD1 was determined by transient assay using cotton cotyledons or tomato leaves. Bacterial "lawns" of the *Agrobacterium* were spread on selective plates and grown in the dark at 30 °C for 3 days. Bacteria were then resuspended to an OD600ₙₘ of 1.0 in infiltration buffer (10 mM magnesium chloride and 10 µm acetosyringone (0.1 M stock in DMSO)) and incubated at room temperature for 2-4 h. Cotton plants were grown for 8 days in a controlled temperature growth cabinet (25 °C, 16 h/8 h iight/dark cycle) before infiltration. The underside of the cotyledons was infiltrated by gently pressing a 1 mL syringe against the cotyledon and filling the cotyledon cavity with the *Agrobacterium* suspension. The area of infiltration (indicated by darkening) was noted on the topside of the cotyledon. A maximum of 4 infiltrations were performed per cotyledon. Plants were grown for a further 4 days. The infiltrated areas were then cut out, weighed and frozen in liquid nitrogen. The same procedure was used with leaves from 3 week old tomato seedlings. Protein expression was determined by ELISA and immunoblots as described in Examples 1 and 3.

The ELISA assay for NaD1 expression in tomato leaves (Fig. 14C) and cotton cotyledons (Fig. 14D) illustrated that NaD1 was expressed from the pHEX98 construct and that replacement of the NaD1 tail (T) with the TPP3 tail (T") had no significant effect on NaD1 accumulation. That is, the TPP3 tail was as effective as the NaD1 tail. Protein blot analysis of the expressed proteins (Fig. 14E) showed that the defensin produced from the pHEX98 construct was predominantly mature NaD1. That is, the TPP3 tail had been efficiently processed from the mature NaD1 domain (M).

The chimeric defensin described *supra* is stably expressed in transgenic tomato using the pHEX98 construct. The chimeric defensin provides enhanced fungal resistance due to NaD1 expression in the transgenic tomato. Use of the TPP3 tail provides a transport function for moving the NaD1 to a storage vaculole and for ameliorating toxic effects of NaD1 M domain in transgenic tomato cells.

Transformation is carried out by known techniques for tomato transformation, using a binary vector (McCormick, S. et al. (1986) Plant Cell Rep. 5:81-84) having the SMT' chimeric sequence described above

Transformants are regenerated by a known method of tomato transformation. Seedlings of transgenic plants are assayed for NaD1 (M domain) expression using the ELISA tests as described in Examples 1-3. Plants having normal morphology and expressing detectable amounts of NaD1 are tested for fungal resistance and toxicity to insect pests essentially as described herein.

Further optimization of expression can be achieved by combining both the S (SEQ ID NO:8, residues 1-25) and T (SEQ ID NO:8, residues 74-105) domains of TPP3 with the mature (M) defensin domain of NaD1, to form a chimeric defensin of S'MT' designed for optimal expression of the NaD1 mature domain in transgenic tomato.

### EXAMPLE 5.

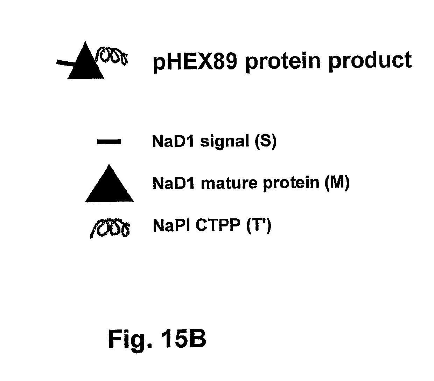

A chimeric defensin, SMT'-type, was transiently expressed in cotton cotyledons using construct pHEX89 (Fig. 15A). The S and M domains were sequences of NaD1 (SEQ ID NO:1, residues 1-72); T' was obtained from NAPI, a proteinase inhibitor from *Nicotiana alata* (SEQ ID NO:35; see also Fig. 4 (Fig. 15B)). The amino acid sequence of the C-terminal NaPI vacuolar targeting sequence is given in US patent 6,031,087, incorporated herein by reference to the extent not inconsistent herewith. The exemplified chimeric defensin is diagrammed in Fig 15B.

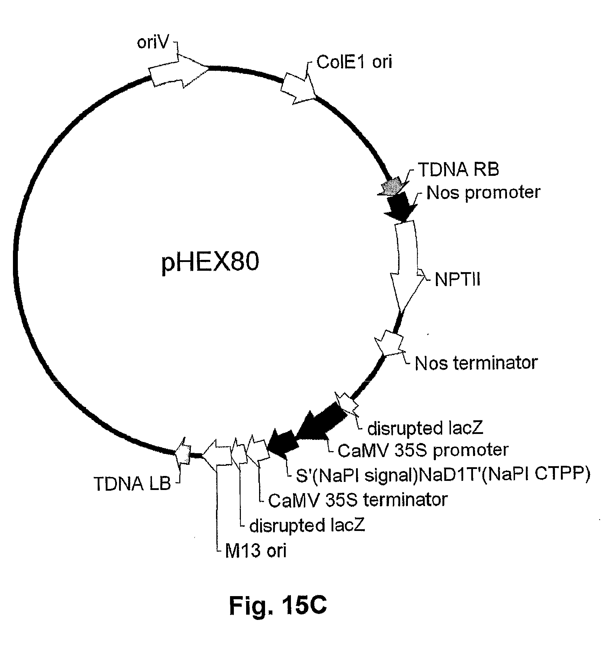

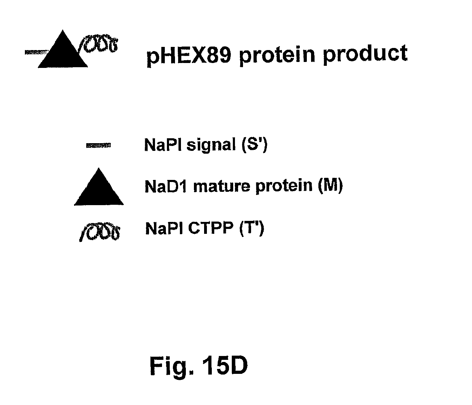

Further optimization of expression can be achieved by combining both the signal peptide (S')and the C-terminai vacuolar targeting sequence of NaPI (T') with the mature (M) defensin domain of NaD1, to form a chimeric defensin of S'MT' designed for optimal expression of the NaD1 mature domain in transgenic cotton. The construct pHEX80 used for expression of S'MT' is given in Fig. 15C and a diagram of the protein product is presented in Fig. 15D.

Transient expression of pHEX89 and pHEX80 in cotton cotyledons was conducted as described in Example 4. Quantification of NaD1 expression by ELISA is presented in Fig. 14D. NaD1 was expressed from both the pHEX89 (SMT') and pHEX80 (S'MT') constructs. Replacement of the NaD1 tail with the NaPI tail had no significant effect on NaD1 accumulation during transient expression in cotton. Replacement of the NaD1 signal sequence with the signal sequence of NaPI in the pHEX80 construct increased expression of NaD1 (Fig. 14D). Protein blot analysis of the expressed proteins (Fig. 14E) showed that mature defensin accumulated during expression from the pHEX89 and pHEX80 constructs. That is, the NaPI tail was removed efficiently from the mature NaD1 defensin domain(M).

To confirm that the NaPI tail was sufficient to target NaD1 to the vacuole, a DNA construct was prepared encoding the NaPI signal peptide in front of the coding sequence for the green fluorescent protein (GFP) followed by the NaPI CTPP (pHEX96). An identical construct without DNA encoding the NaPI CTPP (pHEX97) was also prepared (Fig. 16A). pHEX70 which encodes GFP with the NaPI signal peptide and the NaD1 CTPP was used to demonstrate that the NaD1 CTPP was also sufficient to direct a protein to the vacuole. These constructs were expressed transiently in the leaves of *Nicotiana benthamiana* as described in Example 4 for cotton and GFP fluorescence was examined using a Leica confocal microscope.

GFP produced from the pHEX96 and pHEX70 [GFP + NaPI CTPP] constructs accumulated in the vacuole while GFP from the pHEX97 construct (GFP no CTPP) was detected in the cytoplasm and extracellular space (Fig. 16B).

The SMT'-type chimeric defensin is stably expressed in transgenic cotton using the pHEX89 construct. The chimeric defensin provides enhanced fungal resistance due to NaD1 expression in the transgenic cotton compared to the untransformed parental line. Use of the NaPI tail provides a transport function for moving the NaD1 to a storage vacuole and for ameliorating toxic effects of NaD1 M domain in transgenic cotton cells.

Transformation is carried out by known techniques for cotton transformation, using a binary vector having the SMT' chimeric sequence described above.

Transformants are regenerated by a known method of cotton transformation. (Example 1). Seedlings of Transgenic plants are assayed for NaD1 expression using the ELISA tests as described in Examples 1-3. Plants having normal morphology and expressing detectable amounts of NaD1 are tested for fungal resistance essentially as described herein.

Further optimization of expression can be achieved by combining both the S and C-terminal vacuolar targeting sequences of NaPI with the mature (M) defensin domain of NaD1, to form a chimeric defensin of S'MT' designed for optimal expression of the NaD1 mature domain in transgenic cotton.

### EXAMPLE 6.

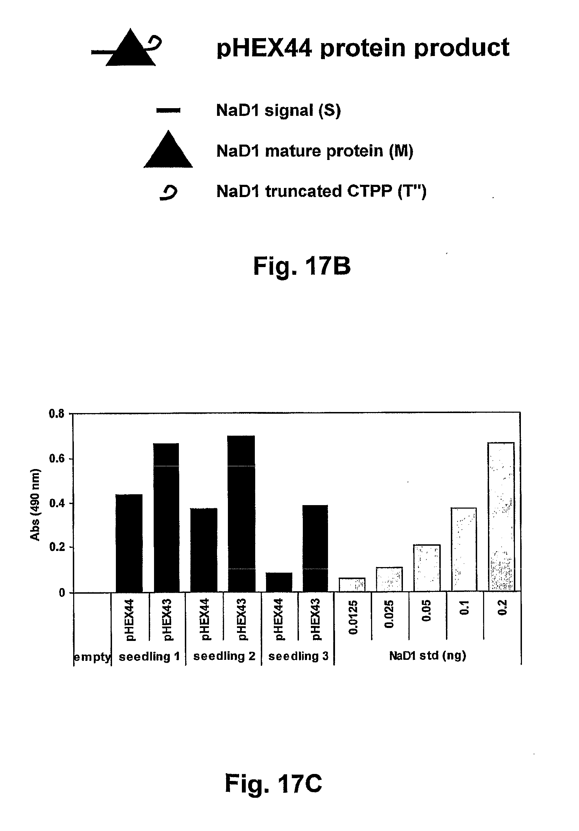

A chimeric defensin, SMT'-type, was transiently expressed in cotton and *Nicotiana benthamiana* using pHEX44 (Fig. 17A). N. benthamiana was a gift from Richard McKnight, University of Utago, New Zealand. The plant is also available from commercial sources. The S and M domains were sequences of NaD1 previously described; T' was a truncated version of the NaD1 CTPP (Fig. 17B). The CTPP consists of the first four amino acids (VDFE) of the NaD1 CTPP (Fig. 4; SEQ ID NO:1, residues 73-76).

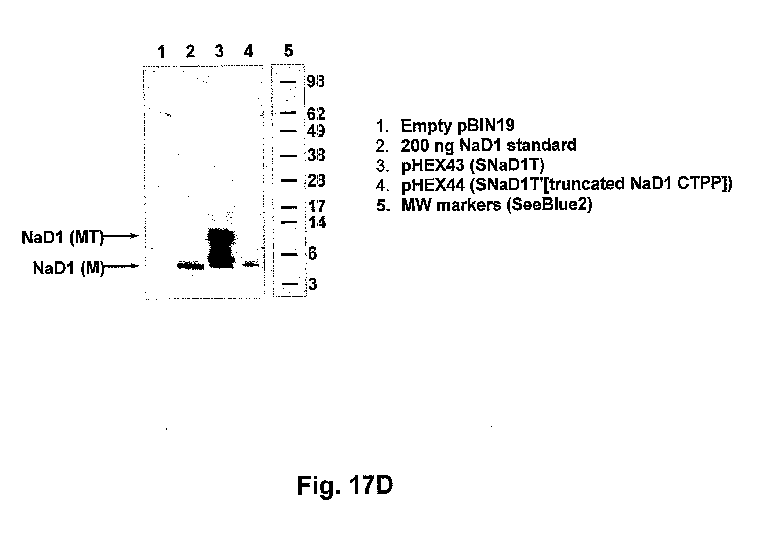

Transient expression of pHEX44 in cotton cotyledons was conducted as described in Example 4. Quantification of NaD1 expression by ELISA is presented in Fig. 17C. Truncation of the complete 33 amino acid NaD1 CTPP (T) to four amino acids (VFDE) did not abolish NaD1 expression but led to a substantial decrease in NaD1 accumulation. Decreased production of NaD1 from the construct with the truncated CTPP (pHEX44) was also evident when constructs were expressed transiently in *N. benthamiana* and NaD1 production was examined on protein blots (Fig. 17D).

A cotton transformation experiment was conducted using the pHEX44 construct. The transgenic cotton lines were produced by *Agrobacterium-*mediated transformation as described in Example 1. The expression of NaD1 (SEQ ID NO:1 residues 26-72) from DNA encoding SMT' was determined by ELISA using specific antisera as described in Example 1. Leaf samples were collected from plantlets in tissue culture.

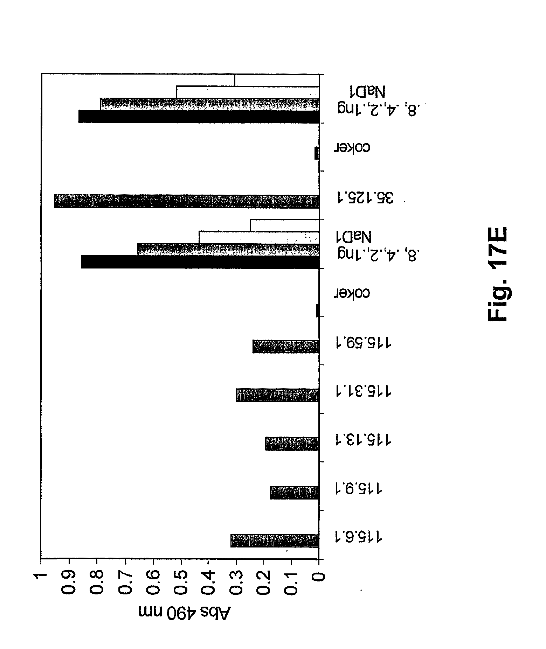

Five plants expressing detectable levels of mature NaD1 were identified (Fig. 17E). Expression levels were lower than those usually obtained with the homozygous line 35.125.1 (U.S. Patent 7,041,877) which had been transformed with the pHEX3 construct (SMT) (Fig 17E).

In summary, the four amino acid element (VFDE, Fig. 4) is not adequate for full expression of NaD1(M). Nevertheless, the exemplified chimeric defensin can provide enhanced fungal resistance provided by NaD1 expression in transgenic cotton and tobacco.

### EXAMPLE 7.

A chimeric defensin, SMT"-type, was expressed transiently (See Example 4) in cotton cotyledons using the DNA construct pHEX92 (Fig. 18A). The S and M domains were sequences of NaD2 (SEQ ID NO:33); T" was obtained from NaD1 (SEQ ID NO:1, residues 73-105) (Fig. 18B). For comparison, a second construct pHEX91 (Fig. 18B) encoding S and M domains of NaD2 without a C-terminal tail (T) (Fig. 18C) was also transiently expressed in cotton cotyledons. The nucleic acid and amino acid sequence of NaD2 (SM) is presented in Fig. 19.

Transient expression of pHEX92 and 91 in cotton cotyledons was conducted as described in Example 4. Quantification of NaD2 expression by ELISA is presented in Fig. 18D. Addition of the NaD1 tail to the NaD2 defensin(M) increased the level of NaD2 accumulated during transient expression compared to the level of NaD2 produced from the pHEX91 construct which encoded NaD2 without a C-terminal tail. Immunoblots with the NaD2 antibody confirmed that more NaD2 was produced from the pHEX92 construct (NaD2 defensin plus NaD1 CTPP) and that the NaD1 CTPP had been proteolytically removed (Fig. 18E).

The SMT"-type chimeric defensin is stably expressed in transgenic *Arabidopsis thaliana* and cotton, using the pHEX92 construct. The chimeric defensin provides enhanced fungal resistance due to NaD2 expression in the transgenic cotton compared to the untransformed parental line. Use of the NaD1 tail provides a transport function for moving the NaD2 to a storage vacuole and for ameliorating the toxic effects of NaD2 expressed without a tail in transgenic cotton cells.

Transformation is carried out by known techniques for cotton transformation, using a binary vector having the SMT' chimeric sequence described above.

Transformants are regenerated by a known method of cotton transformation. Seedlings of transgenic plants are assayed for NaD2 (M domain) expression using the ELISA test as described in any of Examples 1-3, except that the antibody has been prepared against NaD2 (SEQ ID NO:33, residues 32-78). Plants having normal morphology and expressing detectable amounts of NaD2 (SEQ ID NO:33) are tested for fungal resistance essentially as described herein.

### EXAMPLE 8.

A chimeric defensin, SMT"-type, was transiently expressed in cotton cotyledons using the DNA construct pHEX76 (Fig. 20A). The S and M domains were sequences of Rs-AFP2 (SEQ ID NO:16); T" was obtained from NaD1 (SEQ ID NO:1, residues 73-105). The amino acid sequence of Rs-AFP2 is presented in Fig. 3 and SEQ ID NO:17.

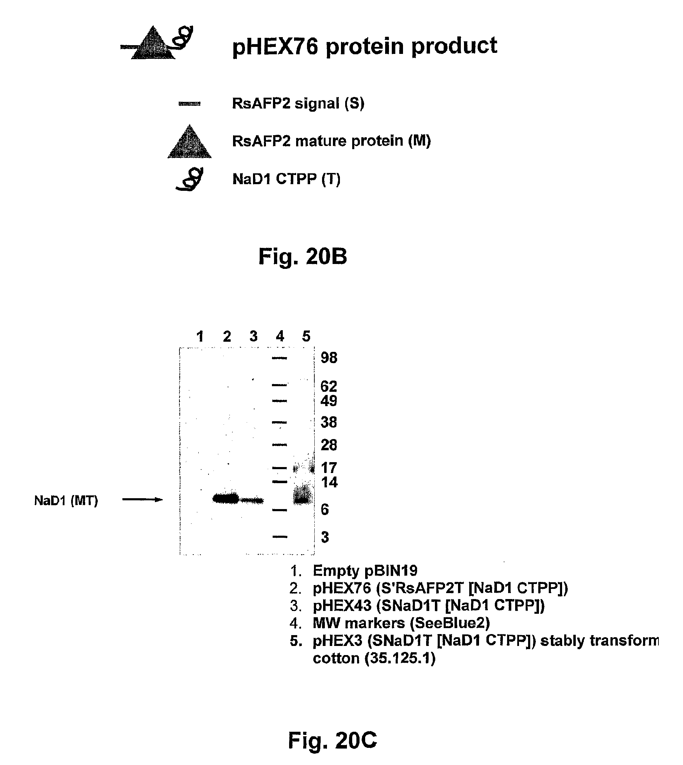

Transient expression of pHEX76 in cotton cotyledons was conducted as described in Example 4. Analysis of protein expression was achieved using protein blots with the antibody to the CTPP from NaD1 (see Example 2 for description of the antibody). Rs-AFP2 plus CTPP (MT") was produced from the pHEX76 construct (Fig. 20C). Interestingly, expression from pHEX76 appeared higher than expression from the pHEX43 and pHEX3 constructs which both encoded NaD1(M) + the NaD1 CTPP(T). This result may be a reflection of the level of defensin produced or the rate of removal of the NaD1 CTPP(T) from the mature defensin domains. An antibody to Rs-AFP(M) was not available for assessment of Rs-AFP(M) accumulation.

The SMT' chimeric defensin of Fig 20A and 20B is stably expressed in transgenic cotton plants. The exemplified chimeric defensin [Fig. 20B] provides enhanced fungal resistance provided by Rs-AFP2 (SEQ ID NO:17) expression in transgenic plants. Use of the NaD1 (SEQ ID NO:1, residues 73-105) tail provides a transport function for moving Rs-AFP2 (SEQ ID NO:17) to a storage vacuole and ameliorating toxic effects of Rs-AFP2 in transgenic dicotyledonous and monocotyledonous cells.

Transformation and regeneration of cotton is carried out as described herein, Example 1. Seedlings of transgenic plants are assayed for Rs-AFP2 (M domain) (SEQ ID NO:17, residues 30-80) expression using an ELISA test with an antibody raised against Rs-AFP2. Plants having normal orphology and expressing detectable amounts of Rs-AFP2 are tested for fungal resistance essentially as described herein.

### EXAMPLE 9.

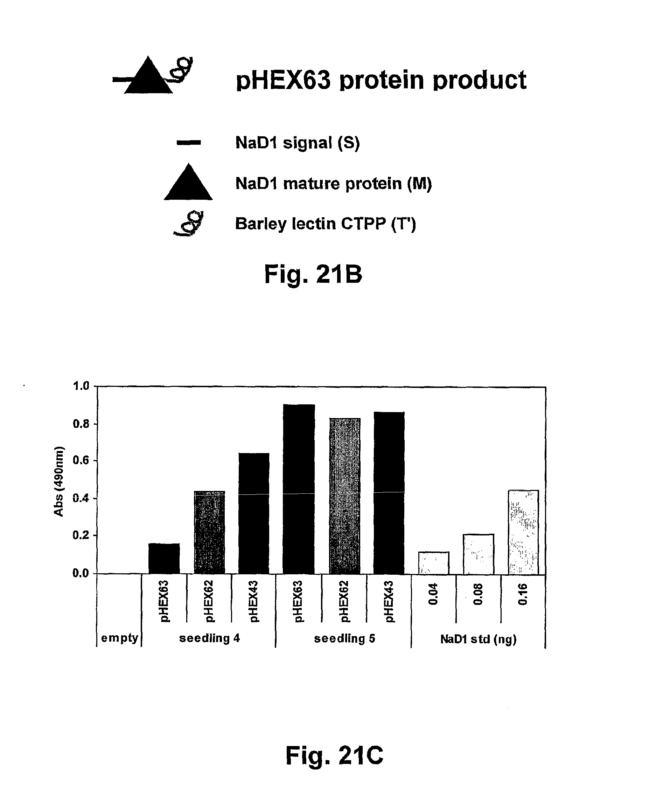

A chimeric defensin, SMT'-type, was transiently expressed in cotton cotyledons and *Nicotiana benthamiana* leaves using the DNA construct pHEX63 (Fig. 21A). The S and M domains were sequences of NaD1 (SEQ ID NO:1, residues 1-72); T' is obtained from BL, a lectin of barley *(Hordeum vulgare)* (SEQ ID NO:24) (Fig. 21B). The amino acid sequence of the C-terminal vacuolar targeting sequence from barley lectin (BL) is given in Fig. 4.

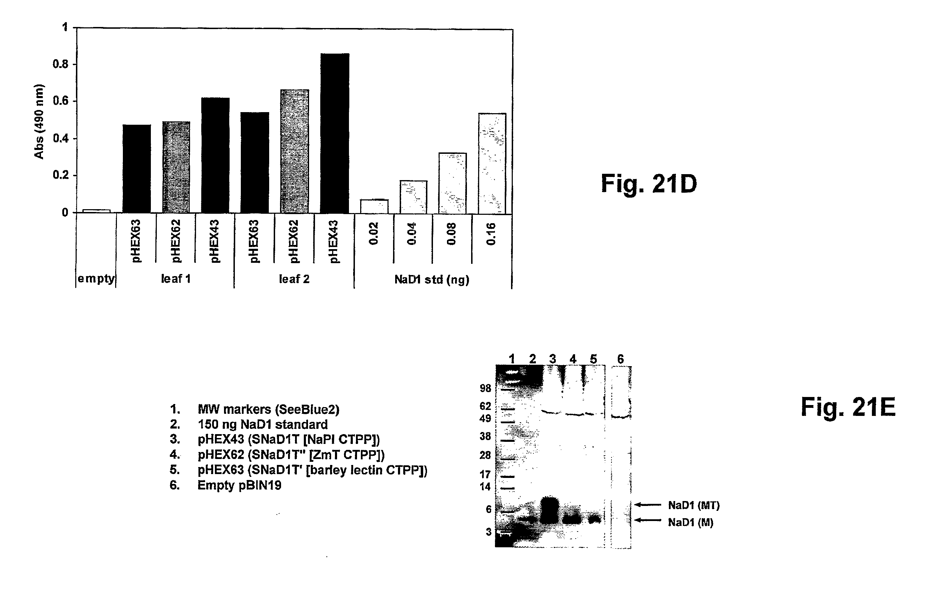

Transient expression of pHEX63 was conducted as described in Example 4. Analysis of protein expression in cotton cotyledons was performed by ELISA (Fig. 21C). Exchanging the NaD1 CTPP(T) for the CTPP sequence from barley lectin had no major effects on levels of expression when the variation between seedlings was taken into account. Similar results were obtained during transient expression of pHEX63 in *N. benthamiana* leaves (Fig. 21D). Interestingly, the protein blot analysis of the expressed proteins (Fig. 21E) indicated that the barley lectin CTPP domain was processed from the NaD1 mature defensin(M) more efficiently than the NaD1 CTPP (T).

The SMT'-type chimeric defensin of Fig. 21B is stably expressed in transformed cotton plants using the DNA construct pHEX63. Transformation is carried out as described in Example 1, using a binary vector having the SMT' chimeric sequence described above.

Transformants are regenerated as previously described. Seedlings of transgenic plants are assayed for NaD1 (M domain) expression using an ELISA test as described in any of Examples 1-3. Plants having normal morphology and expressing detectale amounts of NaD1 are tested for fungal resistance essentially as described herein.

The exemplified chimeric defensin provides enhanced fungal resistance provided by NaD1 expression in transgenic plants. Use of the BL tail provides a transport function for moving the NaD1 to a storage vacuole and ameliorating toxic effects of NaD1 in transgenic dicotyledonous and monocolyledonous cells.

### EXAMPLE 10.

A chimeric defensin, SMT"-type, was transiently expressed in cotton cotyledons using the DNA construct pHEX62 (Fig. 22A). The S and M domains were sequences of NaD1 (SEQ ID NO:1, residues 1-72); T" was obtained from ZmESR-6, a defensin of corn (*Zea mays)* (SEQ ID NO:18, residues 80-107) [Fig. 22B]. The amino acid sequence of the C-terminal targeting sequence from ZmESR-6 is given in Figs. 3B and 3D.

Transient expression of pHEX62 was conducted as described in Example 4. Analysis of protein expression in cotton cotyledons (Fig. 21C) and *N*. *benthamiana* leaves(Fig. 21D) was performed by ELISA. As observed for the barley lectin CTPP in Example 9 substitution of the NaD1 CTPP (T) with the CTPP(T") from the maize defensin (ZmESR-6) had no significant effect on the levels of NaD1 produced during transient expression (Figs. 21C and 21D). Furthermore, immunoblots demonstrated that the ZmESR-6 CTPP was also processed more efficiently than the NAD1-CTPP from the NaD1 mature domain (Fig. 21E). Thus, CTPP sequences from monocots function in dicotyledonous plants for efficient expression of defensins (M-domain).

A cotton transformation experiment was conducted using pHEX62 construct. The transgenic cotton line were produced by *Agrobacterium-*mediated transformation as described in Example 1. The expression of NaD1 (SEQ ID NO:1 residues 26-72) from DNA encoding SMT" was determined by ELISA using specific antisera as described in Example 1. Leaf samples were collected from plantlets in tissue culture.

Two plants expressing detectable levels of the mature NaD1 were identified (Fig. 21F).

Plants having normal morphology and expressing detectable amounts of NaD1 are tested for fungal resistance essentially as described herein. The exemplified chimeric defensin provides enhanced fungus resistance provided by NaD1 expression in transgenic plants. Use of the ZmESR-6 C-terminal sequence (or part thereof) provides a transport function for moving the NaD1 to a storage vacuole and ameliorating toxic effects of NaD1 in transgenic monocotyledonous and dicotyledonous plant cells.

As used herein, "comprising" is synonymous with "including," "containing," or "characterized by," and is inclusive or open-ended and does not exclude additional, unrecited elements or method steps. As used herein, "consisting of' excludes any element, step, or ingredient not specified in the claim element. As used herein, "consisting essentially of" does not exclude materials or steps that do not materially affect the basic and novel characteristics of the claim. Any recitation herein of the term "comprising", particularly in a description of components of a composition or in a description of elements of a device, is understood to encompass those compositions and methods consisting essentially of and consisting of the recited components or elements. The invention illustratively described herein suitably may be practiced in the absence of any element or elements, limitation or limitations which is not specifically disclosed herein.

The terms and expressions which have been employed are used as terms of description and not of limitation, and there is no intention in the use of such terms and expressions of excluding any equivalents of the features shown and described or portions thereof, but it is recognized that various modifications are possible within the scope of the invention claimed. Thus, it should be understood that although the present invention has been specifically disclosed by preferred embodiments and optional features, modification and variation of the concepts herein disclosed may be resorted to by those skilled in the art, and that such modifications and variations are considered to be within the scope of this invention as defined by the appended claims.

In general the terms and phrases used herein have their art-recognized meaning, which can be found by reference to standard texts, journal references and contexts known to those skilled in the art. The following definitions are provided to clarify their specific use in the context of the invention.

All patents and publications mentioned in the specification are incorporated by reference to the extent there is no inconsistency with the present disclosure, and those references reflect the level of skill of those skilled in the art to which the invention pertains.

One skilled in the art readily appreciates that the present invention is well adapted to carry out the objects and obtain the ends and advantages mentioned, as well as those inherent in the present invention. The methods, components, materials and dimensions described herein as currently representative of preferred embodiments are provided as examples and are not intended as limitations on the scope of the invention. Changes therein and other uses which are encompassed within the spirit of the invention will occur to those skilled in the art, are included within the scope of the claims.

Although the description herein contains certain specific information and examples, these should not be construed as limiting the scope of the invention, but as merely providing illustrations of some of the embodiments of the invention. Thus, additional embodiments are within the scope of the invention and within the following claims.

**TABLE 10.**

| List of constructs | | | |
|---|---|---|---|
| **Defensin constructs** | | | |
| **pHEX number** | **Signal peptide (S)** | **Mature defensin protein (M)** | **CTPP (T)** |
| 3 | NaD1 | NaD1 | NaD1 |
| 22 | NaD1 | NaD1 | - |
| 43 | NaD1 | NaD1 | NaD1 |
| 44 | NaD1 | NaD1 | truncated NaD1 |
| 62 | NaD1 | NaD1 | ZmESR-6 |
| 63 | NaD1 | NaD1 | barley lectin |
| 76 | RsAFP2 | RsAFP2 | NaD1 |
| 80 | NaPI | NaD1 | NaPI |
| 89 | NaD1 | NaD1 | NaPI |
| 91 | NaD2 | NaD2 | - |
| 92 | NaD2 | NaD2 | NaD1 |
| 98 | NaD1 | NaD1 | TPP3 |

| **GFP constructs** | | | |
|---|---|---|---|
| **pHEX number** | **Signal peptide (S)** | **Mature protein** | **CTPP (T)** |
| 70 | NaPI | GFP | NaD1 |
| 71 | NaPI | GFP | ZmESR-6 |
| 72 | NaPI | GFP | barley lectin |
| 95 | - | GFP | - |
| 96 | NaPI | GFP | NaPI |
| 97 | NaPI | GFP | - |

**TABLE 11**

| SEQ ID NOS - Figure 3 | | |
|---|---|---|
| 1 | NaD1 | amino acid sequence |
| 2 | PhD1 | amino acid sequence |
| 3 | PhD2 | amino acid sequence |
| 4 | FST | amino acid sequence |
| 5 | NaThio1 | amino acid sequence |
| 6 | NeThio2 | amino acid sequence |
| 7 | NpThio1 | amino acid sequence |
| 8 | TPP3 | amino acid sequence |
| 9 | CcD1 | amino acid sequence |
| 10 | Nt-thionin | amino acid sequence |
| 11 | NTS13 | amino acid sequence |
| 12 | TGAS118 | amino acid sequence |
| 13 | PPT | amino acid sequence |
| 14 | J1-1 | amino acid sequence |
| 15 | J1-2 | amino acid sequence |
| 16 | Rs-AFP1 | amino acid sequence |
| 17 | Rs-AFP2 | amino acid sequence |
| 18 | ZmESR-6 | amino acid sequence |
| 19 | Art v1 (mature domain through C-terminal domain) | amino acid sequence |
| 20 | SF18 (mature domain through C-terminal domain) | amino acid sequence |
| | | |

| SEQ ID NOS - Figure 4 | | |
|---|---|---|
| 21 | Tobacco-β1,3-glucanase | amino acid sequence |
| 22 | Tobacco osmotin AP-24 | amino acid sequence |
| 23 | Brazil nut 25 albumin | amino acid sequence |
| 24 | Barley lectin | amino acid sequence |
| 25 | Wheat germ agglutinin | amino acid sequence |
| 26 | Tobacco CBP 20 | amino acid sequence |
| 27 | Soybean β-conglycinin ά subunit | amino acid sequence |
| 28 | proCon A | amino acid sequence |
| 29 | Barley polyamine oxidase | amino acid sequence |
| 30 | Tobacco chitinase A | amino acid sequence |
| 31 | Phaseolin | amino acid sequence |
| | | |
| 42 | NatD1 | amino acid sequence |
| 43 | SGN U372549 | amino acid sequence |
| | | |

| SEQ ID NOS - Figure 19 | | |
|---|---|---|
| 32 | NaD2 | DNA sequence |
| 33 | NaD2 | amino acid sequence |
| | | |

| SEQ ID NOS - other | | |
|---|---|---|
| 34 | TPP3 CTPP | amino acid sequence |
| 35 | NaPI | amino acid sequence |
| 36 - Table 3 | Sweet potato sporamin | amino acid sequence |
| 37 - Table 3 | Potato 22 kDa protein N-terminal propeptide | amino acid sequence |
| 38 - Table 3 | Barley aleurain N-terminal propeptide | amino acid sequence |
| 39 - Table 3 | Potato cathepsin D inhibitor N-terminal propeptide | amino acid sequence |
| 40 | Modified CTPP | amino acid sequence |
| 41 | Vacuolar Translocation sequence | amino acid sequence |
| 44 | NaPI S-peptide | amino acid sequence |

Embodiments of the invention are also described in the claims of the International application as filed:
1. A chimeric plant defensin, the defensin being a peptide consisting essentially of a signal peptide (S), a mature (M) domain and a C-terminal propeptide tail (T) domain, the M domain being that of a first plant defensin and the T domain being that of a second plant defensin or a non-defensin plant vacuolar translocation peptide.
2. The chimeric plant defensin of claim 1, wherein the M domain is selected from the group of plant M domains having the sequence:
   X₁ X₂ X₃ X₄ X₅ X₆ X₇ C₈ X₉ X₁₀ X₁₁ X₁₂ X₁₃ X₁₄ X₁₅ X₁₆ X₁₇ X₁₈ X₁₉ C₂₀ X₂₁ X₂₂ X₂₃ X₂₄ X₂₅ X₂₆ X₂₇ X₂₈ C₂₉ X₃₀ X₃₁ X₃₂ X₃₄ X₃₅ X₃₆ X₃₇ X₃₈ X₃₉ X₄₀ X₄₁ X₄₂ X₄₃ X₄₄ X₄₅ X₄₆ C₄₇ X₄₈ X₄₉ X₅₀ X₅₁ X₅₂ X₅₃ X₅₄ X₅₅ C₅₆ X₅₇ C₅₈ X₅₉ X₆₀ X₆₁ C₆₂ X₆₃
   Where:
   - C₈ is disulfide bonded to C₆₂
   - X₁-X₅, X₂₆₋₂₈, X₆₃ is no amino acid or any amino acid
   - C₂₀ is disulfide bonded to C₄₇
   - X₆, X₇, X₉₋₁₂, X₁₄, X₁₅, X₁₇, X₁₉, X₂₁₋₂₅, X₃₀₋₃₂, X₄₆, X₅₇, X₅₉₋₆₁, is any amino acid • C₂₉ is disulfide bonded to C₅₆
   - X₁₃ is S, A, C, V, K, P or NO AMINO ACID • and C₃₃ is disulfide bonded to C₅₈
   - X₁₆ is F, W, Y or H
   - X₁₈ is G, F, K or S
   - X₃₄₋₃₇ is any amino acid or up to two of X₃₄₋₃₇ is no amino acid
   - X₃₉₋₄₄ is any amino acid or up to two of X₃₉₋₄₄ is no amino acid
   - X₃₈ is E or A
   - X₄₅ is G or A
   - X₄₈₋₅₅ is any amino acid or up to three of X₄₈₋₅₅ is no amino acid
   and the T domain is a peptide selected from the group of T domain peptides consisting of SEQ ID NO:
   1, amino acids 73-105;
   1, Amino acids 73-76;
   2, amino acids 73-103;
   3, amino acids 75-101;
   5, amino acids 74-106;
   6, amino acids 73-106;
   7, amino acids 74-106;
   8, amino acids 74-105;
   9, amino acids 76-107;
   18, Amino acids 80-107;
   19, amino acids 54-108;
   20, amino acids 58-154;
   21-31, 35, 40-44, entire sequence.
3. The chimeric defensin of claim 2, wherein the M domain is a peptide selected from the group of plant M domains consisting of SEQ ID NO:
   1, amino acids 26-72;
   2, amino acids 26-72;
   3, amino acids 26-74;
   4, amino acids 26-72;
   5, amino acids 26-73;
   6, amino acids 26-72;
   7, amino acids 26-73;
   8, amino acids 26-73;
   9, amino acids 27-75;
   10, amino acids 31-77;
   11, amino acids 31-77;
   12, amino acids 25-72;
   13, amino acids 31-77;
   14, amino acids 28-75;
   15, amino acids 28-74;
   16, amino acids 30-80;
   17, amino acids 30-80;
   18, amino acids 26-79;
   19, amino acids 1-53 ;
   20, amino acids 1-57 or
   33, amino acids 32-78
4. A chimeric defensin according to claim 1, wherein the M-domain is a peptide selected from the group of plant defensin M domains consisting of SEQ ID NO:
   1, amino acids 26-72;
   2, amino acids 26-72;
   3, amino acids 26-74;
   4, amino acids 26-72;
   5, amino acids 26-73;
   6, amino acids 26-72;
   7, amino acids 26-73;
   8, amino acids 26-73;
   9, amino acids 27-75;
   10, amino acids 31-77;
   11, amino acids 31-77;
   12, amino acids 25-72;
   13, amino acids 31-77;
   14, amino acids 28-75;
   15, amino acids 28-74;
   16, amino acids 30-80;
   17, amino acids 30-80;
   18, amino acids 26-79;
   19, amino acids 1-53;
   20, amino acids 1-57 or
   33, Amino acids 32-78.
   and the T domain is a peptide selected from the group of T-domain peptides consisting of SEQ ID NO:
   1, amino acids 73-105;
   1, Amino acids 73-76;
   2, amino acids 73-103;
   3, amino acids 75-101;
   5, amino acids 74-106;
   6, amino acids 73-106;
   7, amino acids 74-106;
   8, amino acids 74-105;
   9, amino acids 76-107;
   18, Amino acids 80-107;
   19, amino acids 54-108;
   20, amino acids 58-154 or
   21-31, 35, 40-44, entire sequence.
5. A chimeric defensin according to claim 4, wherein the T domain is a peptide selected from the group of T-domain peptides consisting of SEQ ID NO:
   1, amino acids 73-105;
   1, amino acids 73-76
   8, Amino acids 74-105;
   35, (entire sequence);
   24, (entire sequence); or
   18, Amino acids 80-107
6. A chimeric defensin according to claim 5, wherein the T domain is a peptide comprising SEQ ID NO:1, amino acids 73-105.
7. A chimeric defensin according to claim 4, wherein the T domain is a peptide comprising SEQ ID NO:35.
8. A chimeric defensin according to claim 4, wherein the M domain is a peptide consisting of the amino acid sequence of SEQ ID NO: 1, amino acids 26-72 and the T domain is a peptide selected from the group of T-domain peptides consisting of SEQ ID NO:
   1, amino acids 73-76
   8, Amino acids 74-105;
   35, (entire sequence);
   24, (entire sequence); or
   18, Amino acids 80-107
9. A chimeric defensin according to claim 5, wherein the M domain is a peptide consisting of the amino acid sequence of SEQ ID NO: 17, amino acids 30-80.
10. A chimeric defensin according to claim 7, wherein the M domain is a peptide selected from the group consisting of SEQ ID NO:1, amino acids 28-72 or 18, amino acids 26-79.
11. The chimeric plant defensin of claim 1, further comprising a signal sequence (S).
12. The chimeric plant defensin of claim 2, further comprising a signal sequence (S) selected from the group of signal peptides consisting of SEQ ID NO:
   1, amino acids 1-25;
   2, amino acids 1-25;
   3, amino acids 1-25;
   4, amino acids 1-25;
   5, amino acids 1-25;
   6, amino acids 1-25;
   7, amino acids 1-25;
   8, amino acids 1-25;
   9, amino acids 1-26;
   10, amino acids 1-30;
   11, amino acids 1-30;
   12, amino acids 1-24;
   13, amino acids 1-30;
   14, amino acids 1-27;
   15, amino acids 1-27;
   16, amino acids 1-29;
   17, amino acids 1-29 ;
   18, amino acids 1-25 or
   44, entire sequence.
13. The chimeric plant defensin of claim 4, further comprising a signal sequence (S), as selected from the group of signal peptides consisting of
   1, amino acids 1-25;
   2, amino acids 1-25;
   3, amino acids 1-25;
   4, amino acids 1-25;
   5, amino acids 1-25;
   6, amino acids 1-25;
   7, amino acids 1-25;
   8, amino acids 1-25;
   9, amino acids 1-26;
   10, amino acids 1-30;
   11, amino acids 1-30;
   12, amino acids 1-24;
   13, amino acids 1-30;
   14, amino acids 1-27;
   15, amino acids 1-27;
   16, amino acids 1-29;
   17, amino acids 1-29 ;
   18, amino acids 1-25 or
   44, entire sequence.
14. The chimeric plant defensin of claim 13, wherein the signal peptide (S) comprises SEQ ID NO:1, amino acids 1-25, the M domain is selected from the group of peptides consisting of SEQ ID NO:1, amino acids 73-105 or SEQ ID NO:33, amino acids 32-78 and the T domain is a peptide selected from the group of peptides consisting of SEQ ID NO:35 or SEQ ID NO:18, amino acids 80-107.
15. The chimeric plant defensin of claim 13, wherein the signal peptide (S) consists of SEQ ID NO:44, the M domain is a peptide selected from the group of peptides consisting of SEQ ID NO:1, amino acids 73-105 or SEQ ID NO:33, amino acids 32-78, and the T domain is a peptide selected from the group consisting of SEQ ID NO:1, amino acids 73-105, 35, entire sequence, or SEQ ID NO:18, amino acids 80-107.
16. A method of making a transgenic plant expressing a chimeric plant defensin comprising transforming a plant cell with a DNA encoding a chimeric plant defensin, the defensin being a peptide consisting essentially of a mature (M) domain and a C-terminal propeptide tail (T) domain, the M domain being that of a first plant defensin and the T domain being that of a second plant defensin or a non-defensin plant vacuolar translocation peptide, whereby a transformed plant cell expressing the chimeric plant defensin is produced, and regenerating the transformed plant cell to produce an adult transgenic plant expressing a chimeric plant defensin.
17. A method according to claim 6, wherein the chimeric defensin M domain is selected from the group of plant M domain peptides having the sequence:
   X₁ X₂ X₃ X₄ X₅ X₆ X₇ C₈ X₉ X₁₀ X₁₁ X₁₂ X₁₃ X₁₄ X₁₅ X₁₆ X₁₇ X₁₈ X₁₉ C₂₀ X₂₁ X₂₂ X₂₃ X₂₄ X₂₅ X₂₆ X₂₇ X₂₆ C₂₉ X₃₀ X₃₁ X₃₂ C X₃₄ X₃₅ X₃₆ X₃₇ X₃₈ X₃₉ X₄₀ X₄₁ X₄₂ X₄₃ X₄₄ X₄₅ X₄₆ C₄₇ X₄₈ X₄₉ X₅₀ X₅₁ X₅₂ X₅₃ X₅₄ X₅₅ C₅₆ X₅₇ C₅₈ X₅₉ X₆₀ X₆₁ C₆₂ X₆₃
   Where:
   - C₈ is disulfide bonded to C₆₂
   - X₁-X₅, X₂₆₋₂₈, X₅₃ is no amino acid or any amino acid
   - C₂₀ is disulfide bonded to C₄₇
   - X₈, X₇, X₉₋₁₂, X₁₄, X₁₅, X₁₇, X₁₉, X₂₁₋₂₅, X₃₀₋₃₂, X₄₆, X₅₇, X₅₉₋₆₁, is
      any amino acid • C₂₉ is disulfide bonded to C₅₈
   - X₁₃ is S, A, C, V, K, P or NO AMINO ACID • and C₃₃ is disulfide bonded to C₅₈
   - X₁₆ is F, W, Y or H
   - X₁₈ is G, F, K or S
   - X₃₄₋₃₇ is any amino acid or up to two of X₃₄₋₃₇ is no amino acid
   - X₃₉₋₄₄ is any amino acid or up to two of X₃₈₋₄₄ is no amino acid
   - X₃₈ is E or A
   - X₄₅ is G or A
   - X₄₈₋₅₅ is any amino acid or up to three of X₄₈₋₅₅ is no amino acid
   whereby a transformed plant cell expressing the chimeric plant defensin is produced, and regenerating the transformed plant cell to produce an adult transgenic plant expressing a chimeric plant defensin.
18. A method according to claim 17, wherein the chimeric defensin M domain is a peptide selected from the group of plant M domain peptides consisting of SEQ ID NO:
   1, amino acids 26-72;
   2, amino acids 26-72;
   3, amino acids 26-74;
   4, amino acids 26-72;
   5, amino acids 26-73;
   6, amino acids 26-72;
   7, amino acids 26-73;
   8, amino acids 26-73;
   9, amino acids 27-75;
   10, amino acids 31-77;
   11, amino acids 31-77;
   12, amino acids 25-72;
   13, amino acids 31-77;
   14, amino acids 28-75;
   15, amino acids 28-74;
   16, amino acids 30-80;
   17, amino acids 30-80;
   18, amino acids 26-79;
   19, amino acids 1-53;
   20, amino acids 1-57 or
   33, amino acids 32-78.
   whereby a transformed plant cell expressing the chimeric plant defensin is produced, and regenerating the transformed plant cell to produce an adult transgenic plant expressing a chimeric plant defensin.
19. A method of making a transgenic plant according to claim 17, wherein the chimeric defensin T-domain is a peptide selected from the group of T domain peptides consisting of SEQ ID NO:
   1, amino acids 73-105;
   1, Amino acids 73-76;
   2, amino acids 73-103;
   3, amino acids 75-101;
   5, amino acids 74-106;
   6, amino acids 73-106;
   7, amino acids 74-106;
   8, amino acids 74-105;
   9, amino acids 76-107;
   18, Amino acids 80-107;
   19, amino acids 54-108;
   20, amino acids 58-154 or
   21-31, 35, 40-44, entire sequence.
20. A method of making a transgenic plant according to claim 16, wherein the chimeric defensin M domain is selected from the group of plant M domains consisting of
   1, amino acids 26-72;
   2, amino acids 26-72;
   3, amino acids 26-74;
   4, amino acids 26-72;
   5, amino acids 26-73;
   6, amino acids 26-72;
   7, amino acids 26-73;
   8, amino acids 26-73;
   9, amino acids 27-75;
   10, amino acids 31-77;
   11, amino acids 31-77;
   12, amino acids 25-72;
   13, amino acids 31-77;
   14, amino acids 28-75;
   15, amino acids 28-74;
   16, amino acids 30-80;
   17, amino acids 30-80;
   18, amino acids 26-79;
   19, amino acids 1-53;
   20, amino acids 1-57 or
   33, amino acids 32-78.
   And the chimeric defensin T domain is a peptide selected from the group of plant T domain peptides consisting of SEQ ID NO:
   1, amino acids 73-105;
   1, Amino acids 73-76;
   2, amino acids 73-103;
   3, amino acids 75-101;
   5, amino acids 74-106;
   6, amino acids 73-106;
   7, amino acids 74-106;
   8, amino acids 74-105;
   9, amino acids 76-107;
   18, Amino acids 80-107;
   19, amino acids 54-108;
   20, amino acids 58-154 or
   21-31, 35, 40-44, entire sequence.
21. A method of making a transgenic plant according to claim 20, wherein the chimeric defensin T domain is a peptide selected from the group of T domain peptides consisting of SEQ ID NO:
   1, amino acids 73-105;
   1, amino acids 73-76
   8, Amino acids 74-105;
   35, (entire sequence);
   24, (entire sequence);
   18, amino acids 80-107 or
   44, entire sequence.
22. A method of making a transgenic plant according to claim 20, wherein the T domain is a peptide comprising SEQ ID NO:1, amino acids 73-105 and the M domain is not SEQ ID NO:1, amino acids 26-73.
23. A method of making a transgenic plant according to claim 20, wherein the T domain is a peptide comprising SEQ ID NO:35.
24. A method of making a transgenic plant according to claim 20, wherein the M domain is a peptide consisting of the amino acid sequence of SEQ ID NO:1, amino acids 26-72, and the T domain is a peptide selected from the group of T-domain peptides consisting of SEQ ID NO:
   1, amino acids 73-76
   8, Amino acids 74-105;
   35, (entire sequence);
   24, (entire sequence); or
   18, Amino acids 80-107
25. A method of making a transgenic plant according to claim 20, wherein the M domain is a peptide comprising the amino acid sequence of SEQ ID NO:17, amino acids 30-80.
26. A method of making a transgenic plant according to claim 23, wherein the M domain is a peptide selected from the group of M domain polypeptides consisting of SEQ ID NO:1, amino acids 28-72; or 18, amino acids 26-79.
27. A transgenic plant made according to the method of claim 16.
28. A transgenic plant made according to the method of claim 20.
29. A transgenic plant made according to the method of claim 22.
30. A transgenic plant made according to the method of claim 23.
31. A transgenic plant made according to the method of claim 24.
32. A transgenic plant made according to the method of claim 25.
33. A transgenic plant made according to the method of claim 26.
34. A method of reducing a toxic effect of defensin expression in a transgenic plant by providing, in reading frame phase with a nucleic acid encoding the defensin, a nucleic acid encoding a vacuolar translocation peptide, wherein the toxic effect is assessed y normalized phenotype or increased expression level in plants expressing defensin together with the vacuolar translocation peptide compared to plants expressing defensin without the vacuolar targeting peptide.
35. The method of claim 34, wherein the transgenic plant is cotton.
36. The method of claim 34 wherein the transgenic plant is rape.
37. The method of claim 34, wherein the transgenic plant is maize.
38. The method of claim 34, wherein the transgenic plant is selected from the group soybean, rice, wheat or sunflower.
39. The method of claim 35, wherein the defensin is a defensin M domain peptide selected from the group of M domains consisting of SEQ. ID NO:
   1, amino acids 26-72;
   2, amino acids 26-72;
   3, amino acids 26-74;
   4, amino acids 26-72;
   5, amino acids 26-73;
   6, amino acids 26-72;
   7, amino acids 26-73;
   8, amino acids 26-73;
   9, amino acids 27-75;
   10, amino acids 31-77;
   11, amino acids 31-77;
   12, amino acids 25-72;
   13, amino acids 31-77;
   14, amino acids 28-75;
   15, amino acids 28-74;
   16, amino acids 30-80;
   17, amino acids 30-80;
   18, amino acids 26-79;
   19, amino acids 1-53;
   20, amino acids 1-57 or 33, amino acids 32-78.
40. The method of claim 35, wherein the vacuolar translocation peptide is selected from the group of peptides consisting of SEQ ID NO:
   1, amino acids 73-105;
   1, Amino acids 73-76;
   2, amino acids 73-103;
   3, amino acids 75-101;
   5, amino acids 74-106;
   6, amino acids 73-106;
   7, amino acids 74-106;
   8, amino acids 74-105;
   9, amino acids 76-107;
   18, Amino acids 80-107;
   19, amino acids 54-108;
   20, amino acids 58-154 or
   21-31, 35, 40-44, entire sequence.
41. The method of claim 40, wherein the defensin is a defensin of M domain peptide of SEQ ID NO:1, amino acids 26-72.
42. The method of claim 39, wherein the vacuolar translocation peptide is a peptide of SEQ ID NO:1, amino acids 73-105.
43. A method according to claim 18, wherein the transgenic plant is a cotton plant.
44. A transgenic plant made by the method of claim 17.
45. A transgenic plant made by the method of claim 18.
46. A method of increasing fungus resistance in a plant variety comprising transforming a plant cell of the plant variety with DNA encoding a chimeric plant defensin comprising a signal domain (S) and a mature domain (M) together having the amino acid sequence by SEQ ID NO: 1 (residues 1-72) and a C-terminal propeptide tail domain (T) having the amino acid sequence selected from the group of T domain peptides consisting of SEQ ID NO:
   2, amino acids 72-103;
   3, amino acids 75-101;
   4, amino acids 73-105;
   5, amino acids 74-106;
   6, amino acids 73-105;
   7, amino acids 74-106;
   8, amino acids 74-105;
   9, amino acids 76-107;
   18, amino acids 80-107;
   19, amino acids 53-107;
   20, amino acids 58-154 or
   44, entire sequence.
47. The method of claim 46, wherein the plant variety is a cotton variety.
48. A method of increasing fungus resistance in a cotton variety comprising transforming a cotton variety cell with DNA encoding a defensin having the amino acid sequence of SEQ ID NO: 1 whereby a cotton variety cell expressing the defensin is produced, and regenerating the transformed cell to produce an adult transgenic cotton plant expressing the defensin, whereby increased fungus resistance is conferred on the cotton variety.

## Claims

1. A chimeric plant defensin, the defensin being a peptide comprising an endoplasmic reticulum signal sequence, a mature defensin domain and a C-terminal prodomain/propeptide, the mature defensin domain being that of a first plant defensin and the C-terminal prodomain/propeptide being that of a second plant defensin, wherein the first plant defensin is a defensin that does not naturally contain a C-terminal prodomain/propeptide and the second plant defensin is a defensin that naturally contains a C-terminal prodomain/propeptide.

2. The chimeric plant defensin of Claim 1 wherein the C-terminal prodomain/propeptide comprises a peptide selected from the group of C-terminal prodomain/propeptide domains consisting of:
amino acids 73-105 of SEQ ID NO:1;
amino acids 73-76 of SEQ ID NO:1;
amino acids 74-105 of SEQ ID NO:8;
amino acids 80-107 of SEQ ID NO:18.
amino acids 73-103 of SEQ ID NO:2;
amino acids 75-101 of SEQ ID NO:3;
amino acids 73-105 of SEQ ID NO:4;
amino acids 74-106 of SEQ ID NO:5;
amino acids 73-105 of SEQ ID NO:6;
amino acids 74-106 of SEQ ID NO:7;
amino acids 76-107 of SEQ ID NO:9;
SEQ ID NO: 42; and
SEQ ID NO: 43.

3. The chimeric defensin according to Claim 1 or 2, wherein the mature domain comprises a peptide selected from the group of mature defensin domains consisting of:
amino acids 32-78 of SEQ ID NO:10;
amino acids 32-78 of SEQ ID NO:11;
amino acids 26-72 of SEQ ID NO:12;
amino acids 32-78 of SEQ ID NO:13;
amino acids 28-75 of SEQ ID NO:14;
amino acids 28-74 of SEQ ID NO:15;
amino acids 30-80 of SEQ ID NO:16;
amino acids 30-80 of SEQ ID NO:17; and
amino acids 32-78 of SEQ ID NO:33.

4. The chimeric defensin according to Claim 1 or 2 or 3, wherein the mature defensin domain comprises amino acids 30-80 of SEQ ID NO:17.

5. The chimeric plant defensin of any one of Claims 1 to 4 wherein the endoplasmic reticulum signal sequence comprises a peptide selected from the group consisting of:
amino acids 1-25 of SEQ ID NO:1;
amino acids 1-25 of SEQ ID NO:2;
amino acids 1-25 of SEQ ID NO:3;
amino acids 1-25 of SEQ ID NO:4;
amino acids 1-25 of SEQ ID NO:5;
amino acids 1-25 of SEQ ID NO:6;
amino acids 1-25 of SEQ ID NO:7;
amino acids 1-25 of SEQ ID NO:8;
amino acids 1-26 of SEQ ID NO:9;
amino acids 1-31 of SEQ ID NO:10;
amino acids 1-31 of SEQ ID NO:11;
amino acids 1-25 of SEQ ID NO:12;
amino acids 1-31 of SEQ ID NO:13;
amino acids 1-27 of SEQ ID NO:14;
amino acids 1-27 of SEQ ID NO:15;
amino acids 1-29 of SEQ ID NO:16;
amino acids 1-29 of SEQ ID NO:17;
amino acids 1-25 of SEQ ID NO:18; and
entire sequence of SEQ ID NO:44,

6. A method of making a transgenic plant expressing a chimeric plant defensin, comprising transforming a plant cell with a DNA encoding a chimeric plant defensin of any one of Claims 1 to 5 whereby a transformed plant cell expressing the chimeric plant defensin is produced, and regenerating the transformed plant cell to produce an adult transgenic plant expressing a chimeric plant defensin.

7. A transgenic plant made according to the method of Claim 6.

8. A part, progeny or seed of a transgenic plant according to Claim 7, wherein said part, progeny or seed comprises DNA encoding a chimeric plant defensin of any one of Claims 1 to 5.

9. A method of reducing a toxic effect of transgenic expression of a defensin that does not naturally contain a C-terminal prodomain/propeptide in a host plant by modifying said transgenic defensin by combining with said transgenic defensin a C-terminal prodomain/propeptide from a defensin that naturally contains a C-terminal prodomain/propeptide, wherein said host plant expressing said transgenic defensin modified with said C-terminal prodomain/propeptide exhibits reduced toxic effects compared to an otherwise comparable host plant expressing an unmodified transgenic defensin that does not naturally contain a C-terminal prodomain/propeptide.

10. The method of Claim 9, wherein said C-terminal prodomain/propeptide is as defined in Claim 2 and/or the mature domain is as defined in Claim 3.

11. A method of increasing fungus resistance in a plant variety comprising transforming a plant cell of the plant variety with DNA encoding a chimeric defensin according to any one of Claims 1 to 5.

12. A method of any one of Claims 6 or 9-11, wherein the plant variety is selected from the group consisting of cotton, rape, corn, maize, soybean, rice, wheat and sunflower.

13. A process for preparing a chimeric plant defensin comprising combining an endoplasmic reticulum signal sequence, a mature defensin domain from a defensin that does not naturally contain a C-terminal prodomain/propeptide and a C-terminal prodomain/propeptide from a defensin that naturally contains a C-terminal prodomain/propeptide.

14. The process of Claim 13 wherein said C-terminal prodomain/propeptide comprises a peptide selected from the group of C-terminal prodomain/propeptides consisting of:
amino acids 73-105 of SEQ ID NO:1;
amino acids 73-76 of SEQ ID NO:1;
amino acids 74-105 of SEQ ID NO:8;
amino acids 80-107 of SEQ ID NO:18.
amino acids 73-103 of SEQ ID NO:2;
amino acids 75-101 of SEQ ID NO:3;
amino acids 73-105 of SEQ ID NO:4;
amino acids 74-106 of SEQ ID NO:5;
amino acids 73-105 of SEQ ID NO:6;
amino acids 74-106 of SEQ ID NO:7;
amino acids 76-107 of SEQ ID NO:9;
SEQ ID NO: 42; and
SEQ ID NO: 43.

15. The process of Claim 13 or 14, wherein said mature defensin domain from a defensin that does not naturally contain a C-terminal prodomain/propeptide comprises a peptide selected from the group of mature defensin domains consisting of:
amino acids 32-78 of SEQ ID NO:10;
amino acids 32-78 of SEQ ID NO:11;
amino acids 26-72 of SEQ ID NO:12;
amino acids 32-78 of SEQ ID NO:13;
amino acids 28-75 of SEQ ID NO:14;
amino acids 28-74 of SEQ ID NO:15;
amino acids 30-80 of SEQ ID NO:16;
amino acids 30-80 of SEQ ID NO:17; and
amino acids 32-78 of SEQ ID NO:33.
